# EUROPEAN PATENT APPLICATION

(11) **EP 4 400 840 A2**
(43) Date of publication of application: **17.07.2024**
(21) Application number: 23203909.9
(22) Date of filing: 20.12.2019
(51) Int. Cl.: G01N 33/564

(54) **COMPOSITIONS AND METHODS USEFUL IN DETECTING AND TREATING MULTIPLE SCLEROSIS AND OTHER DEMYELINATING DISEASES**

(30) Priority: 27.12.2018 US 201862785377 P; 18.07.2019 US 201962875779 P
(62) Divisional of application: 19906536.8
(71) Applicant: University of Utah Research Foundation, Salt Lake City, UT 84108 (US)
(72) Inventor: Kriesel, John, Salt Lake City, 84108 (US); Fischer, Karl F., Salt Lake City, 84108 (US); Bhetariya, Preetida, Salt Lake City, 84108 (US); Granger, Jennifer, Salt Lake City, 84108 (US)
(74) Representative: CH Kilger Anwaltspartnerschaft mbB

(57) **Abstract**

Disclosed herein, are methods of detecting one or more microbes in a subject having or suspected of having a demyelinating disease including multiple sclerosis. Also, disclosed herein are methods of treating subjects who are developing or experiencing a worsening demyelination based on microbial detection.

## Description

### CROSS REFERENCE TO RELATED APPLICATIONS

This application claims the benefit of U.S. Provisional Application No. 62/785,377, filed December 27, 2018, and U.S. Provisional Application No. 62/875,779, filed July 18, 2019. The content of these earlier filed applications is hereby incorporated by reference herein in its entirety.

### BACKGROUND

Multiple Sclerosis (MS) is a chronic demyelinating disease of unknown cause, which affects the brain and spinal cord of about 400,000 individuals in the U.S. A number of infections of the central nervous system (CNS) can lead to demyelination, including distemper (dogs), measles (SSPE, humans), JC virus (humans), and influenza (humans) (Atkins, G. et al. Rev Med Virol, 291-303. (2000)). Microbes, particularly viruses, have long been suspected as causative agents of MS, based on the epidemiology of the disease including geographic patterns, isolated outbreaks, and migration studies (Kurtzke, J. Phys Med Rehab Clin N Am 16, 327-349 (2005); Kurtzke, J. F. J Neurovirol 6 Suppl 2, S134-140 (2000); Meinl, E. Concepts of viral pathogesis of MS. Curr Opin Neurology 12, 303-307 (1999); and Murray, J. Infection as a cause of multiple sclerosis. BMJ 325, 1128 (2002)).

### SUMMARY

Disclosed herein are methods of diagnosing a demyelinating disease in a subject, the methods comprising: a) obtaining a sample from the subject, wherein said sample is suspected of containing microbe-specific antibodies; b) incubating the sample with one or more microbe capture agents, wherein the one or more microbe capture agents bind to one or more of the microbe-specific antibodies, and wherein the microbe-specific antibodies are specific to one or more microbes selected from the list in Table 1, Table 2, or Table 8; c) detecting the presence of one or more microbe-specific antibodies in the sample; and d) diagnosing the subject with a demyelinating disease when the one or more microbe-specific antibodies are present in the sample.

Disclosed herein are methods of detecting a demyelinating disease in a subject, the methods comprising: a) obtaining a sample from the subject, wherein said sample is suspected of containing microbe-specific antibodies specific to one or more microbes selected from the list in Table 1, Table 2 or Table 8; b) contacting the sample with one or more microbe capture agents in conditions to allow one or more microbe capture agents bind to one or more of the microbe-specific antibodies to generate a mixed biological sample, wherein each of the one or more microbe capture agents is specific to one or more a microbe-specific antibodies, and wherein the one or more microbe-specific antibodies are specific to one or more microbes selected from the list in Table 1, Table 2 or Table 8; c) incubating said mixed biological sample under conditions such that: said one or more microbe capture agents specifically binds at least one of said microbe-specific antibodies to form a detectable complex; and d) detecting the presence of said detectable complexes, thereby detecting the presence of one or more microbes selected from the list in Table 1, Table 2 or Table 8, wherein the presence of one or more microbes selected from the list in Table 1 in said mixed biological sample detects a demyelinating disease in a subject.

Disclosed herein are methods of diagnosing and treating a demyelinating disease in a subject, the methods comprising: a) obtaining a sample from the subject, wherein said sample is suspected of containing microbe-specific antibodies; b) incubating the sample with one or more microbe capture agents, wherein the one or more microbe capture agents bind to one or more of the microbe-specific antibodies, and wherein the microbe-specific antibodies are specific to one or more microbes selected from the list in Table 1, Table 2 or Table 8; c) detecting the presence of one or more microbe-specific antibodies in the sample; d) diagnosing the subject with a demyelinating disease when the one or more microbe-specific antibodies are present in the sample; and e) administering: i) an antibacterial agent when the one or more microbe-specific antibodies detected is a bacteria selected from the list in Table 1, Table 2 or Table 8, ii) an antiviral agent when the one or more microbe-specific antibodies detected is a virus selected from the list in Table 1, Table 2 or Table 8, iii) an antifungal agent when the one or more microbe-specific antibodies detected is a fungus selected from the list in Table 1, Table 2 or Table 8, or v) a combination thereof to the subject.

Disclosed herein, are methods of detecting and treating a demyelinating disease in a subject, the methods comprising: a) obtaining a sample from the subject, wherein said sample is suspected of containing microbe-specific antibodies specific to one or more microbes selected from the list in Table 1, Table 2 or Table 8; b) contacting the sample with one or more microbe capture agents in conditions to allow one or more microbe capture agents bind to one or more of the microbe-specific antibodies to generate a mixed biological sample, wherein each of the one or more microbe capture agents is specific to one or more a microbe-specific antibodies, and wherein the one or more microbe-specific antibodies are specific to one or more microbes selected from the list in Table 1, Table 2 or Table 8; c) incubating said mixed biological sample under conditions such that: said one or more microbe capture agents specifically binds at least one of said microbe-specific antibodies to form a detectable complex; d) detecting the presence of said detectable complexes, thereby detecting the presence of one or more microbes selected from the list in Table 1, Table 2 or Table 8, wherein the presence of one or more microbes selected from the list in Table 1, Table 2 or Table 8 in said mixed biological sample detects a demyelinating disease in a subject; and e) administering: i) an antibacterial agent when the one or more microbes detected is a bacteria selected from the list in Table 1, Table 2 or Table 8, ii) an antiviral agent when the one or more microbes detected is a virus selected from the list in Table 1 or Table 2, iii) an antifungal agent when the one or more microbes detected is a fungus selected from the list in Table 1, Table 2 or Table 8, or v) or a combination thereof to the subject.

Disclosed herein are methods of detecting one or more antibodies in a sample, the methods comprising: a) obtaining a sample from a subject, wherein the sample is suspected of containing microbe-specific antibodies specific to one or more microbes selected from the list in Table 1, Table 2 or Table 8; b) incubating the sample with one or more microbe capture agents, wherein the one or more microbe capture agents bind to one or more of the microbe-specific antibodies, and wherein the one or more microbe-specific antibodies are specific to one or more microbes selected from the list in Table 1, Table 2 or Table 8; and c) detecting the presence of one or more microbe-specific antibodies in the sample.

Disclosed herein are methods of detecting one or more microbes in a sample, the method comprising: a) obtaining a sample from a subject; wherein the sample is suspected of containing one or more microbes selected from the list in Table 1, Table 2 or Table 8; b) incubating the sample with one or more microbe capture agents, wherein the one or more microbe capture agents bind to one or more of the microbes selected from the list in Table 1, Table 2 or Table 8; and c) detecting the presence of one or more microbes selected from the list in Table 1, Table 2 or Table 8 in the sample.

Disclosed herein are methods of treating a patient with multiple sclerosis (MS) or an MS-related disease, the methods comprising: a) administering an antibacterial agent when the one or more microbe-specific antibodies detected is a bacteria selected from the list in Table 1, Table 2 or Table 8, b) administering an antiviral agent when the one or more microbe-specific antibodies detected is a virus selected from the list in Table 1, Table 2 or Table 8, c) administering an antifungal agent when the one or more microbe-specific antibodies detected is a fungus selected from the list in Table 1, Table 2 or Table 8, or d) administering a combination of a), b), or c) to the subject; wherein the patient is identified as needing an antibacterial agent, antiviral agent, antifungal agent or a combination thereof by i) obtaining a sample from the patient, wherein the sample is suspected of containing microbe-specific antibodies; ii) incubating the sample with one or more microbe capture agents, wherein the one or more microbe capture agents bind to one or more of the microbe-specific antibodies, and wherein the microbe-specific antibodies are specific to one or more microbes selected from the list in Table 1, Table 2 or Table 8; and iii) detecting the presence of one or more microbe-specific antibodies in the sample.

### BRIEF DESCRIPTION OF THE DRAWINGS

The accompanying figures, which are incorporated in and constitute a part of this specification, illustrate several aspects and together with the description serve to explain the principles of the invention.

Additional advantages of the invention will be set forth in part in the description which follows, and in part will be obvious from the description, or can be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

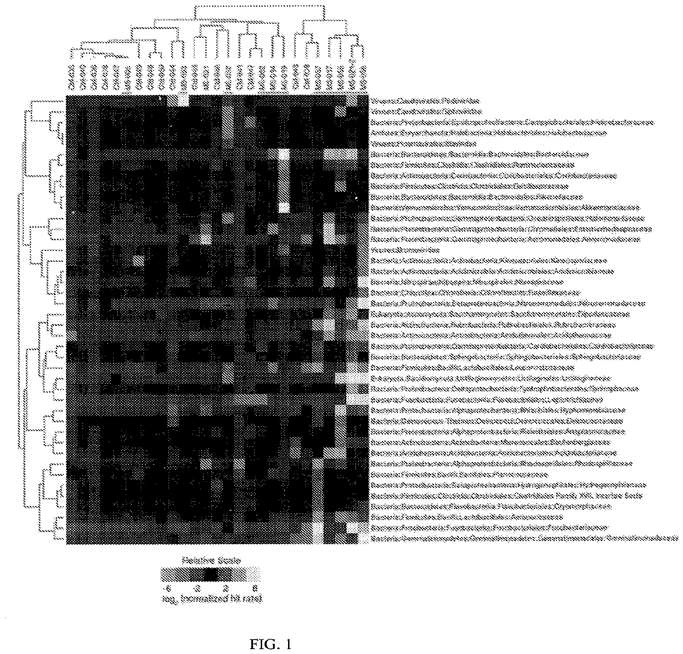

FIG. 1 shows microbial families normalized hit rate hierarchical cluster 3.0 Analysis (de Hoon, M. J., Imoto, S., Nolan, J. & Miyano, S. Bioinformatics (Oxford, England) 20, 1453-1454, (2004)). MS microbes or MS microbial families (N = 42) where at least one of the MS samples (red bars) was significantly overrepresented (false discovery rate, q < 0.05) relative to the set of controls is displayed. The normalized HRs were Log₂ transformed. Rows were centered by subtracting the mean value for each row from every cell in the row. Yellow indicates an increase over the mean value (black) while blue shows a decrease. On the right of the figure there is a prominent cluster containing 4 MS samples and no controls.

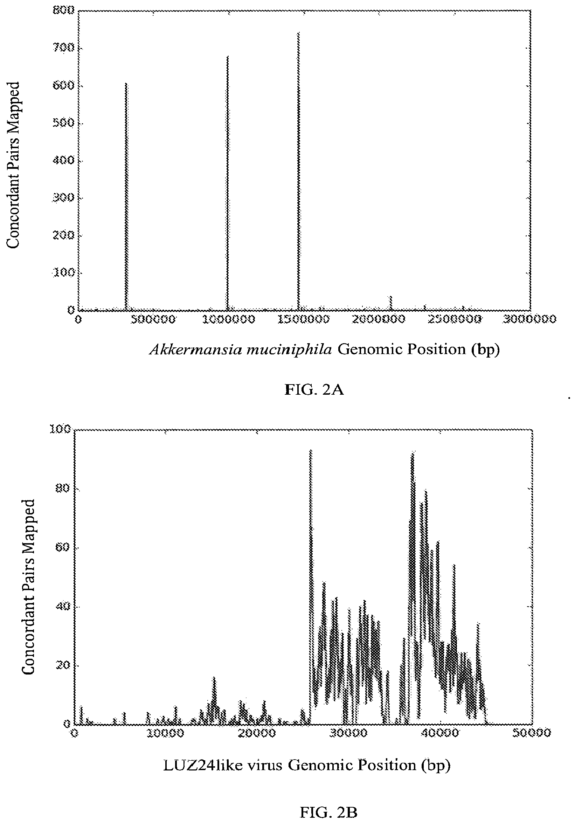

FIGS. 2A-B show representative read-pair mapping to MS microbes. The number of concordant read-pairs mapped (y-axis) to positions along the *Akkermansia* (A) and LUZ24like phage (B) genomes (x-axis) are displayed. The mappings shown were done with Bowtie 2.0 alignments without MAPQ filtering. FIG. 2A shows *Akkermansia muciniphila* read-pair mapping from the brain sample of human subject MS-019. Peaks at 0.3, 1.0, and 1.5 million bp include sequences that map to 16S and 23S rRNA. FIG. 2B shows *Pseudomonas* phage LUZ24like virus read-pair mapping from the brain sample of human subject MS-053.

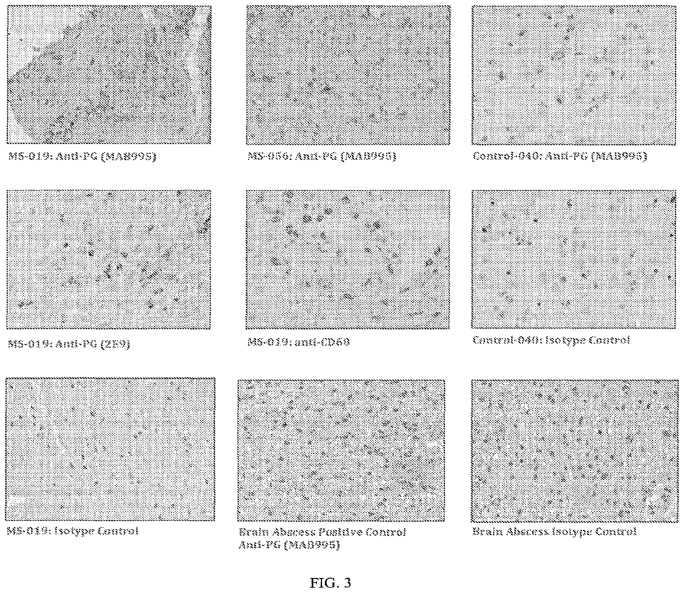

FIG. 3 shows immunohistochemical analysis of human MS brain tissue. Five micron formalin fixed, paraffinized brain tissue sections from four demyelination subjects, three epilepsy controls, and a brain abscess positive control were studied. Photomicrographs displayed are representative. The tissue sections were processed using antigen retrieval followed by casein blocking. The sections were incubated with anti-peptidoglycan mAb (MAB995 or 2E9), anti-CD68 (macrophages, Abeam), anti-lysozyme (macrophages and neutrophils, Abeam), or an equivalent dilution of an isotype control Ab (IgG1, Invitrogen) overnight at 4 degrees. The sections were developed with anti-human IgG-biotin and avidin-HRP. Images shown are magnified 125x. Brain tissue from subject MS-019 shows specific peptidoglycan staining with two different anti-peptidoglycan mAbs. Peptidoglycan signal is also seen in the brain abscess positive control, specimen MS-056, and epilepsy control subject 040.

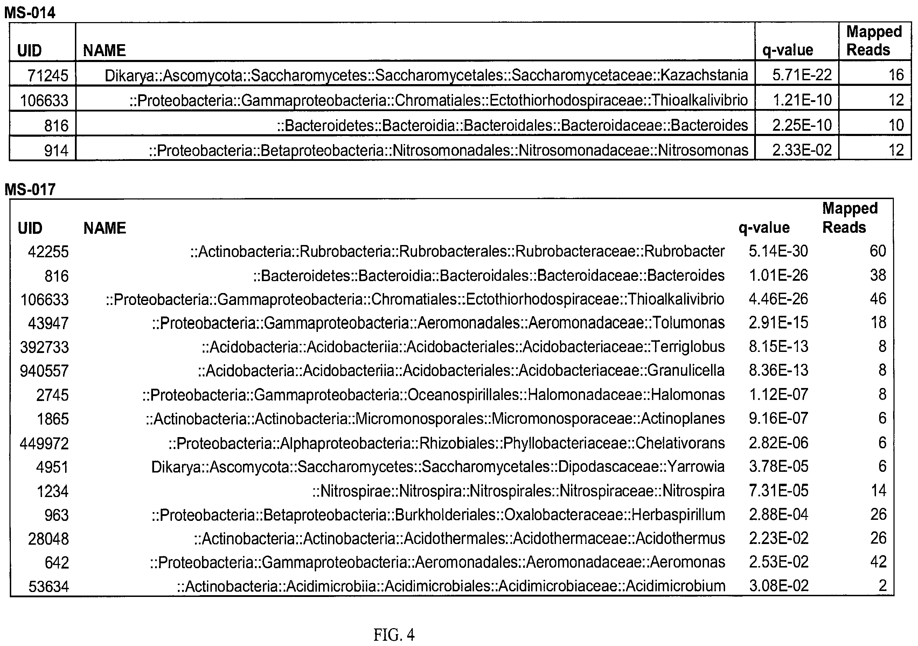

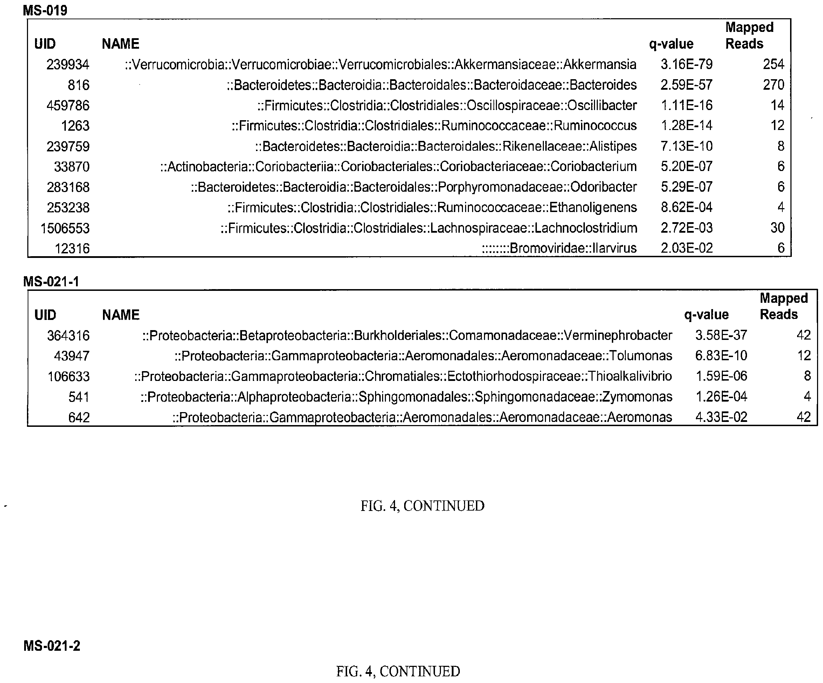

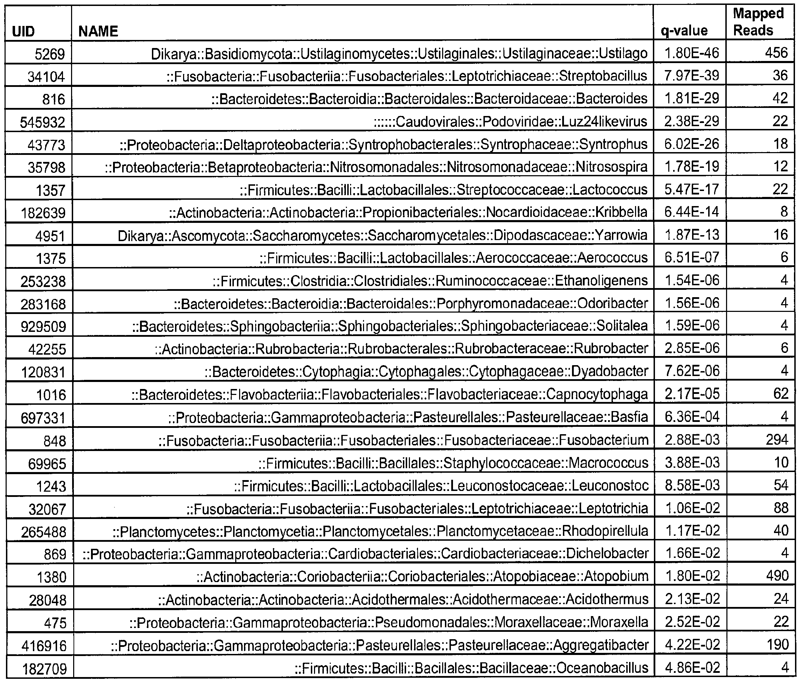

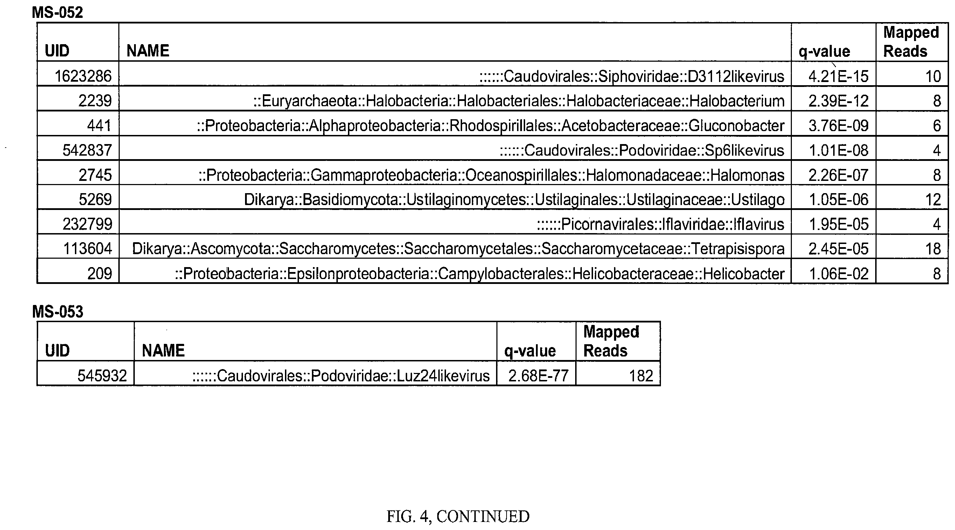

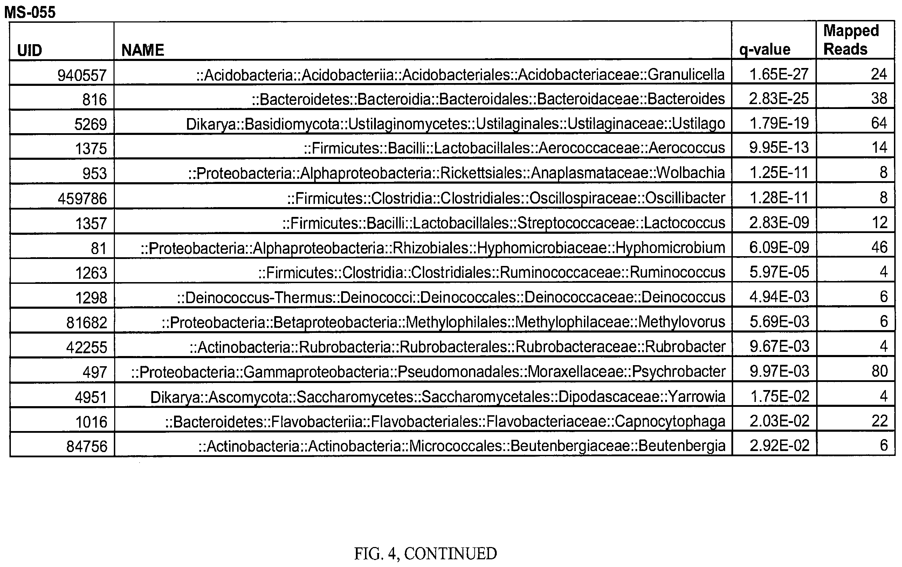

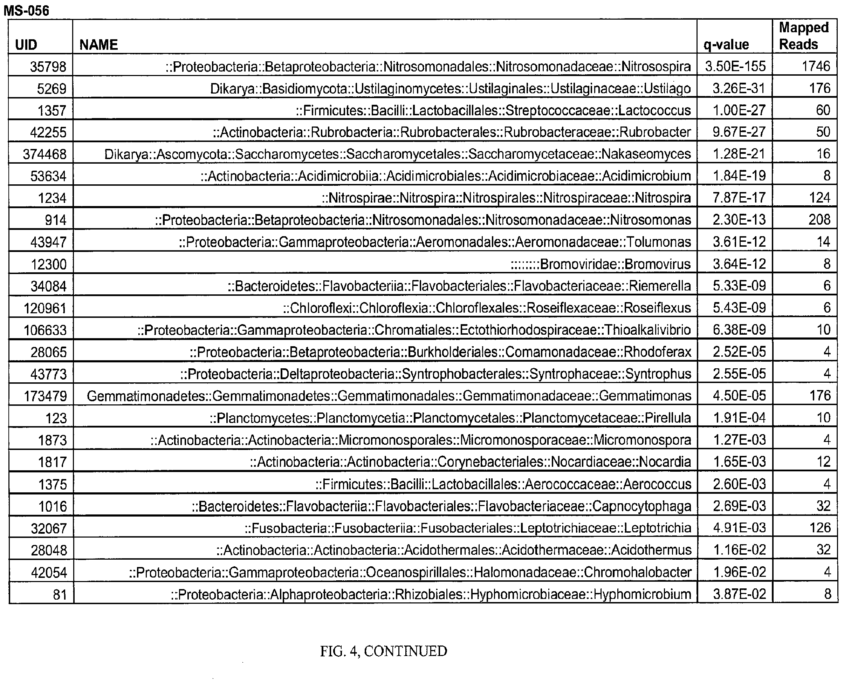

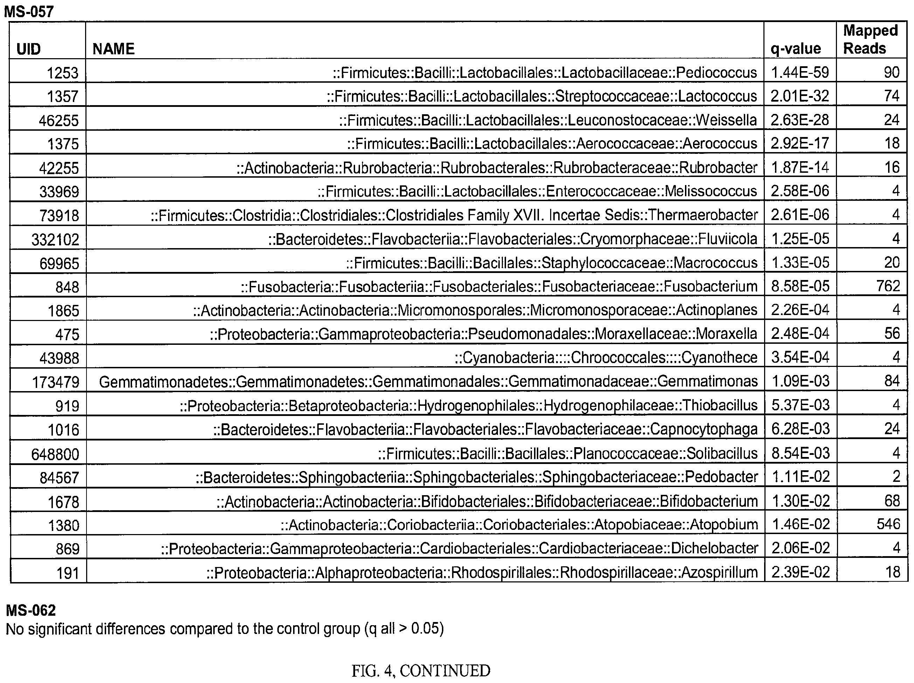

FIG. 4 shows subject/sample specific MS microbes. For the specimens, Taxa (NAME) significantly different than controls with the False Discovery Rate (q) <0.05.

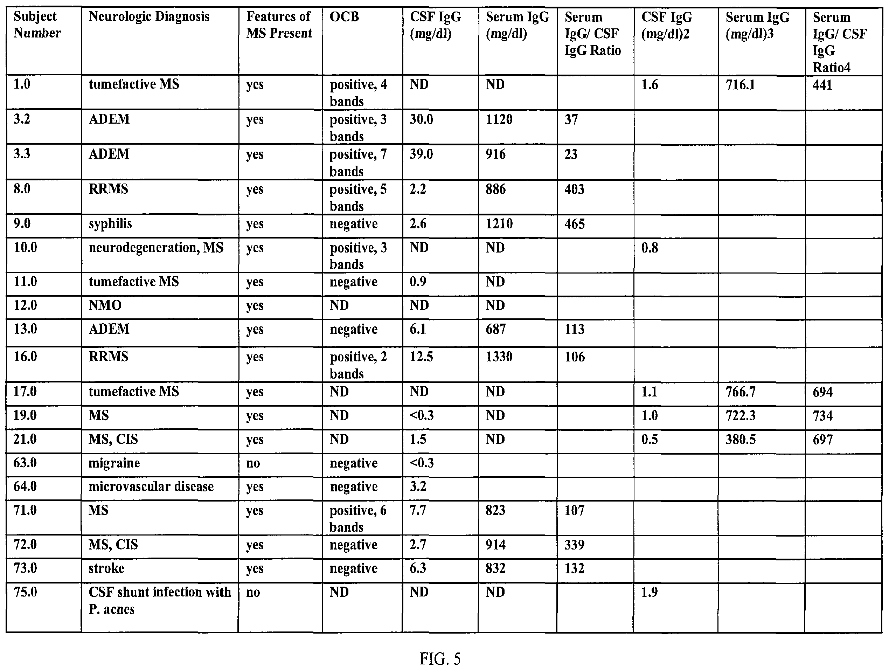

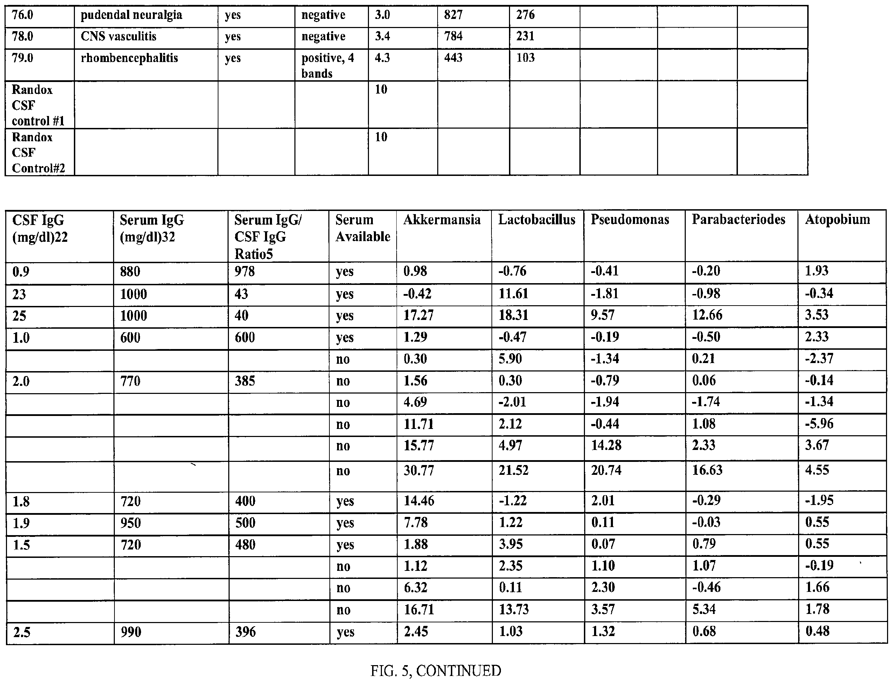

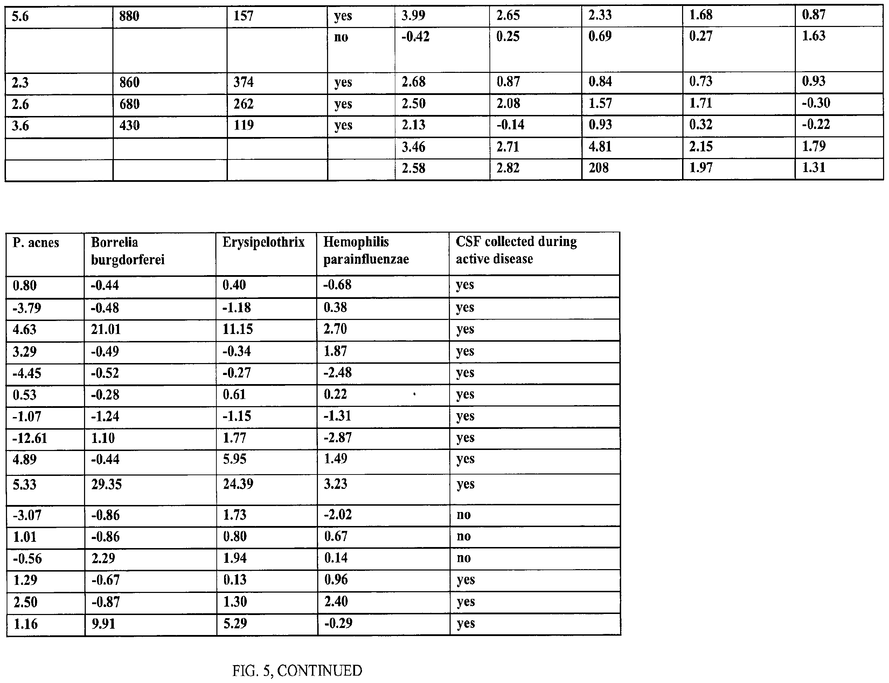

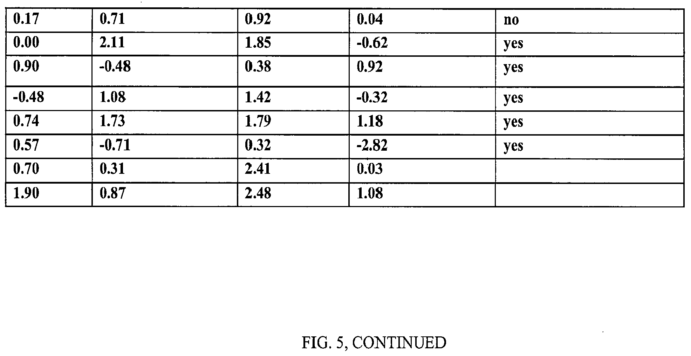

FIG. 5 shows the results of CSF serologies directly compared. OD directly compared with the set of 4 controls, no background adjustment. Z-scores displayed.

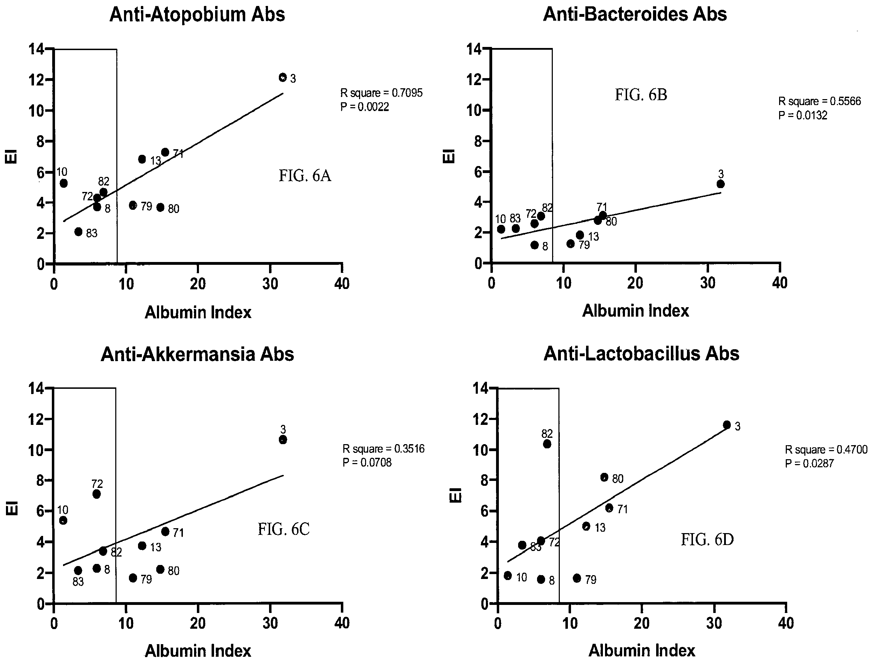

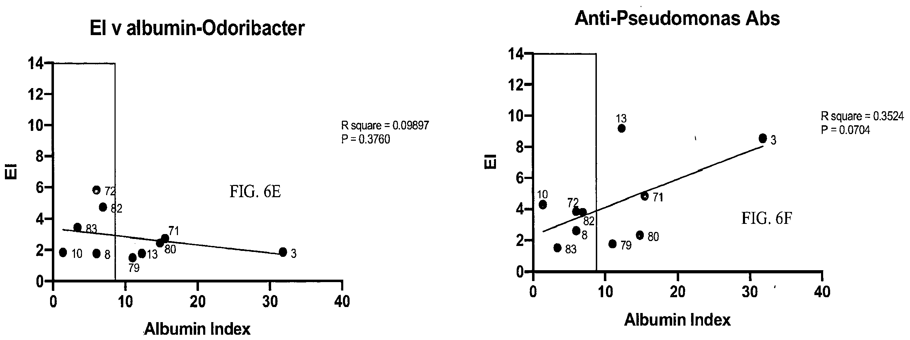

FIGS. 6A-F shows spinal fluid ELISA indices (antibodies) against six MS microbes or MS microbial families plotted against the albumin index for the individual subjects. The ELISA index (EI) is compared to the albumin index (AI) in individual subjects with MS or other neurological diseases. The boxed region indicates the normal albumin index region (0-9) indicating blood-brain barrier (BBB) intactness. The line is linear regression showing the relationship between the EI's for each antigen and BBB intactness. Values within the boxed region and above the line indicate possible intrathecal antibody synthesis (i.e., causality of demyelination). Notably, subjects 10 and 72 are MS patients while 82 has anti-MOG disease. FIG. 6A shows EI v AI for *Atopobium.* FIG. 6B shows EI v AI for *Bacteroides.* FIG. 6C shows EI v AI for *Akkermansia.* FIG. 6D shows EI v AI for *Lactobacillus.* FIG. 6E shows EI v AI for *Odoribacter.* FIG. 6F shows EI v AI for *Pseudomonas.*

### DETAILED DESCRIPTION

The present disclosure can be understood more readily by reference to the following detailed description of the invention, the figures and the examples included herein.

Before the present methods and compositions are disclosed and described, it is to be understood that they are not limited to specific synthetic methods unless otherwise specified, or to particular reagents unless otherwise specified, as such may, of course, vary. It is also to be understood that the terminology used herein is for the purpose of describing particular aspects only and is not intended to be limiting. Although any methods and materials similar or equivalent to those described herein can be used in the practice or testing of the present invention, example methods and materials are now described.

Moreover, it is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including matters of logic with respect to arrangement of steps or operational flow, plain meaning derived from grammatical organization or punctuation, and the number or type of aspects described in the specification.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which can require independent confirmation.

### DEFINITIONS

As used in the specification and the appended claims, the singular forms "a," "an" and "the" include plural referents unless the context clearly dictates otherwise.

The word "or" as used herein means any one member of a particular list and also includes any combination of members of that list.

Ranges can be expressed herein as from "about" or "approximately" one particular value, and/or to "about" or "approximately" another particular value. When such a range is expressed, a further aspect includes from the one particular value and/or to the other particular value. Similarly, when values are expressed as approximations, by use of the antecedent "about," or "approximately," it will be understood that the particular value forms a further aspect. It will be further understood that the endpoints of each of the ranges are significant both in relation to the other endpoint and independently of the other endpoint. It is also understood that there are a number of values disclosed herein and that each value is also herein disclosed as "about" that particular value in addition to the value itself. For example, if the value "10" is disclosed, then "about 10" is also disclosed. It is also understood that each unit between two particular units is also disclosed. For example, if 10 and 15 are disclosed, then 11, 12, 13, and 14 are also disclosed.

As used herein, the terms "optional" or "optionally" mean that the subsequently described event or circumstance may or may not occur and that the description includes instances where said event or circumstance occurs and instances where it does not.

As used herein, the term "sample" is meant a tissue or organ from a subject; a cell (either within a subject, taken directly from a subject, or a cell maintained in culture or from a cultured cell line); a cell lysate (or lysate fraction) or cell extract; or a solution containing one or more molecules derived from a cell or cellular material (e.g. a polypeptide or nucleic acid), which is assayed as described herein. A sample may also be any body fluid or excretion (for example, but not limited to, blood, urine, stool, saliva; tears, bile, cerebral spinal fluid) that contains cells or cell components. In some aspects, the sample can be taken from the brain, spinal cord, cerebral spinal fluid or blood.

As used herein, the term "subject" refers to the target of administration, e.g., a human. Thus the subject of the disclosed methods can be a vertebrate, such as a mammal, a fish, a bird, a reptile, or an amphibian. The term "subject" also includes domesticated animals (e.g., cats, dogs, etc.), livestock (e.g., cattle, horses, pigs, sheep, goats, etc.), and laboratory animals (e.g., mouse, rabbit, rat, guinea pig, fruit fly, etc.). In some aspects, a subject can be a mammal. In some aspects, a subject can be a human. The term does not denote a particular age or sex. Thus, adult, child, adolescent and newborn subjects, as well as fetuses, whether male or female, are intended to be covered.

As used herein, the term "patient" refers to a subject afflicted with a disease or disorder. The term "patient" includes human and veterinary subjects. In some aspects of the disclosed methods, the "patient" has been diagnosed with a need for treatment for multiple sclerosis, such as, for example, prior to the administering step. In some aspects of the disclosed methods, the "patient" has been diagnosed with a need for treatment for a demyelinating disease, such as, for example, prior to the administering step.

As used herein, the term "normal" refers to an individual, a sample or a subject that does not have a demyelinating disease or does not have an increased susceptibility of developing a demyelinating disease.

As used herein, the term "susceptibility" refers to the likelihood of a subject developing or being clinically diagnosed with a disease. For example, a human subject with an increased susceptibility for a demyelinating disease (e.g., multiple sclerosis, neuromyelitis optics, acute disseminated encephalomyelitis or clinically isolated syndrome) can refer to a human subject with an increased likelihood of a subject developing or being clinically diagnosed with a demyelinating disease (e.g., multiple sclerosis, neuromyelitis optics, acute disseminated encephalomyelitis or clinically isolated syndrome).

As used herein, the term "comprising" can include the aspects "consisting of" and "consisting essentially of."

By "specifically binds" is meant that an antibody recognizes and physically interacts with its cognate antigen (for example, a microbe-specific antibody, wherein the microbe-specific antibodies are specific to one or more microbes selected from the list in Table 1, Table 2 or Table 8) and does not significantly recognize and interact with other antigens; such an antibody may be a polyclonal antibody or a monoclonal antibody, which are generated by techniques that are well known in the art.

By "probe," "primer," or oligonucleotide is meant a single-stranded DNA or RNA molecule of defined sequence that can base-pair to a second DNA or RNA molecule that contains a complementary sequence (the "target"). The stability of the resulting hybrid depends upon the extent of the base-pairing that occurs. The extent of base-pairing is affected by parameters such as the degree of complementarity between the probe and target molecules and the degree of stringency of the hybridization conditions. The degree of hybridization stringency is affected by parameters such as temperature, salt concentration, and the concentration of organic molecules such as formamide, and is determined by methods known to one skilled in the art. Probes or primers specific for a microbe-specific antibody (wherein the microbe-specific antibodies are specific to one or more microbes selected from the list in Table 1, Table 2 or Table 8) and have at least 80%-90% sequence complementarity, preferably at least 91%-95% sequence complementarity, more preferably at least 96%-99% sequence complementarity, and most preferably 100% sequence complementarity to the region of the microbe-specific antibody to which they hybridize. Probes, primers, and oligonucleotides may be detectably-labeled, either radioactively, or non-radioactively, by methods well-known to those skilled in the art. Probes, primers, and oligonucleotides are used for methods involving nucleic acid hybridization, such as: nucleic acid sequencing, reverse transcription and/or nucleic acid amplification by the polymerase chain reaction, single stranded conformational polymorphism (SSCP) analysis, restriction fragment polymorphism (RFLP) analysis, Southern hybridization, Northern hybridization, in situ hybridization, electrophoretic mobility shift assay (EMSA).

By "specifically hybridizes" is meant that a probe, primer, or oligonucleotide recognizes and physically interacts (that is, base-pairs) with a substantially complementary nucleic acid (for example, a microbe-specific antibody) under high stringency conditions, and does not substantially base pair with other nucleic acids.

By "high stringency conditions" is meant conditions that allow hybridization comparable with that resulting from the use of a DNA probe of at least 40 nucleotides in length, in a buffer containing 0.5 M NaHPO₄, pH 7.2, 7% SDS, 1 mM EDTA, and 1% BSA (Fraction V), at a temperature of 65°C, or a buffer containing 48% formamide, 4.8X SSC, 0.2 M Tris-Cl, pH 7.6, 1X Denhardt's solution, 10% dextran sulfate, and 0.1% SDS, at a temperature of 42°C. Other conditions for high stringency hybridization, such as for PCR, Northern, Southern, or in situ hybridization, DNA sequencing, etc., are well-known by those skilled in the art of molecular biology. (See, for example, F. Ausubel et al., Current Protocols in Molecular Biology, John Wiley & Sons, New York, NY, 1998). The term "nucleic acid" as used herein refers to a naturally occurring or synthetic oligonucleotide or polynucleotide, whether DNA or RNA or DNA-RNA hybrid, single-stranded or doublestranded, sense or antisense, which is capable of hybridization to a complementary nucleic acid by Watson-Crick base-pairing. Nucleic acids of the invention can also include nucleotide analogs (*e.g.,* BrdU), and non-phosphodiester internucleoside linkages (*e.g.,* peptide nucleic acid (PNA) or thiodiester linkages). In particular, nucleic acids can include, without limitation, DNA, RNA, cDNA, gDNA, ssDNA, dsDNA or any combination thereof.

As used herein, a "control" is a sample from either a normal subject or from tissue that is not demyelinating or demyelinated.

As used herein, "over-expression" means expression greater than the expression detected in normal, non-demyelinting tissue. For example, a nucleic acid that is over-expressed may be expressed about 1 standard deviation above normal, or about 2 standard deviations above normal, or about 3 standard deviations above the normal level of expression. Therefore, a nucleic acid that is expressed about 3 standard deviations above a control level of expression is a nucleic acid that is over-expressed.

As used herein, the term "treat" or "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal (e.g., a human), and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed. For example, "prevent" is meant to mean minimize the chance that a subject who has an increased susceptibility for developing a demyelinating disease will develop a demyelinating disease.

As used herein, the term "reference," "reference expression," "reference sample," "reference value," "control," "control sample" and the like, when used in the context of a sample or expression level of one or more microbes refers to a reference standard wherein the reference is expressed at a constant level among different (i.e., not the same tissue, but multiple tissues) tissues, and is unaffected by the experimental conditions, and is indicative of the level in a sample of a predetermined disease status (e.g., not suffering from a demyelinating disease). The reference value can be a predetermined standard value or a range of predetermined standard values, representing no illness, or a predetermined type or severity of illness.

As used herein, the term "diagnosed" means having been subjected to a physical examination by a person of skill, for example, a physician, and found to have a condition that can be diagnosed or treated by the compounds, compositions, or methods disclosed herein. For example, "diagnosed with a disorder of demyelinating disease" means having been subjected to a examination by a person of skill, for example, a physician, and found to have a condition that can be diagnosed or treated by a compound or composition disclosed herein.

As used herein, the term "microbe" refers to a microscopic organism (i.e., a living thing that is too small to be seen with the naked eye). Generally, the term "microbe" is used to describe different life forms that can have different sizes and characteristics. For example, a microbe can refer to bacteria, archaea, fungi, protists, viruses and microscopic animals.

As used herein, the term "microbe capture agent" refers to a molecule that can bind or capture at least one microbe (e.g., directly) or can capture a microbe-specific antibody wherein the microbe-specific antibody is specific to one or more microbes selected from the list in Table 1, Table 2 or Table 8 (e.g., capture or bind at least one microbe indirectly).

Acute tumefactive MS is an acute tumor-like variant where some patients with demyelinating disease present with large acute lesions, often associated with edema and/or ring enhancement on imaging studies (Kepes, J. J. Ann Neurol 33, 18-27, (1993); and Lucchinetti, C. F. et al. Brain 131, 1759-1775, (2008)). This type of inflammatory demyelinating disease is also called pseudotumoral MS, transitional sclerosis, diffuse myelinoclastic sclerosis, and Marburg variant MS. The initial description by Kepes (Kepes, J. J. Ann Neurol 33, 18-27, (1993)) suggested that a few such patients would go on to develop MS. However, a more recent, much larger study of 168 patients with biopsy-confirmed CNS inflammatory demyelinating disease showed that the majority of such patients (79%) go on to develop clinically definite MS (Lucchinetti, C. F. et al. Brain 131, 1759-1775, (2008)). Clinically isolated syndrome (CIS) refers to a single attack compatible with MS, such as optic neuritis. Sixty to 80 percent of patients with a CIS and magnetic resonance imaging (MRI) lesions go on to develop MS, while approximately 20-40 percent have a self-limited process (Frohman, E. M. et al. Neurology 61, 602-611 (2003); Brex, P. A. et al. N Engl J Med 346, 158-164, (2002); and Olek, M. in UpToDate (ed Francisco Gonzalez-Scarano) (UpToDate, Waltham, MA, 2011).

The pathology of multiple sclerosis (MS) is well summarized by Lucchinetti (Popescu, B. F., Pirko, I. & Lucchinetti, C. F. Continuum (Minneapolis, Minn.) 19, 901-921, (2013): "The pathologic hallmark of multiple sclerosis (MS) is multiple focal areas of myelin loss within the CNS called plaques or lesions.... Acute active MS lesions are hypercellular demyelinated plaques massively infiltrated by macrophages evenly distributed throughout the lesion forming the classic 'sea of macrophages.' These macrophages contain myelin debris, an indication that they have taken up and degraded the remnants of the destroyed myelin sheaths (i.e., active demyelination)."

Given these factors, including known infectious causes of demyelination and the macrophage-dominated pathology of MS plaques, it is contemplated herein that microbes within brain parenchyma might trigger the onset of MS, or the worsening of existing MS disease. Disclosed herein are the results from testing whether the microbial sequence content of primary demyelination brain samples would differ from that in a set of controls. The feasibility of RNA extractions and deep sequencing from such tissues was demonstrated.

Disclosed herein are methods that can be used to demonstrate the microbial causes of multiple sclerosis in individual patients. The first step was to obtain RNA sequencing data from MS brain lesions and compare the results against a set of controls or a reference sample. Microbial taxa that are significantly overrepresented in the MS group were then used to prepare a MS microbes or MS microbial families list.

Disclosed herein are also methods that can be used alone or in combination with the methods described herein that can be used to demonstrate microbial causes of multliple sclerosis in individual patients and targets for subsequent therapy. For example, disclosed herein are methods that can be used to assess the intactness of the blood brain barrier. In some aspects, said method can be used to detect and/or correct for a leaky blood brain barrier that can be associated with multiple sclerosis and other demyelinating diseases. In some aspects, said method can be used to determine whether antibodies in the CSF of MS patients that are directed to some of the MS microbes of MS microbial families disclosed herein indicate intrathecal antibody synthesis.

Described herein are sets of microbial reagents that can be used in a set of assays (e.g., serologic). Disclosed herein are assays that can be used to detect antibodies in the CSF of MS patients that are directed against some of the MS microbes or MS microbial families. An exemplary list of MS microbes or MS microbial families is provided in Figure 4 and/or Figure 5 as well as Table 1, Table 2 and/or Table 8. The results of the methods disclosed herein can be used to direct antimicrobial therapy in patients with MS and related diseases (e.g., acute disseminated encephalomyelitis or neuromyelitis optics) who are developing (i.e., new onset) or experiencing worsening (i.e., relapses of) demyelination. For instance, a patient with CSF reactivity to *Akkermansia mucinophilia* might be treated with intravenous meropenem, predicted to be active, and not with ceftriaxone, predicted to be inactive against this anaerobic bacterium.

The MS microbes or MS microbial families described herein can also be used to develop specific PCR reagents to detect one or more of the MS microbes or MS microbial families disclosed herein that are causing demyelination. For example, primers can be developed and used to detect these MS-causing organisms. CSF samples can be used from patients who are early in their course and developing demyelination, before the appearance of specific anti-microbial antibodies and oligoclonal bands. Specific PCR primers can be developed and used to detect the MS microbes or MS microbial families disclosed herein in brain tissue, spinal cord, CSF, or blood. A number of primer sets can be multiplexed in a single test. Again, the detection of microbial DNA (or less likely RNA) in the CSF of patients with early demyelination (or those having attacks of established MS) could be used to direct specific antimicrobial treatments.

The methods disclosed here can be used to detect and treat the putative underlying cause of MS and several related demyelinating disorders including but not limited to neuromyelitis optics (NMO), acute disseminated encephalomyelitis (ADEM), and clinically isolated syndrome (CIS). An advantage of the methods disclosed herein is that they can serve to limit or reverse the damage to the CNS. For instance, a patient with CSF reactivity to *Akkermansia mucinophilia* might be treated with intravenous meropenem, predicted to be active, and not with ceftriaxone, predicted to be inactive against this anaerobic bacterium. Current MS treatments are unreliable and inconsistent where there are no specific factors underlying the choice of treatment, and no reasonable expectations of success or failure. Current treatments for acute MS attacks include strategies that are broadly immunosuppressive (e.g., steroids, rituximab) that can have serious side effects.

Described herein are methods and microbial reagents that can be used alone or in a set of assays (e.g., serologic). Described herein are methods that can be used to detect antibodies in the CSF of MS patients that are directed against some but not all of the MS microbes or MS microbial families described herein.

Described herein are methods that can be used alone or in combination with a set of assays. Described herein are methods comprising determining a microbial ELISA index (EI) and comparing the results of the EI with the albumin index of the subject. In some aspects, said method can be used to determine whether antibodies in the CSF of MS patients that are directed to one or more of the MS microbes of MS microbial families disclosed herein indicate intrathecal antibody synthesis. Further, said method can be used to identify a subject that may not have been detected or was excluded using any of the other methods of detection described herein. For example, the disclosed methods of determining a microbial ELISA index (EI) and comparing the results of the EI of the subject with the albumin index of the subject can be used to improve on the identification of a subject with a demyelinating disease as compared to determining a microbial ELISA index (EI) alone.

The assays and methods described herein can be used to direct antimicrobial therapy in patients with MS and related diseases (i.e., ADEM, NMO) who are developing (e.g., new onset) or experiencing worsening (e.g., relapses of) demyelination. For example, a patient with CSF reactivity to *Akkermansia mucinophilia* can be treated with intravenous meropenem, predicted to be active, and not with ceftriaxone, predicted to be inactive against this anaerobic bacterium.

The MS microbes or MS microbial families described herein can also be used to develop specific PCR reagents to detect one or more of the MS microbes or MS microbial families disclosed herein that are causing demyelination. Primers can be developed to detect MS-causing organisms. The reagents (e.g., primers) can be applied to the CSF of patients who are early in their course and developing demyelination, before the appearance of specific anti-microbial antibodies and oligoclonal bands. In some aspects, a number of primer sets would be multiplexed in a single test. Disclosed herein a methods of detection of microbial DNA (or less likely RNA) in the CSF of patients with early demyelination (or those having attacks of established MS) which can be used to direct specific antimicrobial treatments.

Described herein are methods of detecting and treating the putative underlying cause of MS and several related demyelinating disorders including, but not limited to neuromyelitis optics (NMO),
acute disseminated encephalomyelitis (ADEM), and clinically
isolated syndrome (CIS).

Disclosed here are serologic assays
to detect antibodies in the CSF of MS patients that are directed against one or more of the MS microbes or MS microbial families described herein. Disclosed herein are methods to direct antimicrobial therapy in patients with MS and related diseases (e.g., ADEM, NMO) who are developing (e.g. new onset) or experiencing worsening (e.g. relapses of) demyelination.

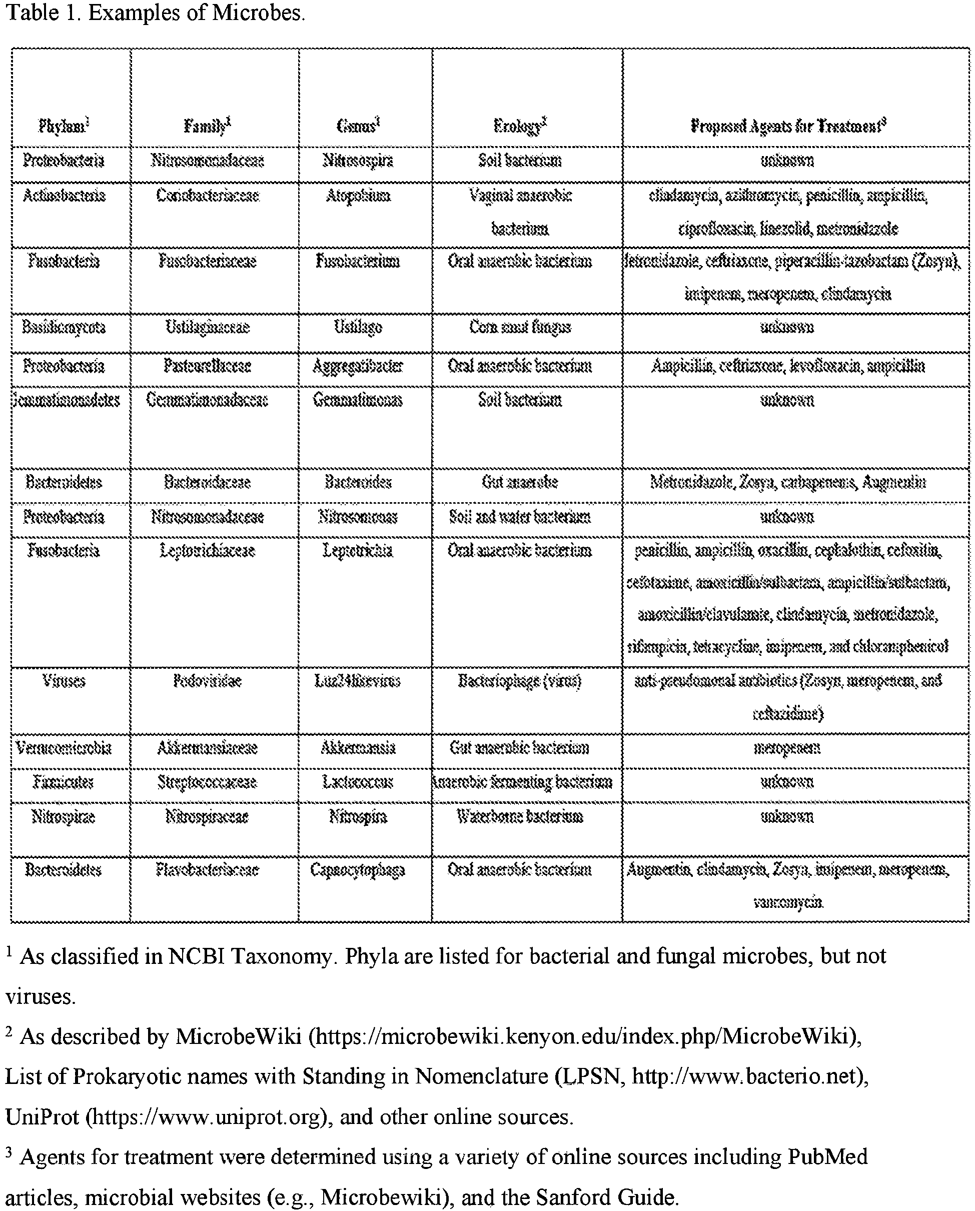

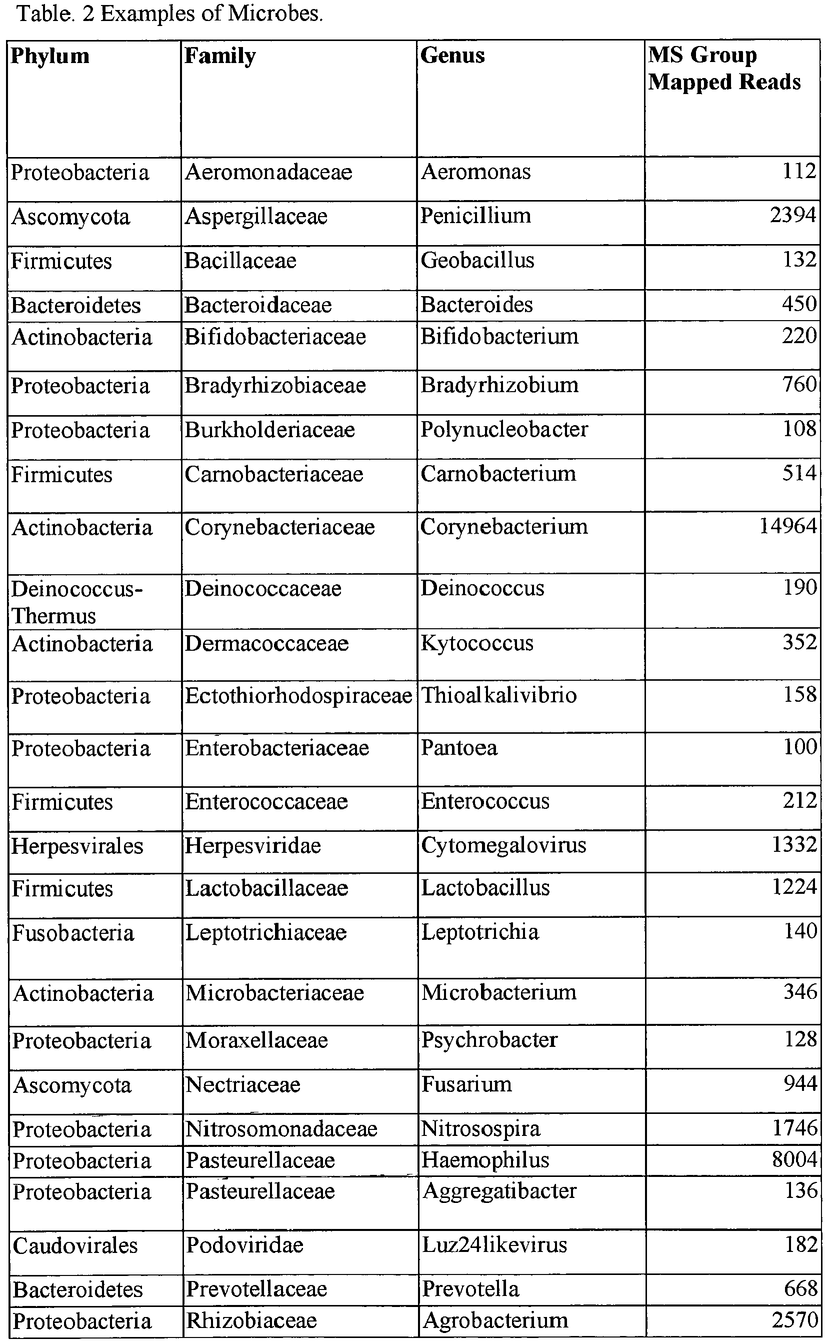

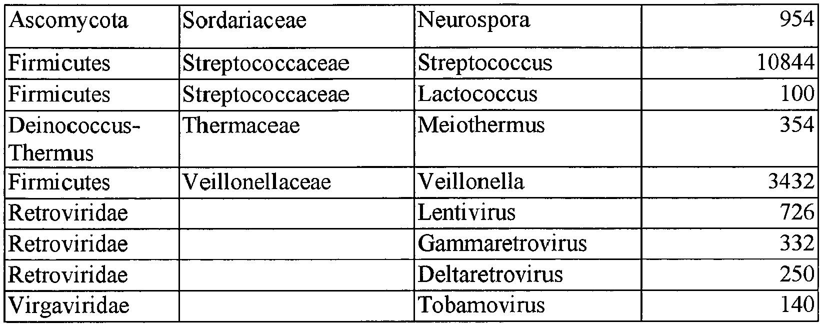

MS microbes or MS microbial families derived from the deep sequencing data. In order to qualify as an MS microbe or MS microbial family at the genus level, at least one specimen from the MS group had read-pair mappings to this taxa significantly increased (q<0.05) over the control group with MAPQ values ≥ 10 (251 separate genera). The MS genus microbes shown had at least 100 mapped reads among all the specimens in the MS group (35 genera).

### METHODS

Disclosed herein are methods of diagnosing and/or detecting a demyelinating disease in a subject. The methods disclosed herein can be useful for making a microbiological diagnosis. The detection methods described herein can be performed directly via PCR amplification of a nucleic acid or via direct detection using antibodies or peptides that specifically bind one or more of the disclosed microbes. The detection methods described herein can also be performed by detecting antibodies specific to one or more of the microbe-specific antibodies. For example, disclosed herein are methods comprising contacting a sample with one or more microbe capture agents in conditions to allow one or more microbe capture agents bind to one or more of the microbe-specific antibodies to generate a mixed biological sample, wherein each of the one or more microbe capture agents is specific to one or more a microbe-specific antibodies, and wherein the one or more microbe-specific antibodies are specific to one or more microbes selected from the list in Table 1, Table 2 or Table 8.

Also disclosed herein are methods of treating a subject at risk for developing a demyelinating disease, treating a subject with a demyelinating disease or treating a subject suscepted of having a demyelinating disease. Once the presence of one or microbes or one or more of the microbe-specific antibodies are determined, the appropriate treatment can be provided or administered.

Disclosed herein are methods of diagnosing and/or detecting a demyelinating disease in a subject, wherein the methods disclosed herein can be used to determine intrathecal production of antibodies. The methods disclosed herein can be useful for making a microbiological diagnosis. The methods disclosed herein can also be used to identify a subject with a demyelinating disease that was not identified in any of the other methods described herein. The methods disclosed herein can be used to determine the intactness of the blood brain barrier. The detection methods described herein can compare a microbial ELISA index (EI) of a subject to an albumin index (AI) of the subject. The methods disclosed herein can be performed via ELISA using antibodies that bind or specifically bind to one or more of the disclosed microbes. For example, disclosed herein are methods comprising contacting a sample with one or more microbe capture agents in conditions to allow one or more microbe capture agents bind to one or more of the microbe-specific antibodies to generate a mixed biological sample, wherein each of the one or more microbe capture agents is specific to one or more a microbe-specific antibodies, and wherein the one or more microbe-specific antibodies are specific to one or more microbes selected from the list in Table 1, Table 2 or Table 8. The EI is a measure of antibodies in a sample (e.g., CSF or spinal fluid). In some aspects, the EI value can be compared to a normal AI (e.g., 0 to 9) that indicates in intact blood brain barrier. Albumin Index = CSF albumin (mg/L) / serum albumin (g/L). The AI an index of blood-brain barrier (BBB) integrity, adjusted for the serum albumin concentration. The AI is increased in BBB dysfunction. The EI for a subject for one or more microbes selected from, for example, the list in Table 1, Table 2 and/or Table 8 can be plotted against the AI of the subject. Linear regression analysis can be performed showing the relationship between the EI's for each antigen and BBB intactness (as assessed by the Albumin Index). A linear regression line or plot line can be generated and provide an "expected EI" for each bacterial antigen or microbe tested, and can be used to correct for a leaky BBB that may be associated with a subject with MS and other demyelinating diseases. A value above the regression line, and particularly those within the normal AI region (e.g., 0-9), indicate likely intrathecal antibody synthesis (i.e. ,causality of demyelination). In some aspects, the methods disclosed herein can be used alone or in combination with any of the other methods described herein.

*Methods of diagnosing.* Disclosed herein are methods of diagnosing a demyelinating disease in a subject. In some aspects, the method can comprise obtaining a sample from the subject. In some aspects, the subject can be suspected of having or can be at risk for having a demyelinating disease. In some apsects, the method can comprise detecting the presence of one or more microbes in the sample. In some aspects, the one or more microbes in the sample can be selected from the list in Table 1, Table 2 or Table 8. In some aspects, the presence of the one or more microbes can be detected indirectly by detecting the presence of one or more antibodies in the sample. In some aspects, the one or more antibodies can be specific to the one or more microbes in the sample. In some aspects, the method can comprise diagnosing the subject with a demyelinating disease. In some aspects, the method can comprise diagnosing the subject with a demyelinating disease when the one or more microbes selected from the list in Table 1 or Table 2 are present in the sample.

In some aspects, the method of diagnosing a demyelinating disease in a subject can comprising obtaining a sample from the subject. In some aspects, the sample can be suspected of containing microbe-specific antibodies. In some aspects, the method can comprise incubating the sample with one or more microbe capture agents. In some aspects, the one or more microbe capture agents can bind to one or more of the microbe-specific antibodies. In some aspects, the microbe-specific antibodies can be specific to one or more microbes selected from the list in Table 1, Table 2 or Table 8. In some aspects, the method can comprise detecting the presence of one or more microbe-specific antibodies in the sample. In some aspects, the method can comprise diagnosing the subject with a demyelinating disease when the one or more microbe-specific antibodies are present in the sample. In some aspects, the method can further comprise repeating incubating the sample with one or more microbe capture agents step and detecting the presence of one or more microbe-specific antibodies in the sample step using different microbe capture agents. In some aspects, the different microbe capture agents can be specific to the one or more microbe-specific antibodies in the sample. In some aspects, the method can further comprise detecting the presence of one or more microbe-specific antibodies specific to one or more of the microbes listed in Table 1, Table 2 or Table 8. In some aspects, the step of detecting can comprise detecting by microarray. In some aspects, the step of detecting can comprise detecting by ELISA. In some aspects, the one or more microbes detected can be from the genus *Akkermansia.* In some aspects, the method of diagnosing can further comprise detecting the presence of one or more microbe-specific antibodies in the sample and determing an ELISA index, comparing the ELISA index to the albumin index. In some aspects, an EI ratio can be determinied. In some aspects, the EI ratio can be >1.0 indicating more microbe-specific antibodies in the CSF compared to the serum against one or more of the microbes. In some aspects, the EI ratio can be < 1.0, indicating more microbe-specific antibodies in serum than in CSF. In some aspects, the method can further comprise determining an ELISA index for one or more microbes, comparing the ELISA index to an AI of the sample by performing a linear regression analysis and generating a plot line. In some aspects, the AI can be equal to or less than 9 and the ELISA index can be above the plot line indicating one or more microbe-specific antibodies in the CSF compared to the serum for one or more microbes.

Disclosed herein are methods of diagnosing and treating a demyelinating disease in a subject. In some aspects, the method can comprising obtaining a sample from the subject. In some aspects, the sample can be suspected of containing microbe-specific antibodies. In some aspects, the method can comprise incubating the sample with one or more microbe capture agents. In some aspects, the one or more microbe capture agents can bind to one or more of the microbe-specific antibodies. In some aspects, the microbe-specific antibodies can be specific to one or more microbes selected from the list in Table 1, Table 2 or Table 8. In some aspects, the method can comprise detecting the presence of one or more microbe-specific antibodies in the sample. In some aspects, the method can comprise diagnosing the subject with a demyelinating disease. In some aspects, the method can comprise diagnosing the subject with a demyelinating disease when the one or more microbe-specific antibodies are present in the sample. In some aspects, the method can comprise administering an antibacterial agent when the one or more microbe-specific antibodies detected is a bacteria selected from the list in Table 1, Table 2 or Table 8. In some aspects, the method can comprise administering an antiviral agent when the one or more microbe-specific antibodies detected is a virus selected from the list in Table 1, Table 2 or Table 8. In some aspects, the method can comprise administering an antifungal agent when the one or more microbe-specific antibodies detected is a fungus selected from the list in Table 1, Table 2 or Table 8. In some aspects, the method can comprise administering a combination of antibacterial agent, antiviral agent, or an antifungal agent to the subject. In some aspects, the method of diagnosing and treating a demyelinating disease in a subject can further comprise detecting the presence of one or more microbe-specific antibodies in the sample and determing an ELISA index, comparing the ELISA index to the albumin index. In some aspects, an EI ratio can be determinied. In some aspects, the EI ratio can be >1.0 indicating more microbe-specific antibodies in the CSF compared to the serum against one or more of the microbes. In some aspects, the EI ratio can be < 1.0, indicating more microbe-specific antibodies in serum than in CSF. In some aspects, the method can further comprise determining an ELISA index for one or more microbes, comparing the ELISA index to the AI of the subject by performing a linear regression analysis and generating a plot line. In some aspects, the AI of the subject can be equal to or less than 9 and the ELISA index can be above the plot line indicating one or more microbe-specific antibodies in the CSF compared to the serum for one or more microbes.

*Methods of indirect detecting.* Disclosed herein are methods of detecting a demyelinating disease in a subject. In some aspects, the method of detecting a demyelinating disease in a subject can comprise obtaining a sample from the subject. In some aspects, the sample can be suspected of containing microbe-specific antibodies specific to one or more microbes selected from the list in Table 1, Table 2 or Table 8. In some aspects, the method can comprise contacting the sample with one or more microbe capture agents to generate a mixed biological sample. In some aspects, each of the one or more microbe capture agents can be specific to one or more a microbe-specific antibodies. In some aspects, the one or more microbe-specific antibodies can be specific to one or more microbes selected from the list in Table 1, Table 2 or Table 8. In some aspects, the method can comprise incubating the mixed biological sample under conditions such that the one or more microbe capture agents can specifically bind to at least one of the microbe-specific antibodies to form a detectable complex. In some aspects, the method can comprise detecting the presence of the detectable complexes, thereby detecting the presence of one or more microbes selected from the list in Table 1, Table 2 or Table 8. In some aspects, the presence of one or more microbes selected from the list in Table 1, Table 2 or Table 8 in the mixed biological sample can detect a demyelinating disease in a subject. In some aspects, the method can further comprise repeating the contacting the sample step and the incubating the mixed biological step using different microbe capture agents. In some aspects, the different microbe capture agents can be specific to the one or more microbe-specific antibodies in the sample. In some aspects, the method can further comprise detecting the presence of one or more microbe-specific antibodies specific to one or more of the microbes listed in Table 1, Table 2 or Table 8. In some aspects, the method can further comprise determining the quantity of one or more of the microbe-specific antibodies in the sample. In some aspects, the step of detecting can comprise detecting by microarray. In some aspects, the step of detecting can comprise detecting by ELISA. In some aspects, the one or more microbes detected can be from the genus *Akkermansia.* In some aspects, the method can further comprise determing an ELISA index. In some aspects, the ELISA index can be compared to or plotted against the albumin index. In some aspects, an EI ratio can be determinied. In some aspects, the EI ratio can be >1.0 indicating more microbe-specific antibodies in the CSF compared to the serum against one or more of the microbes. In some aspects, the EI ratio can be < 1.0, indicating more microbe-specific antibodies in serum than in CSF. In some aspects, the method can further comprise determining an ELISA index for one or more microbes, comparing the ELISA index to the AI of the subject by performing a linear regression analysis and generating a plot line. In some aspects, the AI of the subject can be equal to or less than 9 and the ELISA index can be above the plot line indicating one or more microbe-specific antibodies in the CSF compared to the serum for one or more microbes.

Disclosed herein are methods of detecting and treating a demyelinating disease in a subject. In some aspects, the method can comprise obtaining a sample from the subject. In some aspects, the sample can be suspected of containing microbe-specific antibodies specific to one or more microbes selected from the list in Table 1, Table 2 or Table 8. In some aspects, the method can comprise contacting the sample with one or more microbe capture agents to generate a mixed biological sample. In some aspects, each of the one or more microbe capture agents can be specific to one or more a microbe-specific antibodies. In some aspects, the one or more microbe-specific antibodies can be specific to one or more microbes selected from the list in Table 1, Table 2 or Table 8. In some aspects, the method can comprise incubating the mixed biological sample under conditions such that: said one or more microbe capture agents can specifically bind to at least one of the microbe-specific antibodies to form a detectable complex. In some aspects, the method can comprise detecting the presence of the detectable complexes, thereby detecting the presence of one or more microbes selected from the list in Table 1, Table 2 or Table 8. In some aspects, the presence of one or more microbes selected from the list in Table 1, Table 2 or Table 8 in the mixed biological sample can detect a demyelinating disease in a subject. In some aspects, the method can comprise administering an antibacterial agent when the one or more microbe-specific antibodies detected is a bacteria selected from the list in Table 1, Table 2 or Table 8. In some aspects, the method can comprise administering an antiviral agent when the one or more microbe-specific antibodies detected is a virus selected from the list in Table 1, Table 2 or Table 8. In some aspects, the method can comprise administering an antifungal agent when the one or more microbe-specific antibodies detected is a fungus selected from the list in Table 1, Table 2 or Table 8. In some aspects, the method can comprise administering a combination of antibacterial agent, antiviral agent, or an antifungal agent to the subject. In some aspects, the method can comprise administering an antiparasitic agent. In some aspects, the method can comprise administering a combination of antibacterial agent, antiviral agent, antiparasitic agent or an antifungal agent to the subject.

In some aspects, the different microbe capture agents can be specific to the one or more microbe-specific antibodies in the sample. In some aspects, the method can further comprise detecting the presence of one or more microbe-specific antibodies specific to one or more of the microbes listed in Table 1, Table 2 or Table 8. In some aspects, the method can further comprise determining the quantity of one or more of the microbe-specific antibodies in the sample. In some aspects, the step of detecting can comprise detecting by microarray. In some aspects, the step of detecting can comprise detecting by ELISA. In some aspects, the one or more microbes detected can be from the genus *Akkermansia.* In some aspects, the method can further comprise detecting the presence of one or more microbe-specific antibodies in the sample and determing an ELISA index, comparing the ELISA index to the albumin index. In some aspects, an EI ratio can be determinied. In some aspects, the EI ratio can be >1.0 indicating more microbe-specific antibodies in the CSF compared to the serum against one or more of the microbes. In some aspects, the EI ratio can be < 1.0, indicating more microbe-specific antibodies in serum than in CSF. In some aspects, the method can further comprise determining an ELISA index for one or more microbes, comparing the ELISA index to the AI of the subject by performing a linear regression analysis and generating a plot line. In some aspects, the AI of the subject can be equal to or less than 9 and the ELISA index can be above the plot line indicating one or more microbe-specific antibodies in the CSF compared to the serum for one or more microbes.

Also disclosed herein are methods of diagnosing a demyelinating disease in a subject. In some aspects, the method can comprise obtaining a sample from the subject. In some aspects, the subject can be suspected of having or can be at risk of having a demyelinating disease. In some aspects, the method can comprise detecting the presence of one or more microbes in the sample. In some aspects, the one or more microbes can be selected from the list in Table 1, Table 2 or Table 8. In some aspects, the method can comprise diagnosing the subject with a demyelinating disease when one or more microbes selected from the list in Table 1, Table 2 or Table 8 are present in the sample.

Disclosed herein are methods of detecting one or more antibodies in a sample. In some aspects, the method can comprise obtaining a sample from the subject. In some aspects, the sample can be suspected of containing microbe-specific antibodies specific to one or more microbes selected from the list in Table 1, Table 2 or Table 8. In some aspects, the method can comprise incubating the sample with one or more microbe capture agents. In some aspects, the one or more microbe capture agents can bind to one or more of the microbe-specific antibodies. In some aspects, the one or more microbe-specific antibodies can be specific to one or more microbes selected from the list in Table 1, Table 2 or Table 8. In some aspects, the method can comprise detecting the presence of one or more microbe-specific antibodies in the sample. In some aspects, the detection of the presence of one or more microbe-specific antibodies can be indicative of a demyelinating disease. In some aspects, the method can further comprise detecting the presence of one or more microbe-specific antibodies specific to one or more of the microbes listed in Table 1, Table 2 or Table 8. In some aspects, the method can further comprise determining the quantity of one or more of the microbe-specific antibodies in the sample. In some aspects, the step of detecting can comprise detecting by microarray. In some aspects, the step of detecting can comprise detecting by ELISA. In some aspects, the one or more microbes detected can be from the genus *Akkermansia.* In some aspects, the method can further comprise determing an ELISA index. In some aspects, the method can compare the ELISA index to the albumin index. In some aspects, the method can further comprise detecting the presence of one or more microbe-specific antibodies in the sample and determing an ELISA index, comparing the ELISA index to the albumin index. In some aspects, an EI ratio can be determinied. In some aspects, the EI ratio can be >1.0 indicating more microbe-specific antibodies in the CSF compared to the serum against one or more of the microbes. In some aspects, the EI ratio can be < 1.0, indicating more microbe-specific antibodies in serum than in CSF. In some aspects, the method can further comprise determining an ELISA index for one or more microbes, comparing the ELISA index to the AI of the subject by performing a linear regression analysis and generating a plot line. In some aspects, the AI of the subject can be equal to or less than 9 and the ELISA index can be above the plot line indicating one or more microbe-specific antibodies in the CSF compared to the serum for one or more microbes.

*Methods of direct detecting.* Disclosed herein are methods of detecting one or more microbes in a sample. In some aspects, the method can comprise obtaining a sample from a subject. In some aspects, the sample can be suspected of containing one or more microbes selected from the list in Table 1, Table 2 or Table 8. In some aspects, the method can comprise incubating the sample with one or more microbe capture agents. In some aspects, the one or more microbe capture agents can bind to one or more of the microbes selected from the list in Table 1, Table 2 or Table 8. In some aspects, the method can comprise detecting the presence of one or more microbes selected from the list in Table 1, Table 2 or Table 8 in the sample. In some aspects, the method can further comprise detecting the presence of one or more microbes listed in Table 1, Table 2 or Table 8. In some aspects, the method can further comprise determining the quantity of one or more of the microbes in the sample. In some aspects, detecting the presence of one or more of the microbes is indicative of a demyelinating disease. In some aspects, the step of detecting can comprise detecting by microarray. In some aspects, the step of detecting can comprise detecting by ELISA. In some aspects, the one or more microbes detected can be from the genus *Akkermansia.* In some aspects, the method can further comprise determing an ELISA index. In some aspects, the method can compare the ELISA index to the albumin index. In some aspects, the method can further comprise detecting the presence of one or more microbe-specific antibodies in the sample and determing an ELISA index, comparing the ELISA index to the AI of the subject. In some aspects, an EI ratio can be determinied. In some aspects, the EI ratio can be >1.0 indicating more microbe-specific antibodies in the CSF compared to the serum against one or more of the microbes. In some aspects, the EI ratio can be < 1.0, indicating more microbe-specific antibodies in serum than in CSF. In some aspects, the method can further comprise determining an ELISA index for one or more microbes, comparing the ELISA index to the AI of the subject by performing a linear regression analysis and generating a plot line. In some aspects, the AI of the subject can be equal to or less than 9 and the ELISA index can be above the plot line indicating one or more microbe-specific antibodies in the CSF compared to the serum for one or more microbes.

*Obtaining a tissue sample.* Procedures for the extraction and collection of a sample of a subject's brain or spinal cord tissue, blood and CSF can be done by methods known in the art. Frozen tissue specimens can also be used. As noted above, tissue samples can be obtained from the subject using core needle biopsies. The sample can be whole cells or cell organelles. Cells can be collected by scraping the tissue, processing the tissue sample to release individual cells or isolating the cells from a bodily fluid. The sample can be fresh tissue, dry tissue, cultured cells or tissue. The sample can be unfixed or fixed. Any part of the brain or spinal cord can be obtained and assessed using the methods described herein.

Disclosed herein are methods of detecting microbes within a sample. As will be appreciated by those skilled in the art, the sample solution may comprise any number of sources, including, but not limited to, bodily fluids (including, but not limited to, blood, biopsy tissue, CSF). Samples can range from less than a milliliter and can further range in bacterial concentration. Furthermore, the sample can be present in blood, brain tissue, spinal cord tissue or CSF.

In any of the methods disclosed herein, the subject can be suspected of having a demyelinating disease. In any of the methods disclosed herein, the subject can be at risk of having a demyelinating disease. In some aspects, the demyelinating disease can be multiple sclerosis, neuromyelitis optics, acute disseminated encephalomyelitis or clinically isolated syndrome. In some aspects, the subject can be a human. In some aspects, the sample can be brain tissue, cerebral spinal fluid, spinal cord tissue or blood.

In any of the methods disclosed herein, the methods can further comprise administering to the subject an antibacterial agent, an antiviral agent, an antifungal agent or a combination thereof. In some aspects, the method can comprise administering an antiparasitic agent. In some aspects, the method can comprise administering a combination of antibacterial agent, antiviral agent, antiparasitic agent or an antifungal agent to the subject. In some aspects, the antibacterial agent can be administered to the subject when the one or more microbe-specific antibodies or microbes detected can be a bacteria selected from the list in Table 1, Table 2 or Table 8. In some aspects, the antiviral agent can be administered to the subject when the one or more microbe-specific antibodies or microbes detected is a virus selected from the list in Table 1, Table 2 or Table 8. In some aspects, the antifungal agent can be administered to the subject when the one or more microbe-specific antibodies or microbes detected is a fungus selected from the list in Table 1, Table 2 or Table 8.

In any of the methods disclosed herein, the method can further comprise amplifying one or more nucleic acid sequences in the sample. In some aspects, the one or more nucleic acid sequences correspond to one or more of the microbes in Table 1, Table 2 or Table 8. In some aspects, the one or more nucleic acid sequences can be amplified using a primer pair that can specifically amplify the one or more nucleic acid sequences. In some aspects, the method can further comprise determining whether the expression of the one or more nucleic acid sequences corresponds to the one or more of the microbes in Table 1, Table 2 or Table 8 is overexpressed compared to expression levels of a nucleic acid sequence of the same one or more of the microbes in Table 1, Table 2 or Table 8 in a control. In some aspects, the detecting step can comprise detecting by a hybridization reaction. In some aspects, the hybridization reaction can further comprise hybridizing the sample to one or more primer sets. In some aspects, the hybridization reaction can be a polymerase chain reaction. In some aspects, the step of detecting can comprise detecting by expressed sequence tags. In some aspects, the step of detecting can comprise detecting by localization.

In some aspects, the microbe capture agent can be an antibody, peptide, protein or a probe. In some aspects, the one or more microbes disclosed herein (for example, see, Table 1, Table 2 and Table 8) can be used to bind to one or more of the antibodies from CSF of a subject (for example, a patient that has or is suspected of having of MS.) In some aspects, a bacterial antigen can be sonicated at an OD of 0.5 and be coated on the bottom of a single or multi-well plate. In some aspects, the bacterial antigen can be from *Akkermansia, Atopobium, Lactobacillus, Pseudomonas, Bacteroides* and *Nitrosospira.*

In any of the methods or assays or tests described herein, a positive control, a negative control, or both can be further included. Examples of a positive control include but are not limited to commercial CSF preparations (Randox) and an anti-*Pseudomonas* antibody (ThermoFisher product #PA1-73116). Examples of a negative control include but are not limited to PBS and IgG-depeted Randox CSF (which lacks all antibodies).

In some aspects, one or more methods of detection can be used to demonstrate the binding of a specific CSF antibody against one or more of the microbes disclosed herein. In some aspects, one or more microbes can be tested (and/or detected) in a subject with or suspected of having MS or other autoimmune disease. In some aspects, the method of detection can be an indirect method. In some aspects, the indirect method can be ELISA. In some aspects, two or more microbes can be tested (and/or detected) using ELISA.

*Methods of treating.* Disclosed herein are methods of treating a patient with multiple sclerosis (MS) or an MS-related disease. In some aspects, the method can comprise administering an antibacterial agent, antiviral agent, an antifungal agent, an antiparasitic agent or a combination thereof to the patient. In some aspects, the patient can be identified as needing an antibacterial agent, antiviral agent, an antifungal agent, antiparasitic agent or a combination thereof. In some aspects, the patient can be identified by: obtaining a sample from the patient, wherein the sample is suspected of containing microbe-specific antibodies; incubating the sample with one or more microbe capture agents, wherein the one or more microbe capture agents bind to one or more of the microbe-specific antibodies, and wherein the microbe-specific antibodies are specific to one or more microbes selected from the list in Table 1, Table 2 or Table 8; and detecting the presence of one or more microbe-specific antibodies in the sample. In some aspects, the antibacterial agent can be meropenem or ceftriaxone. In some aspects, the MS-related disease can be neuromyelitis optics, acute disseminated encephalomyelitis or clinically isolated syndrome. In some aspects, the step of detecting can comprise detecting by microarray. In some aspects, the step of detecting can comprise detecting by ELISA. In some aspects, wherein the one or more microbes detected can be from the genus *Akkermansia.* In some aspects, the sample can be brain tissue, cerebral spinal fluid, spinal cord tissue or blood.

In some aspects, the method can further comprise detecting the presence of one or more microbe-specific antibodies specific to one or more microbes are selected from the list in Table 1, Table 2 or Table 8. In some aspects, the method can further comprise determining the quantity of one or more of the microbe-specific antibodies in the sample. In some aspects, the method can further comprise amplifying one or more nucleic acid sequences in the sample. In some aspects, the one or more nucleic acid sequences can correspond to one or more of the microbes in Table 1, Table 2 or Table 8. In some aspects, the one or more nucleic acid sequences can be amplified using a primer pair that can specifically amplify the one or more nucleic acid sequences. In some aspects, the method can further comprise determining whether the expression of the one or more nucleic acid sequences corresponding to the one or more of the microbes in Table 1, Table 2 or Table 8 is overexpressed compared to expression levels of a nucleic acid sequence of the same one or more of the microbes in Table 1, Table 2 or Table 8 in a control. In some aspects, the step of detecting can comprise detecting by hybridization reaction. In some aspects, the hybridization reaction can further comprise hybridizing the sample to one or more primer sets. In some aspects, the step of detecting can comprise detecting by a polymerase chain reaction.

*Methods of detection.* The presence or absence or expression level of one or more microbes or microbe-specific antibodies disclosed herein can be determined directly (e.g., immunoassays, mass spectrometry) or indirectly (e.g., presence or absence of one or more antibodies that are specific to the one or more microbes; determining the mRNA expression of a protein or peptide). Examples of mass spectrometry include but are not limited to ionization sources such as EI, CI, MALDI, ESI, and analysis such as Quad, ion trap, TOF, FT or combinations thereof, spectrometry, isotope ratio mass spectrometry (IRMS), thermal ionization mass spectrometry (TIMS), spark source mass spectrometry, Multiple Reaction Monitoring (MRM) or SRM. Any of these techniques can be carried out in combination with prefractionation or enrichment methods. Examples of immunoassays include but are not limited to immunoblots, Western blots, Enzyme linked Immunosorbant Assay (ELISA), Enzyme immunoassay (EIA), radioimmune assay. Immunoassay methods use antibodies for detection and determination of levels of an antigen are known in the art. The antibody can be immobilized on a solid support such as a stick, plate, bead, microbead or array.

The presence or absence or expression level of one or more microbes or microbe-specific antibodies disclosed herein can be also be determined indirectly by determining the presence or absence of one or more antibodies that are specific to the one or more microbes in a tissue sample. RNA expression methods include but are not limited to extraction of cellular mRNA and Northern blotting using labeled probes that hybridize to transcripts encoding all or part of the gene, amplification of mRNA using gene-specific primers, polymerase chain reaction (PCR), and reverse transcriptase-polymerase chain reaction (RT-PCR), followed by quantitative detection of the gene product by a variety of methods; extraction of RNA from cells, followed by labeling, and then used to probe cDNA or olignonucleotides encoding the gene, *in situ* hybridization; and detection of a reporter gene.

Methods to measure protein expression levels include but are not limited to Western blot, immunoblot, ELISA, radioimmunoassay, immunoprecipitation, surface plasmon resonance, chemiluminescence, fluorescent polarization, phosphorescence, immunohistochemical analysis, microcytometry, microarray, microscopy, fluorescence activated cell sorting (FACS), and flow cytometry. The methods can also include specific protein property-based assays based including but not limited to enzymatic activity or interaction with other protein partners. Binding assays can also be used, and are well known in the art. For instance, a BIAcore machine can be used to determine the binding constant of a complex between two proteins. Other suitable assays for determining or detecting the binding of one protein to another include, immunoassays, such as ELISA and radioimmunoassays. Determining binding by monitoring the change in the spectroscopic can be used or optical properties of the proteins can be determined via fluorescence, UV absorption, circular dichroism, or nuclear magnetic resonance (NMR). Alternatively, immunoassays using specific antibody can be used to detect the expression on of a particular protein on a tumor cell.

The disclosed methods also provide for the quantification and/or qualification of susceptibility of a desired treatment based on the identification (or absence of such identification) of one or more microbes. Said information can lead to therapeutic decisions.

*Protein array.* Disclosed herein are polypeptide or protein arrays. In some aspects, the protein arrays can comprise probes including antibodies, aptamers, and other cognate binding ligands specific to a component of the microbe panels disclosed herein. Protein arrays and methods of constructing the protein arrays are well known to one of ordinary skill in the art.

One type of protein array that can be suitable uses an immobilized "capture antibody." The polypeptides are bound to a solid substrate (e.g., glass) with a treated surface (e.g., aminosilane) or through a biotin-streptavidin conjugation. The arrays are then incubated with a solution containing probe that can bind to the capture antibodies in a manner dependent upon time, buffer components, and recognition specificity. The probes can then be visualized directly if they have been previously labeled, or can be bound to a secondary labeled reagent (e.g., another antibody). The amount of probe bound to the capture antibody that is visualized can depend upon the labeling method utilized; generally, a CCD imager or laser scanner that uses filter sets that are appropriate to excite and detect the emissions of the label can be used. The imager converts the amount of detected photons into an electronic signal (often an 8-bit or 16-bit scale) that can be analyzed using commercially available software packages.

The substrate of the array can be organic or inorganic, biological or non-biological or any combination of these materials. The substrate can be transparent or translucent. Examples of materials suitable for use as a substrate in the array include but are not limited to silicon, silica, quartz, glass, controlled pore glass, carbon, alumina, titanium dioxide, germanium, silicon nitride, zeolites, and gallium arsenide; and metals including gold, platinum, aluminum, copper, titanium, and their alloys. Ceramics and polymers can also be used as substrates. Suitable polymers include, but are not limited to polystyrene; poly(tetra)fluorethylene; (poly)vinylidenedifluoride; polycarbonate; polymethylmethacrylate; polyvinylethylene; polyethyleneimine; poly(etherether)ketone; polyoxymethylene (POM); polyvinylphenol; polylactides; polymethacrylimide (PM I); polyalkenesulfone (PAS); polyhydroxyethylmethacrylate; polydimethylsiloxane; polyacrylamide; polyimide; co-block-polymers; and Eupergit^{®}. Photoresists, polymerized Langmuir-Blodgett films, and LIGA structures can also serve as substrates.

The array can further comprise a coating that can be formed on the substrate or applied to the substrate. The substrate can be modified with a coating by using thin-film technology based on either physical vapor deposition (PVD) or plasma-enhanced chemical vapor deposition (PECVD). Alternatively, plasma exposure can be used to directly activate the substrate. For instance, plasma etch procedures can be used to oxidize a polymeric surface (i.e., polystyrene or polyethylene to expose polar functionalities such as hydroxyls, carboxylic acids, aldehydes and the like).

The coating can comprise a metal film. Examples of metal films include but are not limited to aluminum, chromium, titanium, nickel stainless steel zinc, lead, iron, magnesium, manganese, cadmium, tungsten, cobalt, and alloys or oxides thereof. In some aspects, the metal film can be a noble metal film. Examples of noble metals that can be used for a coating include, but are not limited to, gold, platinum, silver, copper, and palladium. In some aspects, the coating comprises gold or a gold alloy. Electron-beam evaporation can be used to provide a thin coating of gold on the surface. In some aspects, the metal film can be from about 50 nm to about 500 nm in thickness.

Alternatively, the coating can be silicon, silicon oxide, silicon nitride, silicon hydride, indium tin oxide, magnesium oxide, alumina, glass, hydroxylated surfaces, and a polymer.

The arrays described herein can comprise a collection of addressable elements. Such elements can be spacially addressable, such as arrays contained within microtiter plates or printed on planar surfaces wherein each element can be present at distinct X and Y coordinates. Alternatively, elements can be addressable based on tags, beads, nanoparticles, or physical properties. The microarrays can be prepared according to the methods known to one of ordinary skill in the art. The term "arrays" as used herein can refer to any biologic assay with multiple addressable elements. In some aspects, the addressable elements can be polypeptides (e.g., antibodies or fragments thereof) or nucleic acid probes. As used herein, "elements" refer to any probe (polypeptide or nucleic acid based) that can be bound by a one or more microbes disclosed herein, polypeptide fragment or transcript encoding such polypeptides, as related or associated with any of the microbes disclosed herein. Molecules can be, but are not limited to, proteins, polypeptides, peptides, RNA, DNA, lipids, glycosylated molecules, carbohydrates, polypeptides with phosphorylation modifications, and polypeptides with citrulline modifications, aptamers, oxidated molecules, and other molecules.

For the elements described herein, "addressability" refers to the location, position, tags, cleavable tags or markers, identifiers, spectral properties, electrophoretic properties, or other physical properties that enable identification of the element. An example of addressability, also known as coding, is spatial addressability, where the position of the molecule is fixed, and that position is correlated with the identity. This type of spatial array can generally be synthesized or spotted onto a planar substrate, producing, for example, microarrays, where a large number of different molecules are densely laid out in a small area (e.g., comprising at least about 400 different sequences per cm2, and can be 1000 sequences per cm² or as many as 5000 sequences per cm², or more). Less dense arrays (e.g., ELISA or RIA plates) where wells in a plate each contain a distinct probe can comprise from about 96 sequences per plate, up to about 100 sequences per cm², up to the density of a microarray. Other spatial arrays utilize fiber optics, where distinct probes can be bound to fibers, which can be formed into a bundle for binding and analysis. Methods for the manufacture and use of spatial arrays of polypeptides are known in the art.

An alternative to this type of spatial coding array is the use of molecular "tags," where the target probes can be attached to a detectable label, or tag, which can provide coded information about the sequence of the probe. These tags can be cleaved from the element, and subsequently detected to identify the element. In some aspects, a set of probes can be synthesized or attached to a set of coded beads, wherein each bead can be linked to a distinct probe, and wherein the beads can be coded in a manner that allows identification of the attached probe. In this type of "tag array," flow cytometry can be used for detection of binding. For example, microspheres having fluorescence coding and can identify a particular microsphere. The probe can be covalently bound to a "color coded" object. A labeled target polypeptide can be detected by flow cytometry, and the coding on the microsphere can be used to identify the bound probe (e.g., immunoglobulin, antigen binding fragments of immunoglobulins, or ligands).

In some aspects, the array can be an immunoglobulin (e.g., antibody or antigen-binding fragment thereof) array. As used herein, an "immunoglobulin array" refers to a spatially separated set of discrete molecular entities capable of binding to target polypeptides arranged in a manner that allows identification of the polypeptides contained within the sample. In some aspects, the array can comprise one or more of proteins, polypeptides, peptides, RNA, DNA, lipid, glycosylated molecules, polypeptides with phosphorylation modifications, and polypeptides with citrulline modifications, aptamers, and other molecules.

### COMPOSITIONS

*Antibodies.* Disclosed herein are isolated antibodies, antibody fragments and antigen-binding fragments thereof, that can specifically bind to one or more of the microbes or microbe-specific antibodies disclosed herein. Optionally, the isolated antibodies, antibody fragments, or antigen-binding fragment thereof can be neutralizing antibodies.

As used herein, the term "antibodies" is used in a broad sense and includes both polyclonal and monoclonal antibodies. In addition to intact immunoglobulin molecules, also disclosed herein are antibody fragments or polymers of those immunoglobulin molecules, and human or humanized versions of immunoglobulin molecules or fragments thereof, as long as they are chosen for their ability to interact with the polypeptides disclosed herein. "Antibody fragments" are portions of a complete antibody. A complete antibody refers to an antibody having two complete light chains and two complete heavy chains. An antibody fragment lacks all or a portion of one or more of the chains. Examples of antibody fragments include, but are not limited to, half antibodies and fragments of half antibodies. A half antibody is composed of a single light chain and a single heavy chain. Half antibodies and half antibody fragments can be produced by reducing an antibody or antibody fragment having two light chains and two heavy chains. Such antibody fragments are referred to as reduced antibodies. Reduced antibodies have exposed and reactive sulfhydryl groups. These sulfhydryl groups can be used as reactive chemical groups or coupling of biomolecules to the antibody fragment. A preferred half antibody fragment is a F(ab). The hinge region of an antibody or antibody fragment is the region where the light chain ends and the heavy chain goes on.

Antibody fragments for use in the methods disclosed herein can bind antigens (e.g. microbe-specific antibodies or one or more of the microbes described herein). In some aspects, the antibody fragment can be specific for an antigen. An antibody or antibody fragment is specific for an antigen if it binds with significantly greater affinity to one epitope than to other epitopes. The antigen can be any molecule, compound, composition, or portion thereof to which an antibody fragment can bind. For example, the antigen can be a microbe-specific antibody or one or more of the microbes described herein. An analyte can be any molecule, compound or composition of interest. The antibodies or antibody fragments can be tested for their desired activity using the *in vitro* assays described herein, or by analogous methods.

The term "monoclonal antibody" as used herein refers to an antibody obtained from a substantially homogeneous population of antibodies, i.e., the individual antibodies within the population are identical except for possible naturally occurring mutations that may be present in a small subset of the antibody molecules. Also disclosed are "chimeric" antibodies in which a portion of the heavy or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity (See, U.S. Patent No. 4,816,567 and Morrison et al., Proc. Natl. Acad. Sci. USA, 81:6851 6855 (1984)).

Monoclonal antibodies can be made using any procedure which produces monoclonal antibodies. For example, disclosed monoclonal antibodies can be prepared using hybridoma methods, such as those described by Kohler and Milstein, Nature, 256:495 (1975). In a hybridoma method, a mouse or other appropriate host animal is typically immunized with an immunizing agent to elicit lymphocytes that produce or are capable of producing antibodies that will specifically bind to the immunizing agent.

Monoclonal antibodies may also be made by recombinant DNA methods, such as those described in U.S. Patent No. 4,816,567 (Cabilly et al.). DNA encoding the disclosed monoclonal antibodies can be readily isolated and sequenced using conventional procedures (*e.g.*, by using oligonucleotide probes that are capable of binding specifically to genes encoding the heavy and light chains of murine antibodies). Libraries of antibodies or active antibody fragments can also be generated and screened using phage display techniques, e.g., as described in U.S. Patent No. 5,804,440 to Burton et al. and U.S. Patent No. 6,096,441 to Barbas et al.

*In vitro* methods are also suitable for preparing monovalent antibodies. Digestion of antibodies to produce fragments thereof, such as an Fv, Fab, Fab', or other antigen binding portion of an antibody, can be accomplished using routine techniques known in the art. For example, digestion can be performed using papain. Examples of papain digestion are described in WO 94/29348 published Dec. 22, 1994 and U.S. Patent No. 4,342,566, the contents of which are hereby incorporated by reference in its entirety for its teaching of papain digestion of antibodies to prepare monovaltent antibodies. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

Fragments, whether attached to other sequences, can also include insertions, deletions, substitutions, or other selected modifications of particular regions or specific amino acid residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment. (Zoller, M.J. Curr. Opin. Biotechnol. 3:348-354, 1992).

As used herein, the term "antibody" or "antibodies" can also refer to a human antibody or a humanized antibody. Many non-human antibodies (e.g., those derived from mice, rats, or rabbits) are naturally antigenic in humans, and thus can give rise to undesirable immune responses when administered to humans. Therefore, the use of human or humanized antibodies in the methods serves to lessen the chance that an antibody administered to a human will evoke an undesirable immune response.

Human antibodies can be prepared using any technique. Examples of techniques for human monoclonal antibody production include those described by Cole et al. (Monoclonal Antibodies and Cancer Therapy, Alan R. Liss, p. 77, 1985) and by Boemer et al. (J. Immunol., 147(1):86 95, 1991). Human antibodies (and fragments thereof) can also be produced using phage display libraries (Hoogenboom et al., J. Mol. Biol., 227:381, 1991; Marks et al., J. Mol. Biol., 222:581, 1991).

Human antibodies can also be obtained from transgenic animals. For example, transgenic, mutant mice that are capable of producing a full repertoire of human antibodies, in response to immunization, have been described (see, e.g., Jakobovits et al., Proc. Natl. Acad. Sci. USA, 90:2551-255 (1993); Jakobovits et al., Nature, 362:255 258 (1993); Bruggermann et al., Year in Immunol., 7:33 (1993)). Specifically, the homozygous deletion of the antibody heavy chain joining region (J(H)) gene in these chimeric and germline mutant mice results in complete inhibition of endogenous antibody production, and the successful transfer of the human germ line antibody gene array into such germ line mutant mice results in the production of human antibodies upon antigen challenge. Antibodies having the desired activity are selected using Env-CD4-co-receptor complexes as described herein.

Optionally, human antibodies can be made from memory B cells using a method for Epstein-Barr virus transformation of human B cells. (See, e.g., Triaggiai et al., An efficient method to make human monoclonal antibodies from memory B cells: potent neutralization of SARS coronavirus, Nat Med. 2004 Aug; 10(8):871-5. (2004)), which is herein incorporated by reference in its entirety for its teaching of a method to make human monoclonal antibodies from memory B cells). In short, memory B cells from a subject who has survived a natural infection are isolated and immortalized with EBV in the presence of irradiated mononuclear cells and a CpG oligonuleotide that acts as a polyclonal activator of memory B cells. The memory B cells are cultured and analyzed for the presence of specific antibodies. EBV-B cells from the culture producing the antibodies of the desired specificity are then cloned by limiting dilution in the presence of irradiated mononuclear cells, with the addition of CpG 2006 to increase cloning efficiency, and cultured. After culture of the EBV-B cells, monoclonal antibodies can be isolated. Such a method offers (1) antibodies that are produced by immortalization of memory B lymphocytes which are stable over a lifetime and can easily be isolated from peripheral blood and (2) the antibodies isolated from a primed natural host who has survived a natural infection, thus eliminating the need for immunization of experimental animals, which may show different susceptibility and, therefore, different immune responses.

Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an Fv, Fab, Fab', or other antigen binding portion of an antibody) which contains a portion of an antigen binding site from a non-human (donor) antibody integrated into the framework of a human (recipient) antibody.

To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones et al., Nature, 321:522-525 (1986), Reichmann et al., Nature, 332:323 327 (1988), and Presta, Curr. Opin. Struct. Biol., 2:593-596 (1992)).

Methods for humanizing non-human antibodies are well known in the art. For example, humanized antibodies can be generated according to the methods of Winter and co-workers (Jones et al., Nature, 321:522 525 (1986), Riechmann et al., Nature, 332:323-327 (1988), Verhoeyen et al., Science, 239:1534 1536 (1988)), by substituting rodent CDRs or CDR sequences for the corresponding sequences of a human antibody. Methods that can be used to produce humanized antibodies are also described in U.S. Patent No. 4,816,567 (Cabilly et al.), U.S. Patent No. 5,565,332 (Hoogenboom et al.), U.S. Patent No. 5,721,367 (Kay et al.), U.S. Patent No. 5,837,243 (Deo et al.), U.S. Patent No. 5, 939,598 (Kucherlapati et al.), U.S. Patent No. 6,130,364 (Jakobovits et al.), and U.S. Patent No. 6,180,377 (Morgan et al.). The antibodies disclosed herein can also be administered to a subject. Nucleic acid approaches for antibody delivery also exist. The broadly neutralizing antibodies to the polypeptides disclosed herein and antibody fragments can also be administered to subjects or subjects as a nucleic acid preparation (e.g., DNA or RNA) that encodes the antibody or antibody fragment, such that the subject's own cells take up the nucleic acid, and produce and secrete the encoded antibody or antibody fragment.

### KITS

In some aspects, a kit is disclosed comprising one or more probes or primers capable of detecting, amplifying or measuring the presence or expression of one or more microbes disclosed herein. Also disclosed are kits comprising one or more of the microbe capture agents, microbes or microbe-specific antibodies disclosed herein.

Disclosed herein, are solid supports comprising one or more primers, probes, polypeptides, or antibodies capable of hybridizing or binding to one or more of the microbes described herein. Solid supports are solid state substrates or supports that molecules, such as analytes and analyte binding molecules, can be associated. Analytes (e.g., calcifying nano-particles and proteins) can be associated with solid supports directly or indirectly. For example, analytes can be directly immobilized on solid supports. Analyte capture agents (e.g., capture compounds) can also be immobilized on solid supports.

As mentioned above, one of ordinary skill in the art can determine the presence of expression level of one or more microbes or microbe-specific antibodies (proteins or nucleic acids) disclosed herein any number of ways. To detect or quantify the level of RNA products of the biomarkers within a sample, arrays, such as microarrays, RT-PCR (including quantitative RT-PCR), nuclease protection assays and Northern blot analyses can be used. Accordingly, in some aspects, the expression levels of one or more of the microbes can be determined using arrays, microarrays, RT-PCR, quantitative RT-PCR, nuclease protection assays or Northern blot analyses.

An array is a form of solid support. An array detector is also a form of solid support to which multiple different capture compounds or detection compounds have been coupled in an array, grid, or other organized pattern.

Solid-state substrates for use in solid supports can include, for instance, any solid material to which molecules can be coupled. Examples of such materials include but are not limited to acrylamide, agarose, cellulose, nitrocellulose, glass, polystyrene, polyethylene vinyl acetate, polypropylene, polymethacrylate, polyethylene, polyethylene oxide, polysilicates, polycarbonates, teflon, fluorocarbons, nylon, silicon rubber, polyanhydrides, polyglycolic acid, poly lactic acid, polyorthoesters, polypropylfumerate, collagen, glycosaminoglycans, and polyamino acids. Solid-state substrates can have any useful form including thin film, membrane, bottles, dishes, fibers, woven fibers, shaped polymers, particles, beads, microparticles, or any combination thereof. Solid-state substrates and solid supports can be porous or non-porous. An example of a solid-state substrate is a microtiter dish (e.g., a standard 96-well type). A multiwell glass slide can also be used. For example, such as one containing one array per well can be used, allowing for greater control of assay reproducibility, increased throughput and sample handling, and ease of automation.

Different compounds can be used together as a set. The set can be used as a mixture of all or subsets of the compounds (e.g., microbe capture agents or microbe-specific antibodies) used separately in separate reactions, or immobilized in an array. Compounds used separately or as mixtures can be physically separable through, for example, association with or immobilization on a solid support. An array can include a plurality of compounds immobilized at identified or predefined locations on the array. Each predefined location on the array can generally have one type of component (that is, all the components at that location are the same). Each location can have multiple copies of the component. The spatial separation of different components in the array allows separate detection and identification of the polynucleotides or polypeptides disclosed herein.

It is not required that a given array be a single unit or structure. The set of compounds can be distributed over any number of solid supports. For example, each compound can be immobilized in a separate reaction tube or container, or on separate beads or microparticles. Different aspects of the disclosed method and use of the gene expression panel or array or diagnostic device can be performed with different components (e.g., different compounds specific for different proteins) immobilized on a solid support.

Some solid supports can have capture compounds, such as antibodies, attached to a solid-state substrate. Such capture compounds can be specific for calcifying nanoparticles or a protein on calcifying nanoparticles. Captured calcified nanoparticles or proteins can then be detected by binding of a second detection compound, such as an antibody. The detection compound can be specific for the same or a different protein on the calcifying nanoparticle.

Methods for immobilizing nucleic acids, peptides or antibodies (and other proteins) to solid-state substrates are well established. Immobilization can be accomplished by attachment, for example, to aminated surfaces, carboxylated surfaces or hydroxylated surfaces using standard immobilization chemistries. Examples of attachment agents include but are not limited to cyanogen bromide, succinimide, aldehydes, tosyl chloride, avidinbiotin, photocrosslinkable agents, epoxides, maleimides and N-[y-Maleimidobutyryloxy] succinimide ester (GMBS), and a heterobifunctional crosslinker. Antibodies can be attached to a substrate by chemically cross-linking a free amino group on the antibody to reactive side groups present within the solid-state substrate. Antibodies can be, for example, chemically cross-linked to a substrate that contains free amino, carboxyl, or sulfur groups using glutaraldehyde, carbodiimides, or GMBS, respectively, as cross-linker agents. In this method, aqueous solutions containing free antibodies can be incubated with the solid-state substrate in the presence of glutaraldehyde or carbodiimide.

A method for attaching antibodies or other proteins to a solid-state substrate is to functionalize the substrate with an amino- or thiol-silane, and then to activate the functionalized substrate with a homobifunctional cross-linker agent such as (Bis-sulfo-succinimidyl suberate (BS3) or a heterobifunctional cross-linker agent such as GMBS. For crosslinking with GMBS, glass substrates can be chemically functionalized by immersing in a solution of mercaptopropyltrimethoxysilane (1% vol/vol in 95% ethanol pH 5.5) for 1 hour, rinsing in 95% ethanol and heating at 120°C for 4 hrs. Thiol-derivatized slides can be activated by immersing in a 0.5 mg/ml solution of GMBS in 1% dimethylformamide, 99% ethanol for 1 hour at room temperature. Antibodies or proteins can be added directly to the activated substrate, which can be blocked with solutions containing agents such as 2% bovine serum albumin, and air-dried. Other standard immobilization chemistries are known by those of ordinary skill in the art.

Each of the components (e.g., antibodies) immobilized on the solid support can be located in a different predefined region of the solid support. Each of the different predefined regions can be physically separated from each other. The distance between the different predefined regions of the solid support can be either fixed or variable. For example, in an array, each of the components can be arranged at fixed distances from each other, while components associated with beads will not be in a fixed spatial relationship. The use of multiple solid support units (e.g., multiple beads) can result in variable distances.

Components can be associated or immobilized on a solid support at any density. Components can be immobilized to the solid support at a density exceeding 400 different components per cubic centimeter. Arrays of components can have any number of components. For example, an array can have at least 1,000 different components immobilized on the solid support, at least 10,000 different components immobilized on the solid support, at least 100,000 different components immobilized on the solid support, or at least 1,000,000 different components immobilized on the solid support.

In addition, the microbes described herein can also be used as markers (i.e., biomarkers) for susceptibility to or presence or progression of a dymyelinating disease. The methods and assays described herein can be performed over time, and the change in the level of the markers assessed. For example, the assays can be performed every 24-72 hours for a period of 6 months to 1 year, and thereafter carried out as needed. Assays can also be completed prior to, during, or after a treatment protocol. Together, the microbes or microbe-specific antibodies disclosed herein can be used to profile an individual's risk or progression of a demyelinating disease. As used within this context, the terms "differentially expressed" or "differential expression" refers to difference in the level of expression of the biomarkers disclosed herein that can be assayed by measuring the level of expression of the products (e.g., RNA or gene product) of the biomarkers, such as the difference in level of messenger RNA transcript or a portion thereof expressed or of proteins expressed of the biomarkers. In some aspects, this difference is significantly different.

To improve sensitivity, more than one microbe disclosed herein can be assayed within a given sample. Binding agents specific for different proteins, antibodies, nucleic acids provided herein can be combined within a single assay. Further, multiple primers or probes can be used concurrently. To assist with such assays, specific biomarkers can assist in the specificity of such tests.

In general, disclosed herein are methods for the identification (including diagnosis) of microbes (e.g., microorganisms) and microbial infections (including polymicrobial infections) in patients. There are a variety of methods used for identification of different microbes within the samples, e.g., providing specificity. In some aspects, a plurality of detection surfaces can be used. In some aspects, each detection surface can have a different specific microbe capture agents. That is, one detection surface may include microbe capture agents comprising antibodies to specific microbial species or genera, and another a different microbe capture agents to a different specific species. In some aspects, a plurality of detection surfaces can be used that are fluidically separated from one another; for example, a plurality of detection modules, for example detection channels, wherein one sample can be divided into the detection modules and can then be subjected to different conditions, e.g., different microbe capture agents, for evaluation. As outlined herein, the plurality of different detection surfaces can have non-specific, or specific microbe capture agents.

In some aspects, the detection surface(s) can rely on non-specific capture of the microbes or microbe-specific antibodies, but the detection method can rely on specific binding ligands; e.g., microbe-specific antibodies to a specific species or genera of microbe can be used with a fluorescent label. In some aspects, simultaneous detection usually relies on different binding ligands containing different labels, while sequential detection can be done using one or more washing steps followed by a different binding ligand with the same label. Another aspect of the invention avoids the use of either specific capture or specific labeling. In some aspects, the method provides for specific identification of a microbe using spatial separation of the microbes on the detection surface based on detectable or known changes. For example, the ability to detect the division of single microorganisms allows identification on the basis of any number of parameters, particularly kinetic parameters, including but not limited to growth rates, assessment of metabolic activity, rate of cell kill with different antibiotics, as well as microorganism morphology, which can include size, shape, and relationships to sibling organisms (e.g., growth into clusters or chains, two-dimensional growth on the surface or three-dimensional growth away from the surface). In addition to the evaluation of rates, single data point analysis may also be done (e.g., increased area associated with an individual microbes on the surface (e.g., positive growth), stagnant area (no positive growth) or loss of area (e.g., negative growth, apoptosis and/or death).

In some aspects, the invention provides a solid support or a kit for detecting a demyelinating disease in subject. In some aspects, the subject can be suspected of having or is at risk for having a demyelinating disease. In some aspects, the invention provides a solid support or a kit for detecting one or more antibodies in a sample. In some aspects, the detection of the presence of one or more microbe-specific antibodies can be indicative of a demyelinating disease. In some aspects, the invention provides a solid support or a kit for detecting one or more microbes in a sample. In some aspects, the sample can be from a subject at risk of developing or is suspected of having a demyelinating disease. The solid support or kit can include nucleic acid probes, antibodies, microbe-specific antibodies, primers, and/or microbe capture agents that can be useful for determining the presence of one or more microbes selected from the list in Table 1, Table 2 or Table 8.

The kit can also comprise reagents that can be used for various auxiliary substances so that the kit can be used easily and efficiently, e.g., solvents, wash buffers etc.

### EXAMPLES

### Example 1: Spectrum of Microbial Sequences and a Bacterial Cell Wall Antigen in Primary Demyelination Brain Specimens Obtained from Living Patients

Abstract: Multiple sclerosis (MS) is an autoimmune disease characterized by multiple lesions in the brain and spinal cord. RNA sequencing was used to identify microbial sequences and characterize human gene expression patterns in 30 human brain biopsy specimens. RNAs which aligned to known microbial taxa, were significantly enriched in 10 of 12 primary demyelination (MS) brain specimens compared to a group of 15 epilepsy controls, leading to a list of 29 MS microbes or MS microbial families genera from 11 different phyla. Most of the MS microbes described herein are anaerobic bacteria. While there were some shared MS microbes or MS microbial families, each of the 10 MS samples with significant microbial RNA enrichment had a distinct set of MS microbes or MS microbial families. The fraction of microbial sequencing reads was greater for the MS group (128.8 PPM) compared to the controls (77.4 PPM, p = 0.016). Bacterial peptidoglycan was demonstrated in brain tissue sections from several MS subjects. Human gene expression analysis showed increased expression of inflammation-related pathways in the MS group. This data shows that demyelinating brain lesions are associated with the presence of microbial RNA sequences and bacterial antigen. This suggests that MS is triggered by the presence of a diverse set of microbes within a lesion.

Materials and Methods: *Subjects.* Characteristics of the study subjects are shown in Table 3. Twelve brain biopsy samples were chosen for sequencing from 11 subjects. Biopsies from 10 of these 11 subjects had pathology showing demyelination. Another subject (MS-062) had well established progressive MS. These 11 subjects are designated, for clarity and brevity, as the MS Group. (One subject, MS-021 was biopsied twice over the course of 3 months.) Findings on the brain biopsies were reviewed by a neuropathologist. The disease courses of the MS subjects ranged from a single demyelinating episode to severe fulminant disease (Marburg or tumefactive disease). Clinical information was compiled from electronic records, paper records, and discussions with treating physicians. Clinical diagnoses and imaging findings were reviewed by an MS neurologist. Three subjects had brain biopsy pathology that showed some other process, designated as Other Neurologic Disease (OND). The Control Group consists of tissue taken from 15 subjects with epilepsy who had some brain tissue removed for control of their seizures. Specimens from the MS and OND subjects were white matter while those from the epilepsy control subjects were primarily from cerebral cortex (gray matter).

*Specimen Preparation, RNA Extractions, and Library Preparation.* The brain biopsy blocks were obtained. Multiple procedures were used to prevent exogenous contamination of the samples: a dedicated microtome was used with a fresh blade for each specimen, the bench area was cleaned and decontaminated between processing of specimens, the top 50 microns of tissue was discarded, sterile disposable instruments were used to handle the specimens, and the microtome was cleaned with alcohol and bleach between specimens. Five sections of 10 µm thickness were collected for RNA extractions.

RNA extraction was performed on the brain biopsies using the Qiagen FFPE RNA Kit, which includes a DNAse step. RNA from 12 of these MS brain biopsy specimens passed quality control and have been sequenced on the Illumina HiSeq 2500 platform in two separate runs. Ten of the 12 sequenced MS samples are from female subjects. One of the female subjects (subject 021) was biopsied twice, 3 months apart. To enrich the samples for microbial sequence, human rRNA was physically depleted from the samples using RiboZero (Illumina Catalog # MRZH116). For sequencing, non-directional complementary DNA libraries were constructed and paired-end 125bp sequencing on a HiSeq-2500 was performed. Fifteen control epilepsy FFPE samples were processed and sequenced in parallel using the same equipment and procedures. Two chronic encephalitis and one anoxic brain injury samples were sequenced to serve as other neurologic disease (OND) controls. Two blank specimens, defined as no visible tissue (i.e., paraffin) and <10% of the sample's reads mapping to human genome or transcriptome, were sequenced and used in the analysis. The RNA-seq yielded 125 bp paired-end reads.

*Sequencing and Quality Control.* Quality control of the paired 125 bp reads was performed with the Sickle program. Low-quality pairs were removed and terminal low-quality base calls were trimmed (Sickle: A sliding-window, adaptive, quality-based trimming tool for FastQ files v. 1.33 (2011). The Sickle parameters were set to discard read-pairs when either member of the pair was trimmed to <40 bp in length. The HQ read-pairs for each sample were aligned to several databases using Bowtie2 (settings: -q --k1 -phred33local) (Langmead, B. & Salzberg, S. L. Fast gapped-read alignment with Bowtie 2. Nat Meth 9, 357-359, doi:10.1038/nmeth.1923 http://www.nature.com/nmeth/journal/v9/n4/abs/nmeth.1923.html - supplementary-information (2012)). Reads which aligned to the human genome and/or human transcriptome (GRCh37 assembly) were analyzed separately for host gene expression differences. The remaining nonhuman reads were then aligned to a panmicrobial database compiled in the Fischer Lab. The panmicrobial database includes a nonredundant viral database (including complete and partial virus sequences), complete bacterial, archaea, fungal, and protist genomes in GenBank. This 11 Gb panmicrobial database contains more than 1.3 million sequence records, each identified by a GenBank identifier (gi), representing 10,654 species.Fischer (Fischer, K. F. The Panmicrobial Database, <http://pathogenomics.path.utah.edu/sequences/panmicrobial_nrdb.fasta.zip> (2017)). Microbial alignments were also performed with Bowtie2 (setting: --end-to-end -phred33). Read-pairs aligning to the same microbial sequence (concordant pairs) were counted and carried forward in the analysis.

Since the RNA-seq did not yield exactly the same number of HQ pairs in each sample, the number of reads from a sample that align to each sequence in panmicrobial database were normalized by dividing by the number of reads in high quality pairs (in millions), yielding Pairs Per Million (PPM). These normalized hit rates (HR) were calculated for each microbial taxon and sample. Taxa PPM were aggregated, where possible, and analyzed at the genus and family levels. The data from 2 sequencing runs (Run 1 and Run 2) were combined and analyzed. A Run 2 specific artifact was observed with many more reads aligning to the phylum Proteobacteria than in Run 1. Taxa affected by this artifact were excluded from the HR analysis by comparing the HRs of the Run 2 blank (no tissue) specimens to the HRs of the Run 1 control samples. Then taxa with a HR in one or both blanks greater than the mean HR of that taxa in the controls from sequencing Run 1 were excluded. The blanks were separate FFPE brain biopsy specimens from enrolled subjects that included no visible tissue.

*Human Gene Expression.* Bowtie (v2.2.5.0), tophat (v2.0.14) and cuff diff (v2.2.1) were used to calculate differential expression levels of known splice variants in the demyelination and control sample groups (Trapnell, C. et al. Nat Biotech 28, 511-515, (2010); and Trapnell, C. et al. Differential analysis of gene regulation at transcript resolution with RNA-seq. Nat Biotech 31, 46-53, (2013). Tophat was run with the '-no-novel-juncs' setting. Cuffdiff was run on the tophat output 'accepted_hits.bam' files for each sample. Controlling the false discovery rate (FDR) < 0.05, differentially expressed transcripts were identified between the groups. The transcriptome model used was derived from GRCh37 Ensembl release 75, May 23 2014. Pathway enrichment of the differentially expressed genes was calculated with Cytoscape and the Reactome Curated Pathway Database (Fabregat, A. et al. Nucleic Acids Res 44, D481-487, (2016)).

*Statistical Analysis.* Demographics and characteristics of the study population were compared using ANOVA, then confirmed with Fishers Exact test for discrete variables (e.g., sex), or the Mann-Whitney nonparametric test (e.g., age, collection year) (Lowry, R. VassarStats: Website for Statistical Computation, <http://vassarstats.net/>). A list of microbes for each MS brain sample was derived by looking for significant outliers within the dataset. HRs from each taxon for each sample were log₂ transformed. Z-scores were calculated for every MS sample using the 15 control samples as the expected distribution. The analysis was performed at the family and genus levels. Z-scores were converted to p-values using the normal distribution. Since microbial sequences that are over-, not under-represented in the MS group was of interest, one-tailed testing was performed. To correct for multiple comparisons, p-values were converted to q-values by the false-discovery rate control method of Benjamini-Hochberg (Benjamini, Y. & Hochberg, Y. Journal of the Royal Statistical Society. Series B (Methodological) 57, 289-300 (1995)). MS microbes or MS microbial families were defined as those significantly (q < 0.05) over-represented in at least one MS sample compared to the set of 15 controls, *and* with HR > 1.0 PPM. Python 2.7 was used to calculate HR-based significance. HR normalization, transformation and Z-score calculation was performed using numpy and pandas; p- and q- values were calculated with scipy.stats (Oliphant, T. E. (2006); McKinney, W. in SciPy 2010*; and* SciPy: Open source scientific tools for Python (2001)).

*Immunohistochemistry.* Five micron pathologic brain tissue sections from subjects MS-019 and MS-056, epilepsy controls 039 and 040, and brain abscess (positive control) were studied. The tissue sections were processed using antigen unmasking (Vector Laboratories, Product H3300) followed by 0.5% casein blocking (Sigma). The sections were incubated with anti-peptidoglycan IgG1 mAb (EMD Millipore product MAB995, Temecula, CA), anti-CD68 (macrophages, ab955, Abeam, Cambridge, MA), anti-lysozyme (macrophages and neutrophils, ab108508, Abcam, Cambridge, MA), or a mouse isotype control Ab (IgG1 isotype control, Invitrogen, Catalog Number MA5-14453) overnight at 4 degrees. An additional anti-peptidoglycan monoclonal Ab (mAb 2E9) was provided. 2E9 is an IgG3 mAb originally developed in 1994 as a reagent for the detection of intestinal flora-derived bacterial antigen in splenic macrophages (Kool, J. et al. Journal of Histochemistry & Cytochemistry 42, 1435-1441, (1994)). This mAb has also been used to show peptidoglycan in brain tissue (Schrijver, I. A. et al. Brain 124, 1544-1554 (2001); and Branton, W. G. et al. Scientific reports 6, 37344, (2016)). The sections were developed with biotinylated anti-mouse IgG Antibody (BA-2000, Vector, California, USA) and streptavidin-peroxidase (S5512, Sigma, St. St. Louis, MO, USA). Imaging was performed on a Zeiss Axioplan microscope.

Results: *Study Population.* The characteristics of the study population are shown in Table 3. Age and sex distributions did not differ significantly between the groups. The brain specimen collections were performed somewhat later in the Control group (median 2012) compared with the MS group (median 2007, p < 0.001). This is attributed to the abundance of primary demyelination specimens (relatively rare) compared with epilepsy surgical controls (more common), leading to a situation where the more recent epilepsy control specimens were enrolled. The Control group had significantly more surgical procedures (median 2) in the month preceding the brain specimen collection compared to the MS group (median 1, p = 0.005). The larger number of surgical procedures is due to mapping procedures performed in the controls prior to excision of the epileptogenic focus.

*Characteristics of the Brain Specimens.* Brain specimens used in this study were formalin fixed and paraffin-embedded FFPE. Biopsy sites included a mix of white and gray matter in the MS and other neurologic disease (OND) groups. The control biopsies were taken primarily from cortex (gray matter). Ten of the 12 biopsies from the MS group had neuropathology readings of demyelination consistent with MS. One of the subjects (MS-021) was biopsied twice because the first specimen was nondiagnostic (i.e., normal brain). Another subject (MS-062) with well-established progressive MS had a biopsy consisting primarily of gray matter that showed perivascular inflammation. The 3 subjects in the OND group were initially suspected to have MS based on radiology and clinical findings, but were later reclassified based on the neuropathology of their specimens (i.e. *not* primary demyelination).

*Clinical Findings in the MS and OND Groups.* Six of the 11 subjects in the MS group had oligoclonal band (OCB) testing in their record. Among these, 4 were positive and 2 were negative. One OND subject (OND-003) also had OCB testing which was positive, despite the ultimate diagnoses of acute disseminated encephalomyelitis (ADEM) and encephalitis, not MS. The 11 subjects in the MS group had disease consistent with multiple sclerosis and 6 received disease modifying therapy (Table 3). There is one fewer MS subject than MS samples because one of the subjects (MS-21) had two brain biopsies performed several months apart.

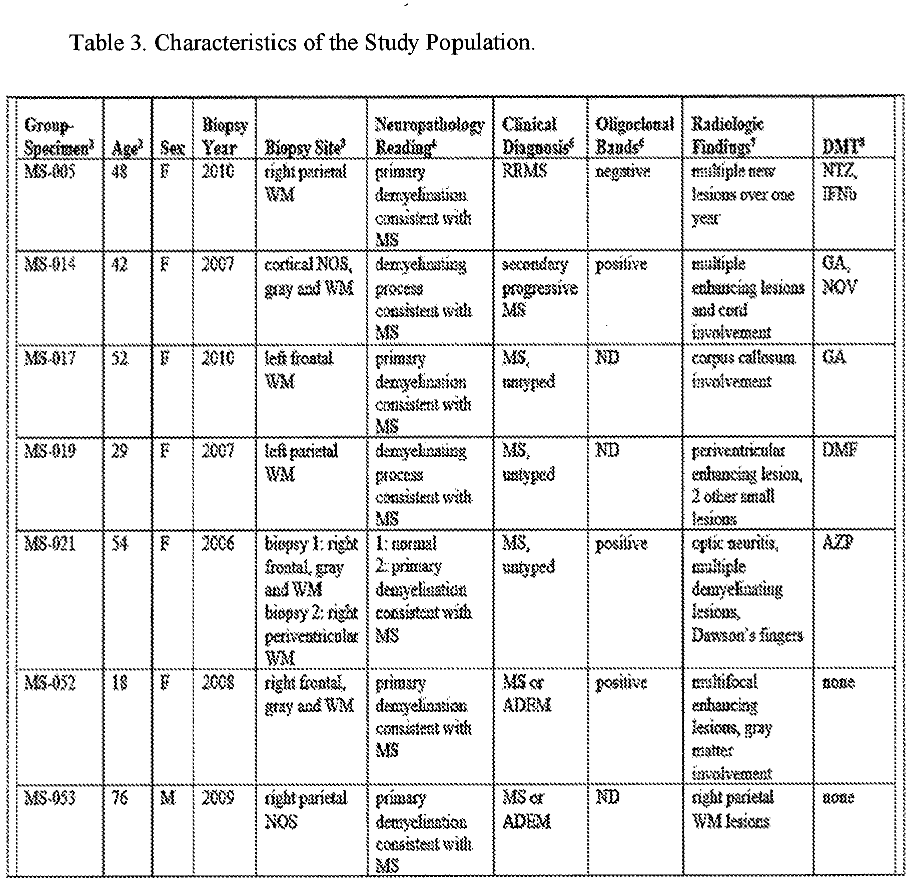

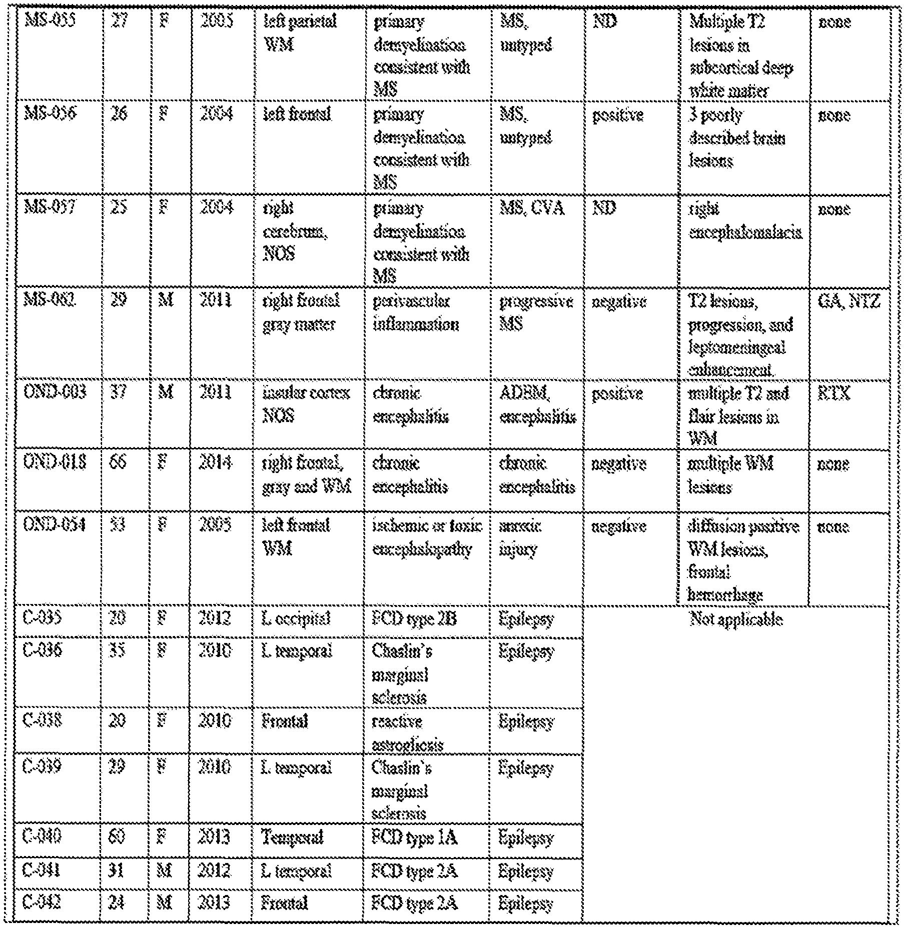

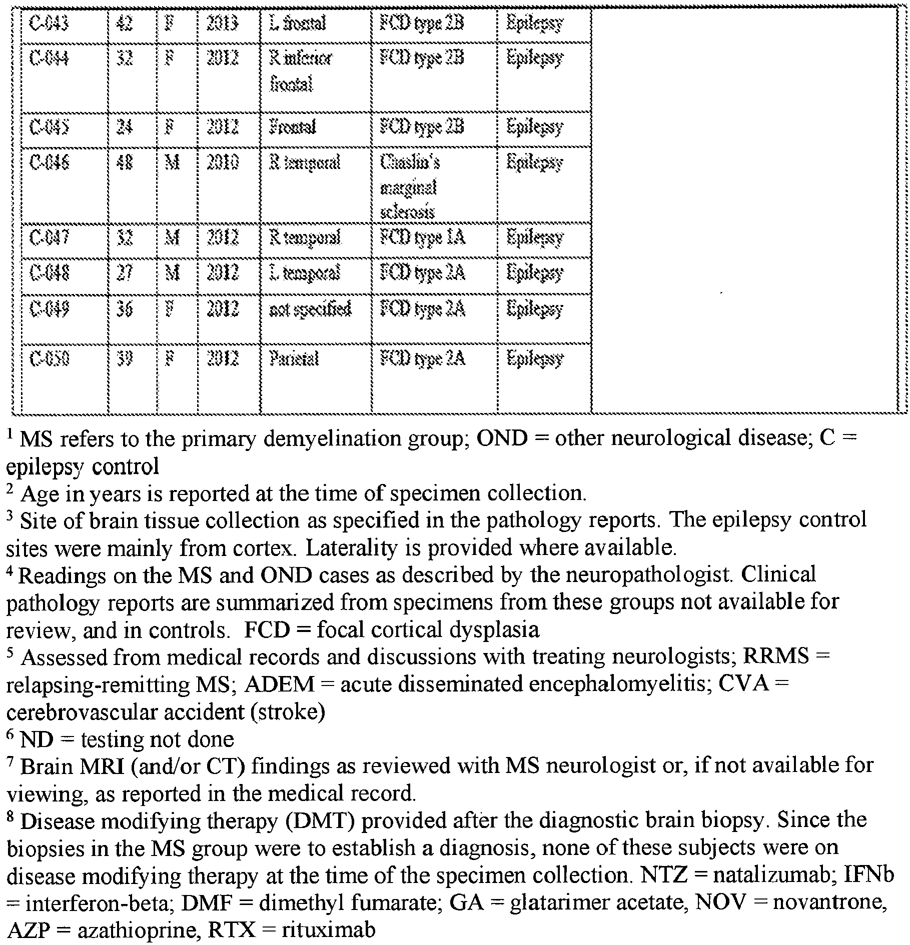

*Sequencing and Alignments.* Among the 32 samples (MS 12, Control 15, OND 3, Blank 2), the total sequencing yield was 1.06 - 2.96 × 10⁸ high-quality read pairs (HQ pairs). There were no significant differences in the number of HQ pairs between the sample groups. Overall, the quality of the sequencing was high with 2.6% of the original unfiltered read-pairs discarded from the dataset. The remaining HQ pairs were used for the microbial and human database alignments. Most of the HQ pairs (95.7%) were full length (125 bp). The mean length of the remaining trimmed reads (4.3% of the HQ pairs) was 123.9 bp.

Alignments to the human and microbial databases are described in Table 4. Reads that aligned to the human genome were excluded from the microbial analysis. A total of 216,159 concordantly aligning pairs from the 30 experimental samples mapped with high quality and specificity to single sequences in the panmicrobial database (Table 5). Most of these microbial reads were bacterial. They mapped to predominantly rRNA sequences from the phyla Proteobacteria (50.3%), Actinobacteria (20.3%), Firmicutes (16.2%), Bacteroidetes (4.6%), or other (8.5%). The fraction of microbial reads was greater for the MS group (128.8 PPM) compared to controls (77.4 PPM, p = 0.016).

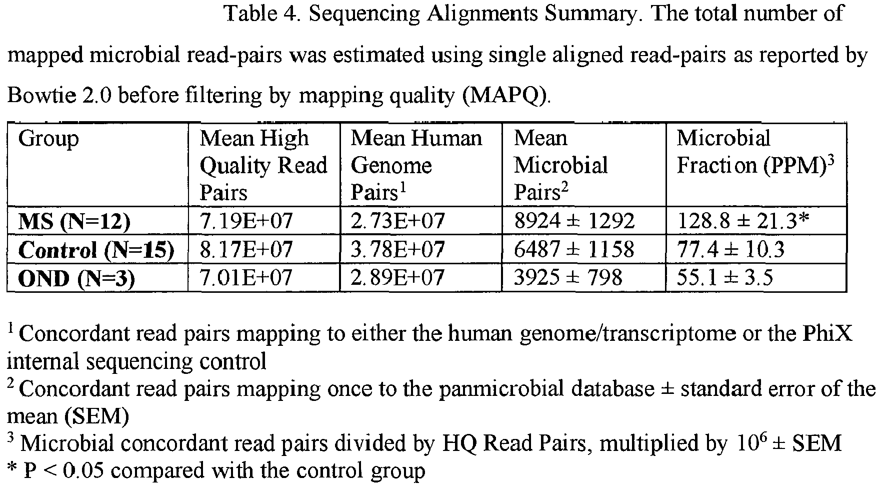

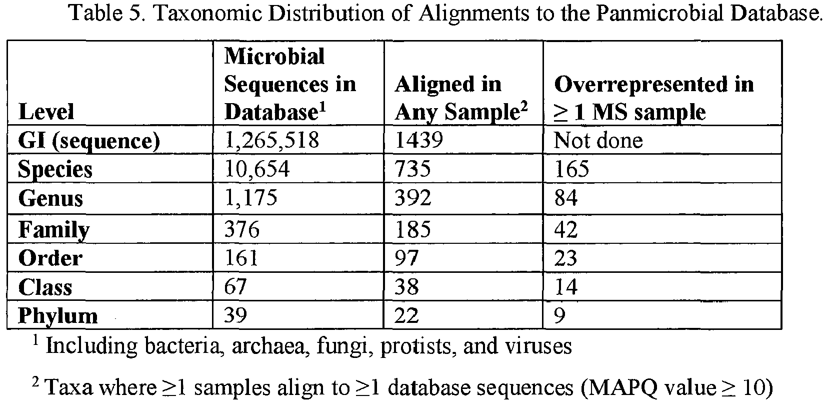

To determine the specific identifies of microbial sequences overrepresented in the MS group, additional filtering was performed using the MAPQ metric, a measure of the specificity of alignment to sequences in the database (Trapnell, C. et al. Nat Biotech 28, 511-515, (2010); and Trapnell, C. et al. Nat Biotech 31, 46-53, (2013)). Human reads, PhiX control reads, and reads where MAPQ <10 were excluded from further analysis. The remaining alignments have a >90% probability of the reported mapping being to the single best sequence match in the panmicrobial database. Where there is a concordant pair, the probability of a correct mapping is > 99%. Significant overrepresentation (q < 0.05) of microbial sequence in at least one of the MS samples was seen for 43 families and 84 genera. The family-level overrepresentation is depicted in Figure 1. The genus-level overrepresentation is shown in Table 6.

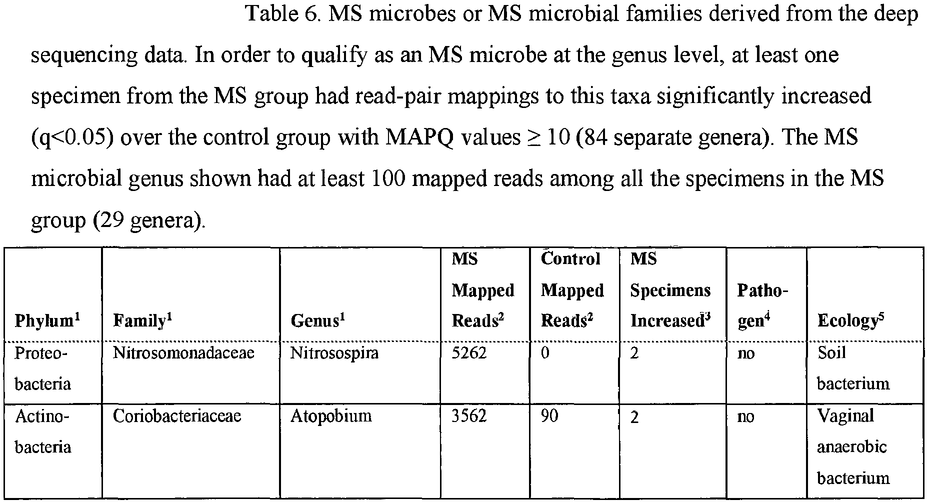

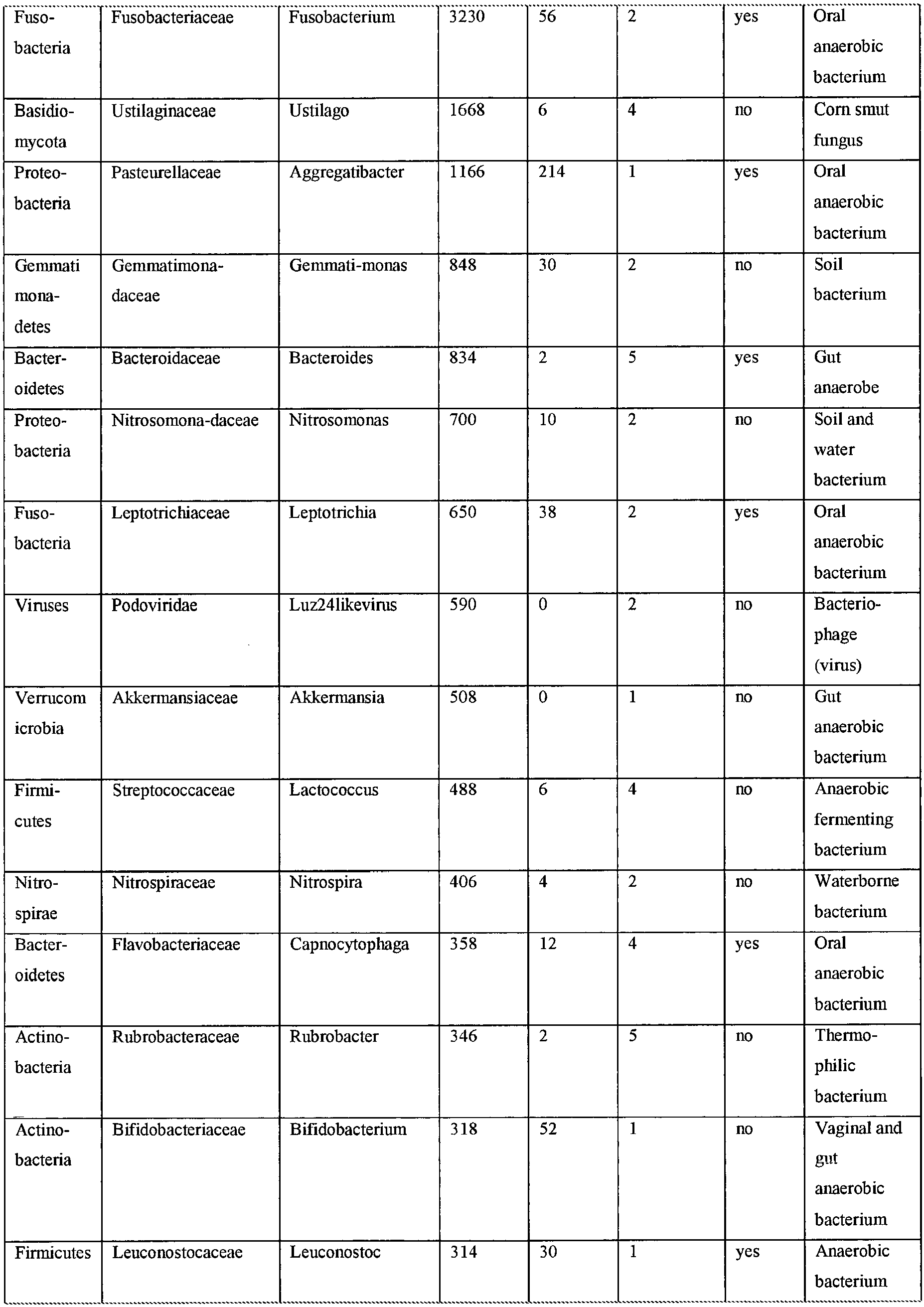

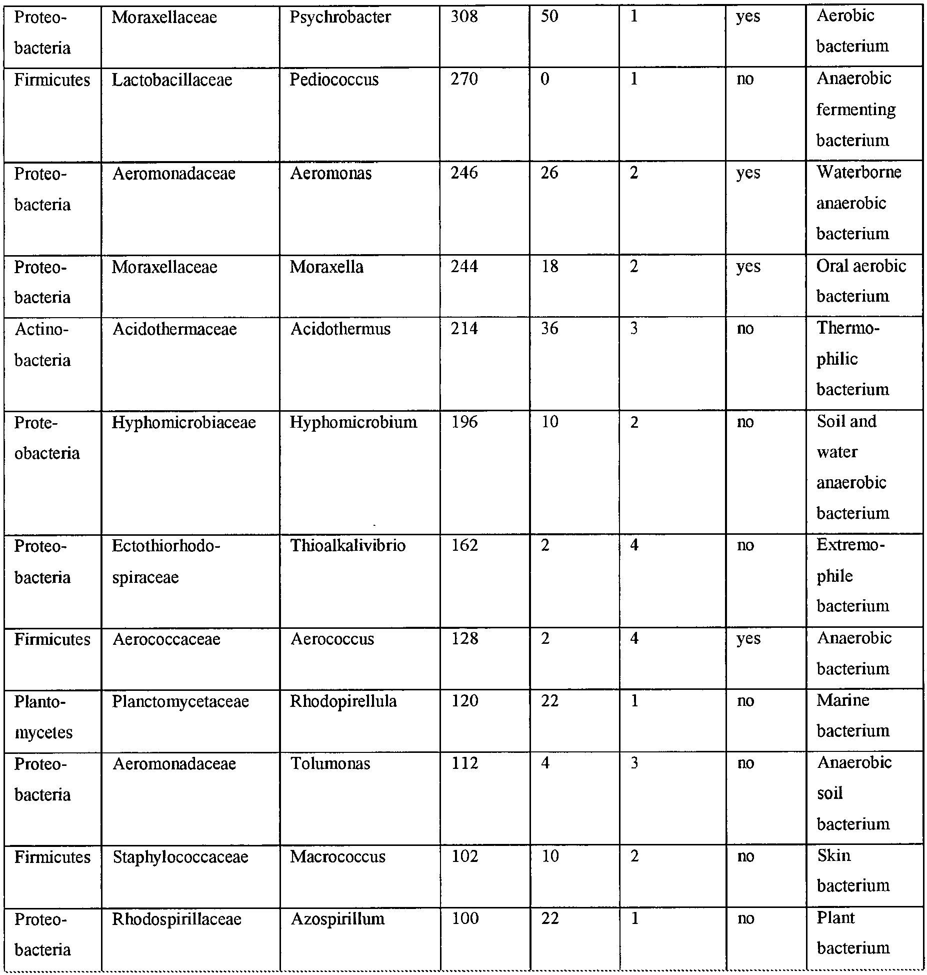

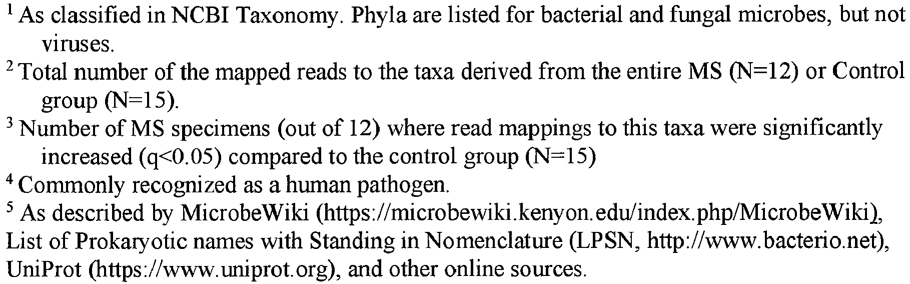

Representative mappings of read pair alignments for *Akkermansia* (sample MS-019) and *Pseudomonas* phage LUZ24like virus (sample MS-053) are shown in Figure 2. The distribution of observed RNA abundances across these genomes are consistent with the expected gene expression. For instance, the prokaryote (*Akkermansia*) mappings are enriched within the rRNA genes with far fewer mappings to other bacterial genes. And for the LUZ24like virus, the observed RNA abundances map to structural genes, as expected in a replicating bacteriophage.

*MS Microbes.* A list of microbes for each MS brain sample was derived by looking for significant outliers within the dataset (see Methods for details of the analysis). Each specimen was compared to the set of controls, with multiple comparisons adjusted using the False Discovery Rate (q < 0.05) (Benjamini, Y. & Hochberg, Y. Journal of the Royal Statistical Society. Series B (Methodological) 57, 289-300 (1995)). Sequences from 42 microbial families were overrepresented in at least one sample in the MS group. MS microbes included 1 archaeal, 35 bacterial, 2 fungal, and 4 viral families (Figure 1).

Significant overrepresentation (q < 0.05) of microbial sequence in at least one of the MS samples was also seen for 84 genera. The list was filtered to include those genera where there were 100 or more mapped reads among the members of the MS group. This led to a more tractable list of 29 MS microbial genera from 11 different phyla (Table 6). The MS microbes or MS microbial families with the greatest number of mapped reads in the entire set of MS samples includes the bacterial genera *Nitrosospira, Atopobium, Fusobacterium,* and *Aggregatibacter,* and the fungal genus *Ustilago,* each with more than 1000 mapped reads. The MS microbes listed in Table 6 include 26 bacterial, 2 fungal, and 1 viral genera. The complete list of 84 genera is not shown.

Another way of ranking the MS microbes is by number of specimens significantly increased over controls: *Bacteroides* and *Rubrobacter* (5 specimens increased); *Ustilago, Lactococcus, Capnocytophaga, Thioalkalivibrio, andAerococcus* (4 specimens increased); and *Acidothermus* and *Tolumonas* (3 specimens increased). The MS microbes or MS microbial families are also listed by subject (sample). (See Table 3. This data is actually specimen-specific, since subject MS-021 was biopsied twice.) Ten of the 12 MS subjects had at least one overrepresented microbial genus. The number of microbial candidates observed in the samples ranged from 0 to 28.

*Analysis of the MS Cluster.* Four MS samples 17, 21-2, 55 and 56 had a similar pattern of enrichment of MS microbes at the family level as displayed on the right side of Figure 1. These samples were compared to the others within the MS group for several technical and clinical parameters. The "cluster" (N=4) vs. "no cluster" (N=8) MS subgroups did not differ significantly in total RNA yield, nor in their microbial fractions. The 4 cluster samples had macrophage prominence on pathologic analysis, compared to 6 of 8 of the no cluster samples (p = NS). Likewise, the interval between the date of neurologic symptom onset and brain biopsy did not differ between the subgroups. As expected, the cluster group had significantly more MS microbes at the genus level (mean 21.0) than the no cluster group (mean 6.4, p = 0.01).

*Human Differential Gene Expression.* Six hundred eighty-two genes were found to be differentially expressed between the MS and Control groups (FDR < 0.05). Compared to the control samples, the MS samples have many overexpressed immune related genes. Analysis of enrichment by pathway shows 5 immune related pathways, a secretion pathway, and 2 cell-surface interaction pathways are significantly enriched for genes that are overexpressed in the MS samples (Table 7). Conversely the control samples, that are relatively free of macrophages, show relatively higher expression of neuronal genes and enrichment in neuronal pathways.

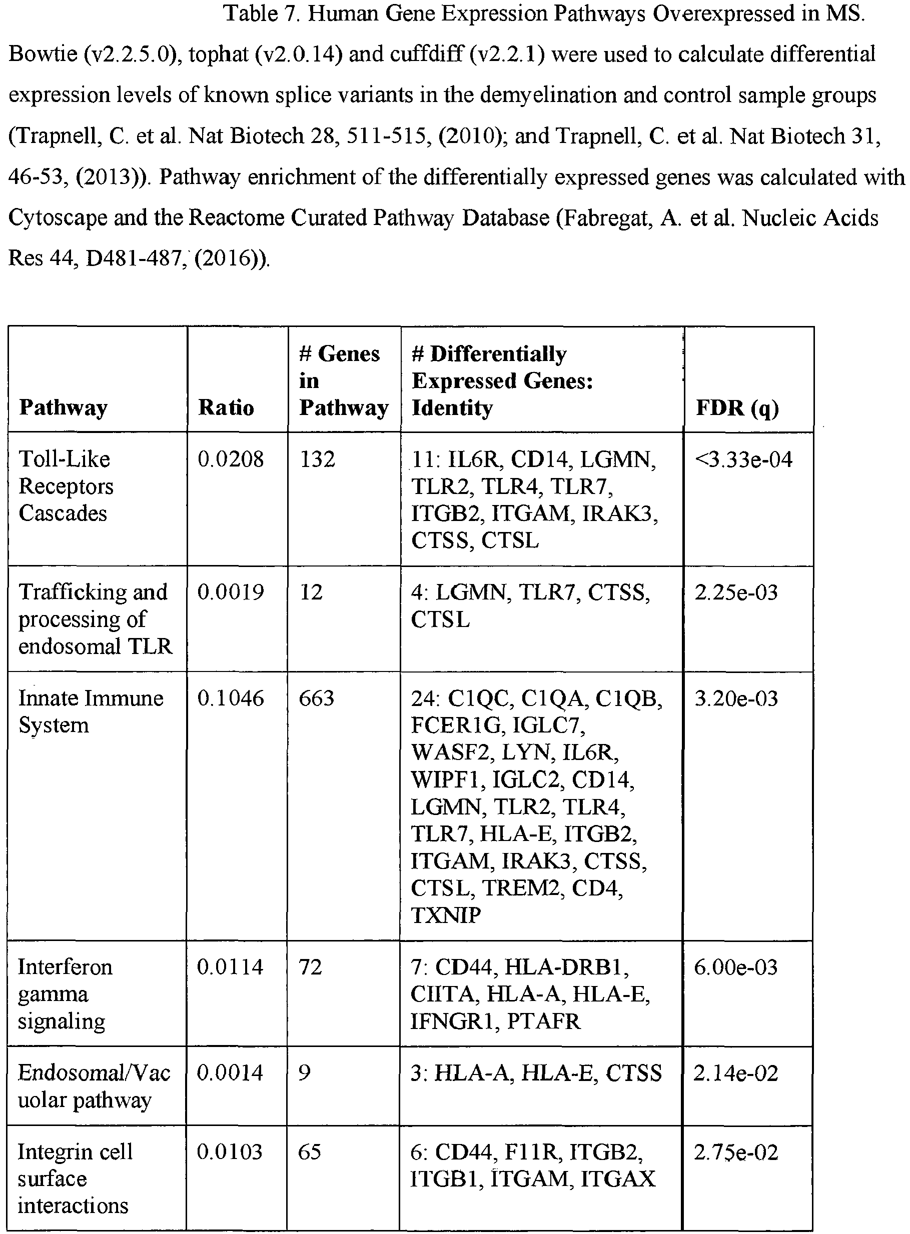

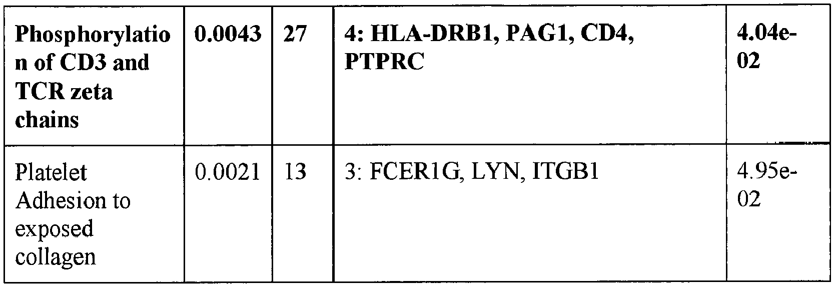

*Immunohistochemical Analysis.* Unfortunately, not all the sequenced specimens from the MS group were large enough to allow subsequent immunohistochemical analysis. Four specimens were subjected to staining with antibacterial (peptidoglycan) monoclonal antibodies and controls. Peptidoglycan is a structural cell wall component of both gram-positive and gram-negative bacteria. Representative brain tissue sections from this analysis along with controls are shown in Figure 3. Peptidoglycan signal was demonstrated in both MS specimens and another brain abscess positive control specimen. Some peptidoglycan signal was also observed in several epilepsy control specimens, but the pattern of staining was much more limited. Matched IgG1 isotype controls support the specificity of the anti-peptidoglycan signal. Anti-CD68 and anti-lysozyme staining confirmed the presence of macrophages and neutrophils in the MS-019 specimen. Conventional neuropathology showed the presence of activated macrophages in most of the other MS specimens.

Discussion: RNA sequencing and immunohistochemistry both suggest the presence of microbes in many of the MS brain specimens that differed from the controls. In the brain biopsy samples where MS microbes were detected, sequences mapped to a diverse set of bacterial taxa. The samples did not shared the same bacterial signatures, and no single bacterial taxon was found in all the samples. This suggests that MS disease is associated with the presence of various microbes within a lesion, or that the macrophage infiltration provides a route for bacterial transport to the lesion. These possibilities are not mutually exclusive and further studies will be required to define how microbial RNA and cell-wall components reach the brain and whether the presence of microbes or their components affects the initiation or course of the disease.

This study has some important limitations. First, the control specimens were taken from tissue affected by epilepsy. Therefore, the control specimens are not from completely normal brain. Autopsy samples could be specifically selected for a normal pathologic appearance, but quality analysis of a few such samples early on in this study showed that the RNA from FFPE autopsy brain samples was of very low quality - too low to be useful for sequencing. Some mismatch between epilepsy controls, taken primarily from cortex (the epileptogenic focus), and the MS samples, containing both cortex and white matter, was therefore unavoidable. Biopsy site effects on the microbial analysis cannot be excluded. Second, many of the MS patients received some disease-modifying therapy (DMT in Table 3), while the epilepsy control subjects were treated for their condition with an entirely different set of drugs. Some drug effects on the microbial composition of the samples cannot be excluded, although this seems unlikely because neither MS nor epilepsy drugs are considered to be antimicrobials. Lastly, the earlier collection dates among the MS samples compared with the controls probably had little or no effect on the results because there was no discernable relationship between collection date and microbial mapped reads.

The data presented here demonstrate a correlation between the presence of microbial macromolecules from taxonomically diverse organisms, primarily bacteria, in MS brain lesions. This is supported by distinct mapping of sequencing reads to some bacterial genomes (e.g., *Atopobium, Fusobacterium Akkermansia*) and by immunohistochemical detection of bacterial peptidoglycan within lesions in several of the subjects. The data reported here does not specifically distinguish between living microbes and the remnants of microbes (e.g., nucleic acid, peptidoglycan) which are no longer viable. However, the presence of the bacteriophage, LUZ241likevirus, in 2 of the MS brain samples (MS-021-2 and MS-053) implies that its host, *Pseudomonas aeruginosa*, was also present. (*Pseudomonas* itself was likely lost from the final analysis when gram-negative bacterial sequences were filtered out.) Since LUZ24likevirus is a lytic (or virulent), not temperate (or lysogenic), bacteriophage, it is likely that *Pseudomonas* was also actively replicating in these samples at the time of collection (Ceyssens, P. J. et al. Virology 377, 233-238, (2008)). More work will be required to distinguish replicating from nonreplicating bacteria in the MS brain specimens, but is it possible that nonviable bacterial components may be sufficient to stimulate macrophage infiltration and demyelination.

Microbial RNAs were also observed in the control specimens, and this was also supported by immunohistochemical analysis. The reasons for this are not clear, but might have to do with mapping electrodes placed and open procedures performed in the epilepsy controls. These open procedures were required to find, map, and remove the epileptogenic foci. Efforts were made to exclude contamination at points along the pipeline, but the FFPE specimens themselves are necessarily not completely sterile or free of microbial RNA, necessitating the use of an experimentals (MS) vs. controls (epilepsy) study design. Microbial reads in the control specimens might also be telling us something about epileptogenic foci within brain tissue.

Many of the MS microbes (listed in Table 6) are from anaerobic bacterial genera. Many of these might be considered to be commensal bacteria (e.g., *Bifidobacterium, Atopobium*) and it is currently unclear if they play a role in MS pathogenesis. However, a variety of anaerobic and nonpathogenic bacterial species have also been observed in brain abscesses by sequencing analysis (Al Masalma, M. et al. Clinical infectious diseases: an official publication of the Infectious Diseases Society of America 54, 202-210, (2012)). Since many brain abscess specimens do not grow in culture and the microbiology of these lesions is complex, the microbes found within brain abscesses might have some role in the pathologic process. This concept of microbiologic complexity could also apply to MS.

Several of the specimens in the MS group had an enrichment of MS microbes at the family level, shown as a cluster on the right side of Figure 1. It's not clear why these 4 samples had a larger number of MS microbes than the other MS brain biopsy samples.

Laman's group in the Netherlands first demonstrated bacterial antigen, specifically peptidoglycan, by immunohistochemistry in brain tissue from donors with MS (Schrijver, I. A. et al. Brain 124, 1544-1554 (2001)). The authors also demonstrated anti-peptidoglycan antibodies in the CSF of patients with active MS. This group went on to hypothesize that peptidoglycan is involved in the development of CNS autoimmunity (Visser, L. et al. Journal of immunology (Baltimore, Md.: 1950) 174, 808-816 (2005)). Since that time, a Canadian group led by Chris Power showed bacterial sequence in brain tissue from patients with a variety of CNS diseases, including one patient with MS (Branton, W. G. et al. PLoS One 8, e54673, (2013)). More recently, this group performed RNA sequencing on 6 autopsy-derived MS and 6 control brain samples (Branton, W. G. et al. Scientific reports 6, 37344, (2016)). Their analysis revealed a preponderance of Proteobacteria sequence in the progressive MS and control brain samples, with Actinobacteria sequence predominating in 3 relapsing-remitting MS brain samples. These results were supported by IHC and gene expression studies, and were interpreted as consistent with a disruption of the microbiota within the demyelinating lesions characteristic of MS. The Power group findings are similar to the report disclosed herein, although more controls, deeper sequencing, different mapping and analysis methods, and fixed paraffinized specimens from living subjects were used.

Enrichment of some bacteria in the stool of MS patients compared with controls has been observed by other groups (Cantarel, B. L. et al. Journal of investigative medicine: the official publication of the American Federation for Clinical Research 63, 729-734, (2015); and Jangi, S. et al. Nature communications 7, 12015, (2016)). The study by Jangi et al. was also a sequencing study, looking at stool microbiota in MS patients compared with healthy controls. Interestingly, sequences mapped to two genera were increased in the stool of MS patients *- Akkermansia* and *Methanobrevibacter.* The present sequencing study in brain tissue specimens, not stool, also identified *Akkermansia* among the MS microbes (see Table 6).

Subject MS-21 was biopsied twice over the course of several months, acting as its own unintentional control. The first biopsy (MS-21-1) was normal while the second (MS-21-2) showed clear-cut demyelination. Interestingly, 5 MS microbes were detected in the first (nearly normal) biopsy with relatively few reads mapped (≤ 50 each). The second diseased biopsy, however, revealed 28 MS microbes and 4 of these MS microbes each had more than 100 reads mapped. While this data is from a single subject, it does support the hypothesis that demyelinating lesions are associated with more bacteria of multiple types that are significantly different than the controls.

The human gene expression analysis identified Toll-Like Receptors Cascades and Trafficking and processing of endosomal TLR pathways as the most significantly overexpressed in brain tissue among the MS subjects compared to the controls. These pathways include TLR2 and TLR4, both associated with immunity to bacterial pathogens, and TLR 7, associated with immunity to ssRNA (viruses). Other groups have shown an associated between MS and TLRs (Bustamante, M. F. et al. Ann Neurol 70, 634-645, (2011); Hamid, K. M. et al. Iranian journal of allergy, asthma, and immunology 15, 75-81 (2016); Hossain, M. J., Tanasescu, R. & Gran, B. Oncotarget 6, 35131-35132, (2015); Nyirenda, M. H. et al. J Immunol 194, 5761-5774, (2015); and White, A. T., Light, A. R., Hughen, R. W., Vanhaitsma, T. A. & Light, K. C. Psychosomatic medicine 74, 46-54, (2012)). Interestingly, TLR4 expression is lower in PBMCs from MS patients compared to controls, whereas we detected higher expression in affected brain tissue (Hamid, K. M. et al.; and Iranian journal of allergy, asthma, and immunology 15, 75-81 (2016); and White, A. T., Light, A. R., Hughen, R. W., Vanhaitsma, T. A. & Light, K. C. Psychosomatic medicine 74, 46-54, (2012)).

The source of the microbial sequences and antigen observed in the MS lesions is currently unknown. There are at least two possibilities: 1) hematogenous seeding from a bacteremia, or 2) microbes were brought in by the infiltrating macrophages. If bacteria are in fact seeded into MS lesions from bacteremia, it seems curious that this has not been discovered before. However, many of the MS microbes identified in this study are anaerobic or even unculturable, and it is well established that recovery of microbes from brain abscesses (with a different pathologic appearance than demyelination) is often difficult. Macrophages could be bringing bacterial RNA and antigens into these MS lesions as a result of an autoimmune process. While macrophages and neutrophils are usually considered to be responders to tissue damage, not initiators, macrophages can actively participate in tissue destruction (Dragomir, A. C., Laskin, J. D. & Laskin, D. L. Toxicology and applied pharmacology 253, 170-177, (2011); and Laskin, D. L., Pilaro, A. M. & Ji, S. Toxicology and applied pharmacology 86, 216-226 (1986)).

Some recent murine studies show that normal gut flora influence brain development and behavior, via peptidoglycan which crosses the blood brain barrier and interacts with pattern-recognition receptor Pglyrp2 (Arentsen, T., Khalid, R., Qian, Y. & Diaz Heijtz, R. Brain, behavior, and immunity 67, 345-354, (2018); Arentsen, T. et al. Molecular psychiatry 22, 257-266, (2017); and Diaz Heijtz, R. et al. Proceedings of the National Academy of Sciences of the United States of America 108, 3047-3052, (2011)). The source of the peptidoglycan seems to be the developing normal gut flora. Another group studying experimental autoimmune encephalomyelitis (EAE, an animal model disease that resembles MS) showed that peptidoglycan works through receptors NOD1, NOD2, and RIP2 and dendritic cells to worsen the disease (Shaw, P. J. et al. Immunity 34, 75-84, (2011)). TLR2 and NOD also appear to mediate EAE disease activity in primates (Visser, L. et al. The American journal of pathology 169, 1671-1685, (2006)). Finally, treatment of EAE mice with oral Lactobacillus paracasei improved measures of disease severity (Libbey, J. E. et al. Beneficial microbes 9, 495-513, (2018)). Whether any of these intriguing findings in mice apply directly to human MS is not clear, but they do demonstrate the importance of peptidoglycan and its sensing molecules to the progression of EAE.

The concept of "immunologic scarring" has been proposed where infections trigger longer term immune dysfunction (Nathan, C. Science (New York, N. Y.) 350, 161, (2015)). This could help explain the persistent or recurrent dysfunction that occurs in MS, even without the persistence of microbial antigen. The present study involved brain biopsies generally taken early on in the disease course for diagnostic purposes.

### Example 2: Spectrum of Microbial Sequences Brain Specimens

Table 8 provides a MS microbial list. Unbiased (deep, next generation) RNA sequencing was performed on 18 formalin-fixed paraffin embedded primary demyelination brain specimens (MS group), 16 epilepsy brain specimens (control group), and 2 blanks (no tissue). Criteria for inclusion into Table 8 include: (1) Overexpression in the MS group where a) the MS:Control Ratio ≥ 3.0; and b) the MS:Blank Ratio > 1.5; and (2) > 1000 total reads summed from the members of the MS group (N=16) (MAPQ filtering was not performed).

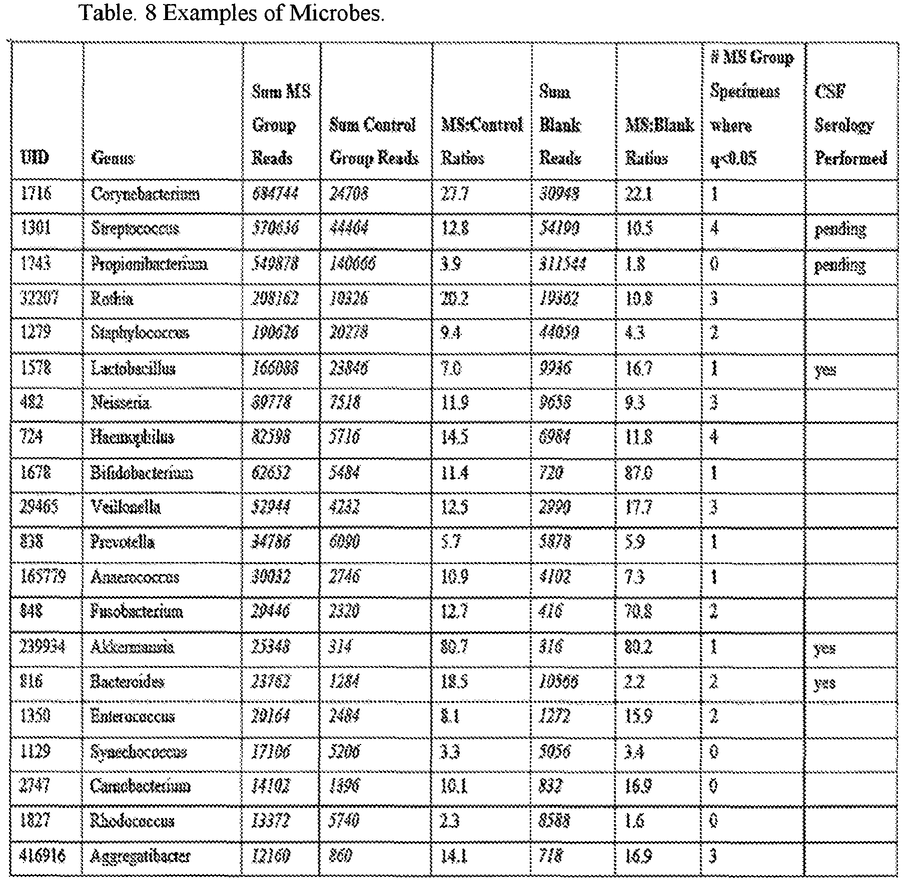

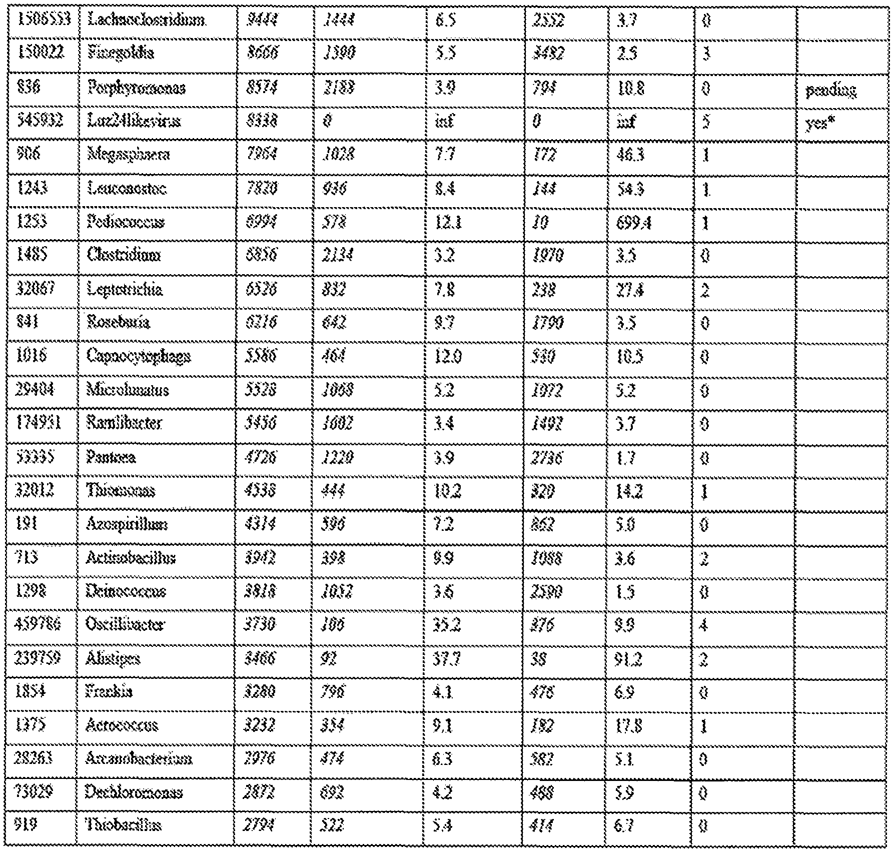

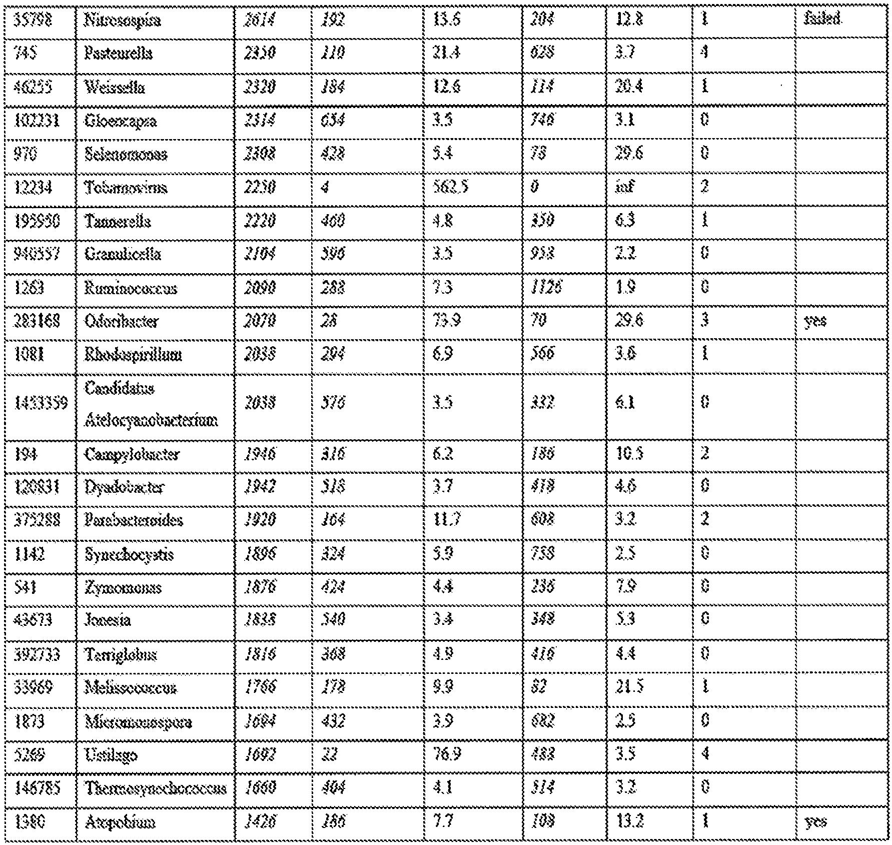

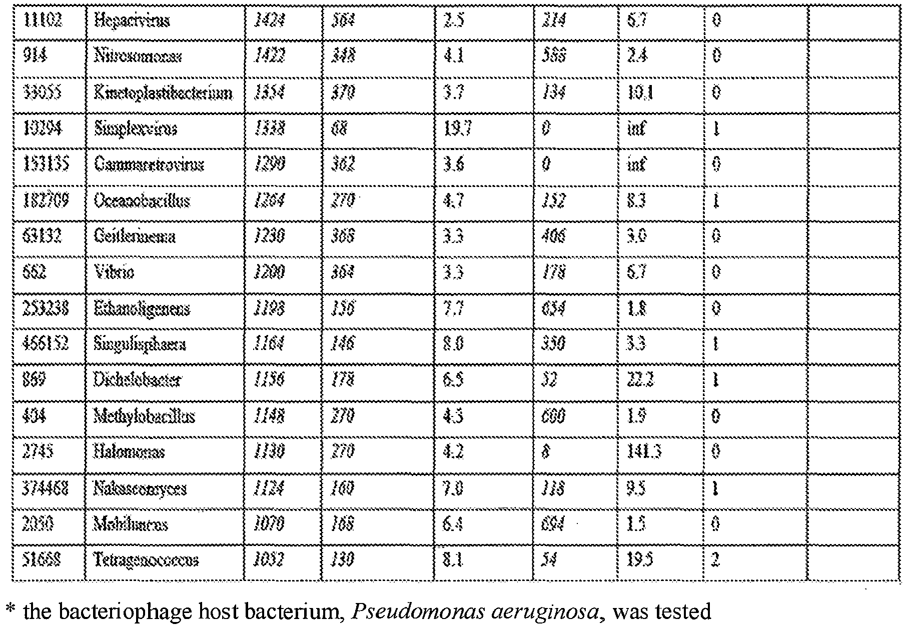

Next, ELISA indices (EI) were averaged for repeat determinations and used to categorize CSF serologic responses among enrolled human subjects with demyelinating disease (DD, MS group), other neurologic diseases (OND group), or shunt patients expected to have normal CSF (control group). EI values were determined by dividing experimental OD values by the average OD of 3 calibrator CSF specimens. Positive and negative controls confirmed the validity of each CSF serology run. Bacterial antigens were selected from the prior sequencing data. Indirect ELISA was performed with CSF (neat) against whole bacteria, sonicated and washed.

Table 9 shows the summary of the serology data for each group compiled from CSF serologies against 6 MS microbes or microbial families. Table 9 is organized to show qualitative differences in antibody responses between the MS, OND, and control groups. The data was statistically analyzed, confirming that the MS and OND groups are more reactive than the controls for most of the antigens tested. The results provided in Table 8 can be used as a panel type test for directing treatment of patients with active MS.

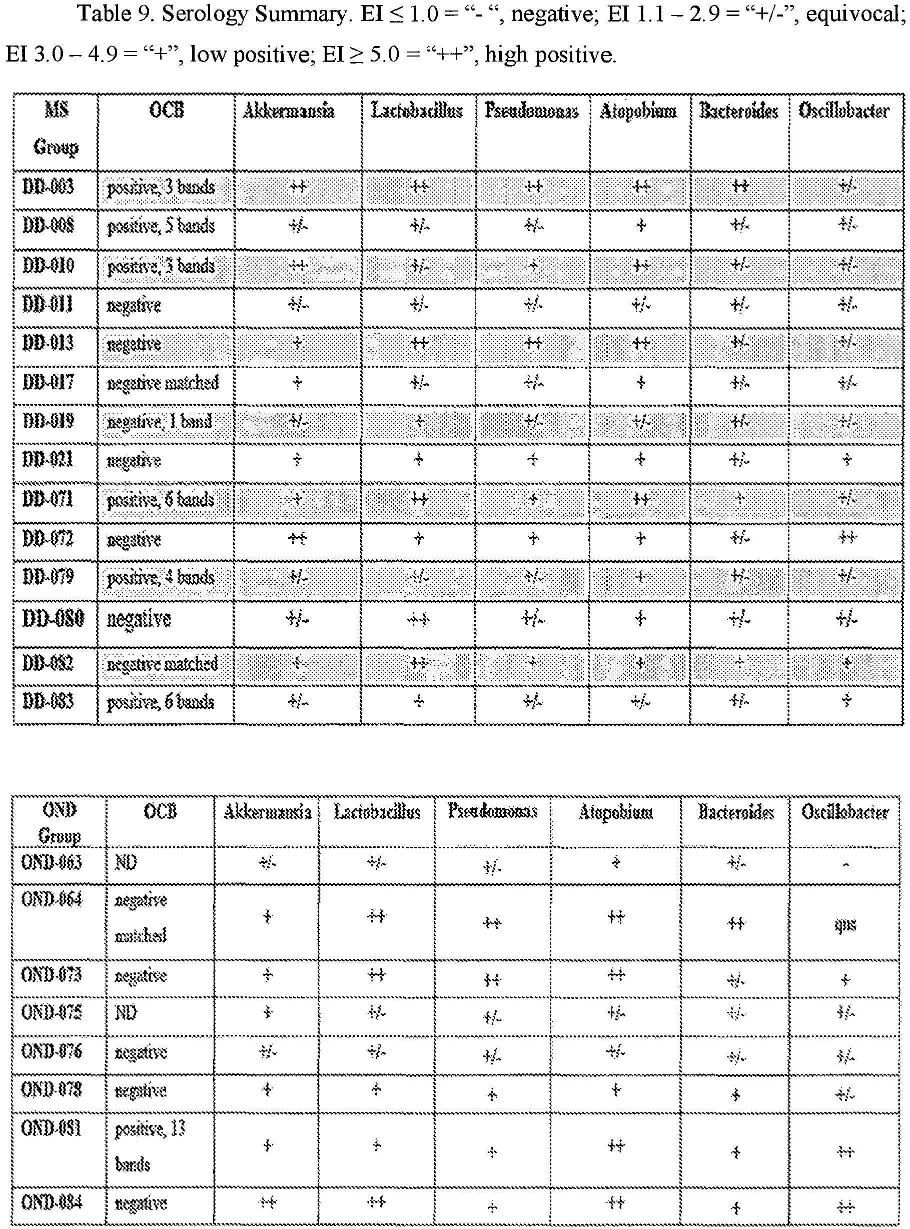

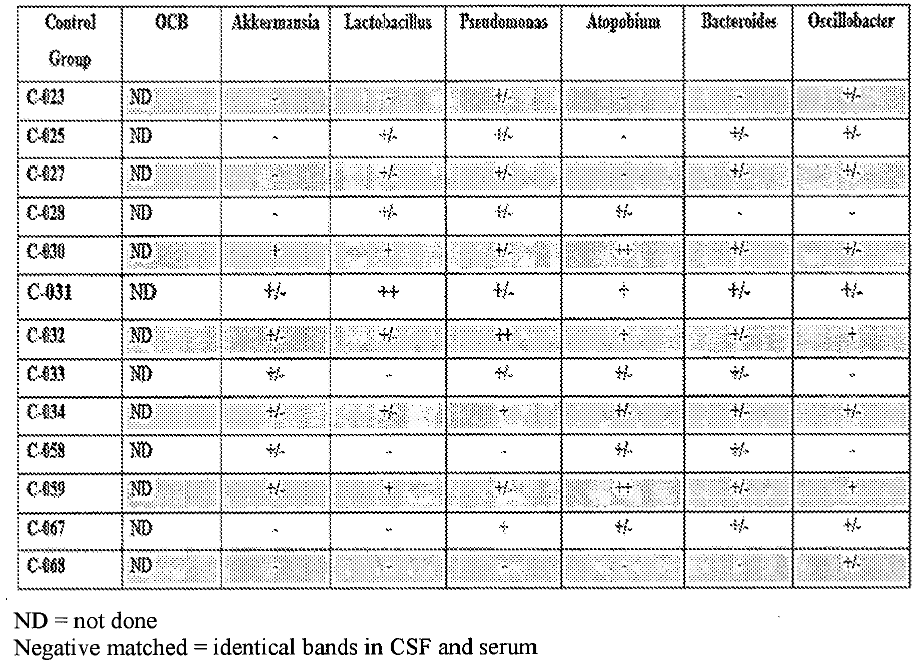

*Microbial ELISA Index compared with the Albumin Index in the assessment of demyelinating disease.* Fig. 6 shows EI vs. Albumin Index. These data show the possible intrathecal synthesis of antibody against some of the MS microbes or MS microbe families in some of the subjects. For example, the data generated in this table can provide which subject has an unexpectedly high antibody levels against any given antigen, accounting for leakage of antibody from the serum across the blood brain barrier. Such test can be used to direct treatment of patients with active MS.

More specifically, Fig. 6 shows spinal fluid ELISA indices (EI, a measure of antibodies) for six MS microbial candidate antigens plotted against the albumin index (AI). The box shows the normal albumin index region (from 0 to 9) indicating blood-brain barrier (BBB) intactness. The displayed line is a linear regression analysis showing the relationship between the EI's for each antigen and BBB intactness (as assessed by the Albumin Index). This line provides an "expected EI" for each bacterial antigen tested, and allowing to correct for the leaky BBB often seen in patients with MS and other demyelinating diseases. Values above the regression line, and particularly those within the normal Albumin Index boxed area, indicate likely intrathecal antibody synthesis (i.e., causality of demyelination).

The albumin index of a subject can be calculated as follows: Albumin Index = CSF albumin (mg/L) / serum albumin (g/L). AI is an index of BBB integrity, adjusted for the serum albumin concentration. AI is increased in BBB dysfunction with some limitations. For example, LeVine SM. Albumin and multiple sclerosis. BMC Neurol. 2016 Apr 12;16:47. doi: 10.1186/s12883-016-0564-9. PMID: 27067000; PMCID: PMC48287

The ELISA index can be calculated/determined as follows. *Bacterial antigen preparation.* Criteria for selection: Prior to harvesting antigen, serial dilutions were conducted in order to obtain various ODs for ELISA optimization. Bacterial strains were obtained from ATCC as freeze-dried, lyophilized cultures. Cultures were rehydrated using the appropriate medium and incubation conditions specified on the product sheets. Organisms were allowed to come out of a lyophilized state, and once within the exponential phase of growth stock cultures were stored at -80°C with a final concentration of 10% glycerol. Broth and plate cultures intended for antigenic harvesting were harvested during the exponential phase or shortly thereafter. Prior to harvesting antigen, cells from broth cultures were washed with PBS, and serial dilutions were conducted from both plate and broth cultures in order to obtain an OD of 1.0, which is equivalent to approximately 10e8 to 10e9 cells. Plates were read on a Gen5 2.08 ELISA plate reader at 600nm, a common metric used for estimating the concentration of bacterial cells in a liquid. Cells were sonicated for 10 seconds with cycle time continuous, a duty cycle of 40%, and at position 4.5 with a Branson Sonifier 250. The sonicated organism was then stored at -80°C for future use.

*ELISA optimization.* Flat bottom 96-well ELISA plates were used and coated with 50µl/well of harvested antigen (sonicated bacteria), covered, and rocked overnight at 4°C. The plates were washed with phosphate buffered saline with Tween 20 (PBST) (washes were done with 100µl/well of buffer), blocked for one hour at room temperature (RT), and again washed with PBST before applying the appropriate primary antibody, which was rocked at RT for two hours. Following incubation of the primary antibody the plate was washed with PBST, and the appropriate secondary antibody was applied and rocked at RT for one hour. Following incubation of the secondary antibody the plate was washed with PBST, followed by 100µl/well of TMB for five minutes. The reaction was stopped with 100µl/well of 2 normal sulfuric acid (2N HCL) and read on a Gen5 2.08 ELISA plate reader at 450nm.

A series of steps were taken in order to optimize the ELISA procedure. 1) various primary, anti-bacterial, antibodies were assessed for best positive control, which consisted of concentrations that yielded a strong and weak (just above the background) positive control. These antibodies consisted of monoclonal anti-peptidoglycan (MAB995), monoclonal anti-LPS, and polyclonal anti-pseudomonas. 2) In addition to using anti-bacterial antibodies, Randox (Randox Laboratories Ltd, UK), a human CSF control that has 10 mg/dl of IgG as a positive control, and depleted Randox as a negative control, were also used. This depletion was accomplished with the HiTrap Protein G HP kit (GE), and depletion was verified with Human Total IgG Platinum ELISA kit (Affymetrix eBioscience). It was determined that two passes were sufficient to obtain optimal depletion. 3) various blocking agents were subsequently assessed with the respective antibody concentrations to find which was most efficient, they included 5X ELISA/ELISPOT Diluent (Invitrogen) (PBS supplemented with bovine serum), Blocker BLOTTO in tris-buffered saline (TBS) (ThermoScientific), Blocker Casein (Thermo Scientific), and Starting Blocker (ThermoScientific). 4) With the aforementioned optimized, the antigen coating concentration was then adjusted using ~0.5, ~1.0, and ~2.0 OD. Indeed, the plates were followed with an appropriate secondary antibody with the specified dilutions: anti-mouse IgG (Vector PI-2000, 1:3000), anti-rabbit IgG (Vector PI-1000, 1:3000), and anti-human (Jackson ImmunoResearch, 1:10,000). These optimization steps were done for each bacterial organism.

Throughout the optimization process, Rabbit anti-Pseudomonas Polyclonal Antibody (ThermoFisher Scientific, Rockford, IL) was the optimal primary, anti-bacterial, antibody for the of organisms tested, albeit to varying concentrations for both strongly and weakly positive. While antibodies specific to the organisms tested can be used, the polyclonal antibody used was sufficient in order to recognize characteristics common amongst bacteria, and much more cost effective. Serial dilutions of the antibody that yielded an OD of approximately 1.0 were used as a strong positive control, and those that were just above background served as a weak positive control. Randox also served as a positive control, albeit in order to establish a control of accuracy for the CSF samples. Similarly, depleted Randox served as a negative control and was used to calculate the ELISA index (EI).

*Analysis of ELISA.* Duplicates were run for each CSF and diluted serum sample, and controls in triplicates. An average OD was taken for each experimental and control sample and divided by depleted Randox, otherwise known as our "cut-off calibrator" in order to obtain the EIs. This was repeated in order to show reproducibility, and with the respective patient serum in order to assess intrathecal synthesis of IgGs. This was accomplished by diluting the serum down to the same concentration of the CSF and by using the ratio of CSF OD to diluted serum OD. Anything over an OD greater than one is suggestive of intrathecal synthesis (Halperin et al. 1989).

The method comprising comparing EIs against the Albumin Index can be used to show and/or determine intrathecal antibody synthesis (e.g., antibodies against one or more of the microbes disclosed herein, for example, listed in Table 8) in patients with demyelination. This method can be used to direct treatments. For instance, subject 3 shows very high EI values for several different MS candidate antigens (e.g., *Atopobium, Akkermansia, and Lactobacillus*)*.* The Albumin Index for subject 3, however, is also very high (~30), indicating a leaky BBB. Moreover, subject 3's antibody level against *Akkermansia* is above the expected level (the linear regression line). This data indicates that subject 3 is making more antibodies against *Akkermansia* than can be accounted for by just leakage from the serum. The disclosed method can be used to assess intrathecal production of antibodies (i.e., antibodies made in the brain and spinal cord) that can be usedto prove causality of the demyelination and then direct treatment,

Other methods for determining intrathecal production of antibodies are known. For example, the most direct method is to determine total IgG concentrations in both serum (typically around 1000 mg/dl) and CSF (typically <5), dilute the serum appropriately, and measure the EI for both CSF and serum wherein total IgG concentrations are the same. This method referred to as the "direct method" in which the ratio of CSF EI to serum EI is 1.0 or less. Ratios > 1.0 indicate intrathecal antibodies production.

A direct comparison of CSF and serum has been carried out for some of the demyelination subjects against 5 bacterial antigens (Akkermansia, Lactobacillus, Pseudomonas, Atopobium, and Bacteroides). This analysis showed CSF/Serum EI ratios >1.0 for MS subjects 82 and 83 against some of the antigens. The other EI ratios were < 1.0, indicating more antibodies in serum than in CSF.

The Microbial EI vs. Albumin Index method, however, identified several other subjects that had higher than expected antibodies levels in their CSF. These include subject 10 (*Atopobium, Akkermansia, Pseudomonas*), subject 72 (*Akkermansia*, *Odoribacter*), subject 13 (*Pseudomonas*), and subject 3 (*Akkermansia*)*.* This method can be used to identify subjects that may otherwise be missed using known methods of determining BBB intactness.

## Claims

1. A method of diagnosing a demyelinating disease in a subject, the method comprising:
a) obtaining a sample from the subject, wherein said sample is suspected of containing microbe-specific antibodies;
b) incubating the sample with one or more microbe capture agents, wherein the one or more microbe capture agents bind to one or more of the microbe-specific antibodies, and wherein the microbe-specific antibodies are specific to one or more microbes selected from the list in Table 1, Table 2 or Table 8;
c) detecting the presence of one or more microbe-specific antibodies in the sample; and
d) diagnosing the subject with a demyelinating disease when the one or more microbe-specific antibodies are present in the sample.

2. A method of detecting a demyelinating disease in a subject, the method comprising:
a) obtaining a sample from the subject, wherein said sample is suspected of containing microbe-specific antibodies specific to one or more microbes selected from the list in Table 1, Table 2 or Table 8;
b) contacting the sample with one or more microbe capture agents in conditions to allow one or more microbe capture agents bind to one or more of the microbe-specific antibodies to generate a mixed biological sample, wherein each of the one or more microbe capture agents is specific to one or more a microbe-specific antibodies, and wherein the one or more microbe-specific antibodies are specific to one or more microbes selected from the list in Table 1, Table 2 or Table 8;
c) incubating said mixed biological sample under conditions such that: said one or more microbe capture agents specifically binds at least one of said microbe-specific antibodies to form a detectable complex; and
d) detecting the presence of said detectable complexes, thereby detecting the presence of one or more microbes selected from the list in Table 1, Table 2 or Table 8,
wherein the presence of one or more microbes selected from the list in Table 1 in said mixed biological sample detects a demyelinating disease in a subject.

3. The method of claim 1 or 2, wherein the subject is suspected of having or is at risk of having a demyelinating disease.

4. The method any of claims 1-3, further comprising repeating step b) and c) using different microbe capture agents that are specific to the one or more microbe-specific antibodies in the sample.

5. The method of any of claims 1-4, wherein the sample is brain tissue, cerebral spinal fluid, spinal cord tissue, or blood.

6. The method of claim 1 or 2, wherein the demyelinating disease is multiple sclerosis, neuromyelitis optics, acute disseminated encephalomyelitis or clinically isolated syndrome.

7. The method of any of claims 1-6, wherein subject is a human.

8. The method of any of claims 1-7, further comprising: administering to the subject an antibacterial agent, an antiviral agent, an antifungal agent or a combination thereof.

9. The method of claim 8, wherein the an antibacterial agent is administered to the subject when the one or more microbe-specific antibodies or microbes detected is a bacteria selected from the list in Table 1, Table 2 or Table 8; an antiviral agent administered to the subject when the one or more microbe-specific antibodies or microbes detected is a virus selected from the list in Table 1, Table 2 or Table 8; or an antifungal agent administered to the subject when the one or more microbe-specific antibodies or microbes detected is a fungus selected from the list in Table 1, Table 2 or Table 8.

10. The method of claim 1 or 2, further comprising detecting the presence of one or more microbe-specific antibodies specific to one or more of the microbes listed in Table 2.

11. The method of claim 1 or 2, further comprising detecting the presence of one or more microbe-specific antibodies specific to one or more of the microbes listed in Table 8.

12. The method of claim 2, further comprising detecting the presence of one or more microbes selected from the group of microbes listed in Table 2.

13. The method of claim 1 or 2, further comprising detecting the presence of one or more microbes selected from the group of microbes listed in Table 8.

14. The method of claim 1 or 2, further comprising determining the quantity of one or more of the microbe-specific antibodies in the sample.

15. The method of any of the preceding claims, further comprising amplifying one or more nucleic acid sequences in the sample, wherein the one or more nucleic acid sequences correspond to one or more of the microbes in Table 1, Table 2. or Table 8.

16. The method of claim 15, wherein the one or more nucleic acid sequences are amplified using a primer pair that specifically amplifies the one or more nucleic acid sequences.

17. The method of claim 16, further comprising determining whether the expression of the one or more nucleic acid sequences corresponding to the one or more of the microbes in Table 1, Table 2 or Table 8 is overexpressed compared to expression levels of a nucleic acid sequence of the same one or more of the microbes in Table 1, Table 2 or Table 8 in a control.

18. The method of any of the preceding claims, wherein the step of detecting comprises detecting by microarray.

19. The method of any of the preceding claims, wherein the step of detecting comprises detecting by ELISA.

20. The method of any of the preceding claims, wherein the one or more microbes detected is from the genus Akkermansia.

21. A method of diagnosing and treating a demyelinating disease in a subject, the method comprising:
a) obtaining a sample from the subject, wherein said sample is suspected of containing microbe-specific antibodies;
b) incubating the sample with one or more microbe capture agents, wherein the one or more microbe capture agents bind to one or more of the microbe-specific antibodies, and wherein the microbe-specific antibodies are specific to one or more microbes selected from the list in Table 1, Table 2 or Table 8;
c) detecting the presence of one or more microbe-specific antibodies in the sample;
d) diagnosing the subject with a demyelinating disease when the one or more microbe-specific antibodies are present in the sample; and
e) administering:
i) an antibacterial agent when the one or more microbe-specific antibodies detected is a bacteria selected from the list in Table 1, Table 2 or Table 8,
ii) an antiviral agent when the one or more microbe-specific antibodies detected is a virus selected from the list in Table 1, Table 2 or Table 8,
iii) an antifungal agent when the one or more microbe-specific antibodies detected is a fungus selected from the list in Table 1, Table 2 or Table 8, or v) a combination thereof to the subject.

22. A method of detecting and treating a demyelinating disease in a subject, the method comprising:
a) obtaining a sample from the subject, wherein said sample is suspected of containing microbe-specific antibodies specific to one or more microbes selected from the list in Table 1, Table 2 or Table 8;
b) contacting the sample with one or more microbe capture agents in conditions to allow one or more microbe capture agents bind to one or more of the microbe-specific antibodies to generate a mixed biological sample, wherein each of the one or more microbe capture agents is specific to one or more a microbe-specific antibodies, and wherein the one or more microbe-specific antibodies are specific to one or more microbes selected from the list in Table 1, Table 2 or Table 8;
c) incubating said mixed biological sample under conditions such that: said one or more microbe capture agents specifically binds at least one of said microbe-specific antibodies to form a detectable complex;
d) detecting the presence of said detectable complexes, thereby detecting the presence of one or more microbes selected from the list in Table 1, Table 2 or Table 8, wherein the presence of one or more microbes selected from the list in Table 1, Table 2 or Table 8 in said mixed biological sample detects a demyelinating disease in a subject; and
e) administering:
i) an antibacterial agent when the one or more microbes detected is a bacteria selected from the list in Table 1, Table 2 or Table 8,
ii) an antiviral agent when the one or more microbes detected is a virus selected from the list in Table 1, Table 2 or Table 8,
iii) an antifungal agent when the one or more microbes detected is a fungus selected from the list in Table 1, Table 2 or Table 8, or
v) or a combination thereof to the subject.

23. The method of any of claims 21-22, wherein the subject is suspected of having or is at risk of having a demyelinating disease.

24. The method of any of claims 21-23, further comprising repeating step b) and c) using different antibodies that are specific to the one or more microbes in the sample.

25. The method of any of claims 21-24, wherein the sample is brain tissue, cerebral spinal fluid, spinal cord tissue, or blood.

26. The method of claim 21, wherein the demyelinating disease is multiple sclerosis, neuromyelitis optics, acute disseminated encephalomyelitis or clinically isolated syndrome.

27. The method of any of claims 21-26, wherein subject is a human.

28. The method of claim 21, further comprising detecting the presence of one or more microbes in the sample, wherein the one or more microbes are selected from the group in Table 2.

29. The method of claim 21, further comprising detecting the presence of one or more microbes in the sample, wherein the one or more microbes are selected from the group in Table 8.

30. The method of claim 22, further comprising determining the antibody level in the sample for each of the microbes tested.

31. The method of any of the preceding claims, further comprising amplifying one or more nucleic acid sequences in a sample, wherein the one or more nucleic acid sequences correspond to one or more of the microbes in Table 1, Table 2 or Table 8.

32. The method of claim 31, wherein the one or more nucleic acid sequences are amplified using a primer pair that specifically amplifies the one or more nucleic acid sequences.

33. The method of claim 32, further comprising determining whether the expression of the one or more nucleic acid sequences corresponding to the one or more of the microbes in Table 1 is overexpressed compared to expression levels of a nucleic acid sequence of the same one or more of the microbes in Table 1 in a control.

34. The method of claim 21 or 22, wherein the step of detecting comprises detecting by microarray.

35. The method of claim 21 or 22, wherein the step of detecting comprises detecting by ELISA.

36. The method of claim 21 or 22, wherein the one or more microbes detected is from the genus *Akkermansia.*

37. A method of detecting one or more antibodies in a sample, the method comprising:
a) obtaining a sample from a subject; wherein the sample is suspected of containing microbe-specific antibodies specific to one or more microbes selected from the list in Table 1, Table 2 or Table 8;
b) incubating the sample with one or more microbe capture agents, wherein the one or more microbe capture agents bind to one or more of the microbe-specific antibodies, and wherein the one or more microbe-specific antibodies are specific to one or more microbes selected from the list in Table 1, Table 2 or Table 8; and
c) detecting the presence of one or more microbe-specific antibodies in the sample.

38. The method of claim 37, wherein the detection of the presence of one or more microbe-specific antibodies is indicative of a demyelinating disease.

39. The method of claim 37, wherein the sample is from a subject at risk of developing or is suspected of having a demyelinating disease.

40. The method of claim 39, wherein the subject is a human.

41. The method of claims 38 or 39, wherein the demyelinating disease is multiple sclerosis, neuromyelitis optics, acute disseminated encephalomyelitis or clinically isolated syndrome.

42. The method of claim 37, wherein the sample is brain tissue, cerebral spinal fluid, spinal cord tissue, or blood.

43. The method of claim 37, further comprising detecting the presence of one or more microbes selected from the group in Table 2.

44. The method of claim 37, further comprising detecting the presence of one or more microbes selected from the group in Table 8.

45. The method of claim 37, further comprising determining the quantity of one or more of the microbe-specific antibodies in the sample.

46. The method of claim 37, wherein the step of detecting comprises detecting by microarray.

47. The method of claim 37, wherein the step of detecting comprises detecting by ELISA.

48. The method of claim 37, further comprising amplifying one or more nucleic acid sequences in the sample, wherein the one or more nucleic acid sequences correspond to one or more of the microbes in Table 1.

49. The method of claim 48, further comprising determining whether the expression of the one or more nucleic acid sequences corresponding to the one or more of the microbes in Table 1 is overexpressed compared to expression levels of a nucleic acid sequence of the same one or more of the microbes in Table 1 in a control.

50. The method of claim 49, wherein the step of detecting comprises detecting by hybridization reaction.

51. The method of claim 50, wherein the hybridization reaction further comprises hybridizing the sample to one or more primer sets.

52. The method of claim 51, wherein the hybridization reaction is a polymerase chain reaction.

53. The method of claim 52, wherein the polymerase chain reaction is reverse transcription polymerase chain reaction.

54. The method of claim 49, wherein the step of detecting comprises detecting by expressed sequence tags.

55. The method of claim 49, wherein the step of detecting comprises detecting by localization.

56. The method of claim 37, wherein the one or more microbes detected is from the genus Akkermansia.

57. A method of detecting one or more microbes in a sample, the method comprising:
a) obtaining a sample from a subject; wherein the sample is suspected of containing one or more microbes selected from the list in Table 1, Table 2 or Table 8;
b) incubating the sample with one or more microbe capture agents, wherein the one or more microbe capture agents bind to one or more of the microbes selected from the list in Table 1, Table 2 or Table 8;
c) detecting the presence of one or more microbes selected from the list in Table 1, Table 2 or Table 8in the sample.

58. The method of claim 57, further comprising determining the quantity of the one or more microbes in the sample.

59. The method of claim 57, wherein the detection of the presence of one or more microbes is indicative of a demyelinating disease.

60. The method of claim 57, wherein the sample is from a subject at risk of developing or is suspected of having a demyelinating disease.

61. The method of claim 57, wherein the subject is a human.

62. The method of claim 59 or 60, wherein the demyelinating disease is multiple sclerosis, neuromyelitis optics, acute disseminated encephalomyelitis or clinically isolated syndrome.

63. The method of claim 57, wherein the sample is brain tissue, cerebral spinal fluid, spinal cord tissue, or blood.

64. The method of claim 57, further comprising detecting the presence of one or more microbes selected from the group in Table 2.

65. The method of claim 57, further comprising detecting the presence of one or more microbes selected from the group in Table 8.

66. The method of claim 57, wherein the step of detecting comprises detecting by microarray.

67. The method of claim 57, wherein the step of detecting comprises detecting by ELISA.

68. The method of claim 57, further comprising amplifying one or more nucleic acid sequences in the sample, wherein the one or more nucleic acid sequences correspond to one or more of the microbes in Table 1.

69. The method of claim 57, further comprising determining whether the expression of the one or more nucleic acid sequences corresponding to the one or more of the microbes in Table 1 is overexpressed compared to expression levels of a nucleic acid sequence of the same one or more of the microbes in Table 1 in a control.

70. The method of claim 57, wherein the step of detecting comprises detecting by hybridization reaction.

71. The method of claim 70, wherein the hybridization reaction further comprises hybridizing the sample to one or more primer sets.

72. The method of claim 71, wherein the hybridization reaction is a polymerase chain reaction.

73. The method of claim 72, wherein the polymerase chain reaction is reverse transcription polymerase chain reaction.

74. The method of claim 57, wherein the step of detecting comprises detecting by expressed sequence tags.

75. The method of claim 57, wherein the step of detecting comprises detecting by localization.

76. The method of claim 57, wherein the one or more microbes detected is from the genus *Akkermansia.*

77. A method of treating a patient with multiple sclerosis (MS) or an MS-related disease, the method comprising:
a) administering an antibacterial agent when the one or more microbe-specific antibodies detected is a bacteria selected from the list in Table 1, Table 2 or Table 8,
b) administering an antiviral agent when the one or more microbe-specific antibodies detected is a virus selected from the list in Table 1, Table 2 or Table 8,
c) administering an antifungal agent when the one or more microbe-specific antibodies detected is a fungus selected from the list in Table 1, Table 2 or Table 8, or
d) administering a combination of a), b), or c) to the subject;
wherein the patient is identified as needing an antibacterial agent, antiviral agent, antifungal agent or a combination thereof by
i. obtaining a sample from the patient, wherein the sample is suspected of containing microbe-specific antibodies;
ii. incubating the sample with one or more microbe capture agents, wherein the one or more microbe capture agents bind to one or more of the microbe-specific antibodies, and wherein the microbe-specific antibodies are specific to one or more microbes selected from the list in Table 1, Table 2 or Table 8; and
iii. detecting the presence of one or more microbe-specific antibodies in the sample.

78. The method of claim 77, wherein the antibacterial agent is meropenem or ceftriaxone.

79. The method of claim 77, wherein the MS-related disease is neuromyelitis optica (NMO), acute disseminated encephalomyelitis
(ADEM), or clinically isolated syndrome (CIS).

80. The method of claim 77, further comprising detecting the presence of one or more microbe-specific antibodies specific to one or more of microbes are selected from the list in Table 2.

81. The method of claim 77, further comprising detecting the presence of one or more microbe-specific antibodies specific to one or more of microbes are selected from the list in Table 8.

82. The method of claim 77, further comprising determining the quantity of one or more of the microbe-specific antibodies in the sample.

83. The method of claim 77, further comprising amplifying one or more nucleic acid sequences in the sample, wherein the one or more nucleic acid sequences correspond to one or more of the microbes in Table 1.

84. The method of claim 83, wherein the one or more nucleic acid sequences are amplified using a primer pair that specifically amplifies the one or more nucleic acid sequences.

85. The method of claim 84, further comprising determining whether the expression of the one or more nucleic acid sequences corresponding to the one or more of the microbes in Table 1 is overexpressed compared to expression levels of a nucleic acid sequence of the same one or more of the microbes in Table 1 in a control.

86. The method of claim 77, wherein the sample is brain tissue, cerebral spinal fluid, spinal cord tissue, or blood.

87. The method of claim 77, wherein the step of detecting comprises detecting by hybridization reaction.

88. The method of claim 87, wherein the hybridization reaction further comprises hybridizing the sample to one or more primer sets.

89. The method of claim 77, wherein the step of detecting comprises detecting by a polymerase chain reaction.

90. The method of claim 77, wherein the step of detecting comprises detecting by microarray.

91. The method of claim 77, wherein the step of detecting comprises detecting by ELISA.

92. The method of claim 77, wherein the one or more microbes detected is from the genus *Akkermansia.*

93. The method of any of the preceding claims, wherein the microbe capture agent is an antibody or a probe.

94. The method of any of the preceding claims, further comprising determining an ELISA index for one or more microbes, comparing the ELISA index to an albumin index of the subject.

95. The method of claim 94, further comprising: performing a linear regression analysis of the ELISA index and the albumin index.

96. The method of claim 94, further comprising generating a plot line.

97. The method of claim 96, wherein when the albumin index is equal to or less than 9 and the wherein ELISA index is above the plot indicates one or more microbe-specific antibodies in the CSF when compared to the serum for the one or more microbes.
